(19) Europäisches Patentamt / European Patent Office / Office européen des brevets

(11) **EP 2 209 001 A1**

(12) **EUROPEAN PATENT APPLICATION**
published in accordance with Art. 153(4) EPC

(43) Date of publication:
**21.07.2010 Bulletin 2010/29**

(51) Int Cl.:
*G01N 33/68* (2006.01)   *A61B 5/145* (2006.01)
*G01N 33/15* (2006.01)   *G01N 33/50* (2006.01)

(21) Application number: **08843277.8**

(22) Date of filing: **20.10.2008**

(86) International application number:
**PCT/JP2008/068981**

(87) International publication number:
**WO 2009/054350 (30.04.2009 Gazette 2009/18)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **25.10.2007 JP 2007277793**

(71) Applicant: **Ajinomoto Co., Ltd.**
**Tokyo 104-8315 (JP)**

(72) Inventors:
• **TAKAHASHI, Mitsuo**
**Kawasaki-shi**
**Kanagawa 210-8681 (JP)**

• **ANDO, Toshihiko**
**Kawasaki-shi**
**Kanagawa 210-8681 (JP)**
• **YAMAKADO, Minoru**
**Tokyo 101-8643 (JP)**

(74) Representative: **Johnson, Richard Alan et al**
**Mewburn Ellis LLP**
**33 Gutter Lane**
**London**
**EC2V 8AS (GB)**

(54) **METHOD FOR EVALUATION OF IMPAIRED GLUCOSE TOLERANCE**

(57)   Provided are a method of evaluating IGT, an IGT-evaluating apparatus, an IGT-evaluating method, an IGT-evaluating system, an IGT-evaluating program, a recording medium, and the like, which are capable of evaluating an impaired glucose tolerance state accurately by utilizing concentrations of amino acids in blood. According to the method of evaluating IGT of the present invention, amino acid concentration data on concentration values of amino acids in blood collected from a subject to be evaluated is measured, and the impaired glucose tolerance state in the subject is evaluated based on the measured amino acid concentration data of the subject.

FIG.1

(BASIC PRINCIPLE OF THE INVENTION)

**Description**

TECHNICAL FIELD

The present invention relates to a method of evaluating IGT (impaired glucose tolerance), an IGT-evaluating apparatus, an IGT-evaluating method, an IGT-evaluating system, an IGT-evaluating program, and a recording medium, which utilize concentrations of amino acids in blood (plasma).
The present invention also relates to a method of searching for prophylactic/ameliorating substance for IGT, wherein a substance for preventing an IGT or ameliorating an IGT state is searched.

BACKGROUND ART

According to a survey on the actuality of diabetes conducted by Ministry of Health, Labour and Welfare, Japan, in 2002 (refer to Nonpatent Literature 1), approximately 7.4 million people were "those who were strongly suspected of having diabetes (those whose HbAlc (hemoglobin Alc) were equal to or more than 6.1% or those who were receiving treatment for diabetes as of the time of survey)" and approximately 8.8 million people were "those in whom the possibility of diabetes could not be ruled out (those whose HbA1c were equal to or more than 5.6% and less than 6.1% and who were not receiving treatment for diabetes as of the time of survey)", and the number of these people was summed up to approximately 16.2 million nationwide. That is, one in every six adults was estimated to suffer from diabetes or prediabetes. This survey elucidated the actuality of diabetes, which has come to be called "the national disease of the 21st century", and posed a significant problem in terms of health and medical care of the population.
In impaired glucose tolerance, which is a prediabetic, risks of developing diabetic complications associated with future progression to diabetic and cardiovascular disorders due to arteriosclerosis are high. Recently, the number of people who have symptoms of metabolic syndrome, which is manifested as a combination of a plurality of obesity, hyperglycemia based on insulin resistance, hypertension, and hyperlipidemia, is increasing due to lifestyles under the background of high-fat diet and lack of exercise. Currently, these symptoms are becoming a focus of attention as an urgent issue in health insurance because they are perceived to increase the number of people who develop arteriosclerosis over time and eventually result in cardiovascular disorders such as myocardial infarction and cerebrovascular disorders such as cerebral infarction. Both diabetes and metabolic syndrome are attributable to lifestyles, and are strongly correlated with each other.
According to a classification provided by American Diabetes Association (ADA) in 1997 (refer to Nonpatent Literature 2), diabetes and prediabetes that is prone to develop into diabetes are assessed based on the following assessment category using Fasting Plasma Glucose (FPG) and Oral Glucose Tolerance Test (OGTT). Whether a postprandial blood glucose level is high or low is assessed based on blood glucose levels measured over time subsequent to ingestion of solution containing 75 g of glucose in OGTT. Because particularly a 2-hour OGTT value is used in the assessment, 2-hour OGTT values are abbreviated as OGTT below.

Assessment category

When FPG is equal to or more than 126 mg/dl or OGTT is equal to or more than 200 mg/dl, a state is assessed as diabetes.
When FPG is equal to or more than 110 mg/dl and less than 126 mg/dl and OGTT is less than 140 mg/dl, a state is assessed as Impaired Fasting Glucose: IFG.
When FPG is less than 110 mg/dl and OGTT is equal to or more than 140 mg/dl and less than 200 mg/dl, a state is assessed as Impaired Glucose Tolerance: IGT.
When FPG is equal to or more than 110 mg/dl and less than 126 mg/dl and OGTT is equal to or more than 140 mg/dl and less than 200 mg/dl, a state is assessed as IFG/IGT.
When FPG is less than 110 mg/dl and OGTT is less than 140 mg/dl, a state is assessed as Normal Glucose Tolerance (NGT).
According to a categorization provided by Japan Diabetes Society in 1999 (refer to Nonpatent Literature 3), the IFG, IGT, and IFG/IGT are collectively classified as borderline diabetes. While a subject with FPG of less than 110 mg/dl would be regarded as NGT by conventional assessment that only relies on FPG, the subject is categorized, for the first time, into NGT (OGTT of less than 140 mg/dl), IGT (OGTT of equal to or more than 140 mg/dl and less than 200 mg/dl), or diabetes (OGTT of equal to or more than 200 mg/dl) by OGTT measurement.
Large-scale studies have so far elucidated that a group of subjects with IGT/diabetes has a greater risk of cardiovascular disorders compared with a group of subjects with NGT (refer to Nonpatent Literature 4), which has raised awareness of necessity of OGTT measurement. Extraction of a group of subjects with IGT/diabetes that would be missed out if only relying on FPG is important from the viewpoints of early improvement of lifestyles and early treatment.

On the other hand, while importance of OGTT is recognized, a consultation rate for OGTT in mass screening is low. For example, although OGTT measurement is normally offered in an overnight-stay health screening during a comprehensive medical examination, the consultation rate is approximately one-sixth. Further, OGTT measurement is rarely conducted in a workplace health screening. Reasons for this low consultation rate for OGTT include, for example, burden imposed on a subject who receives consultation (such as load with high concentration glucose and prolonged restraint) and operational burden imposed on a person conducting OGTT measurement. In view of the above circumstance, a simple measurement method alternative to OGTT is demanded. This simple measurement method is also useful as a method for pre-screening those who need to have OGTT measured from among subject who receive consultation.

Currently, as a simple assessment method for IGT alternative to OGTT, a method in which the standard value of FPG is lowered from 110 mg/dl to 100 mg/dl (refer to Nonpatent Literature 5) and a method in which FPG and HbA1c are combined (refer to Nonpatent Literature 6) have been proposed. However, because the false-positive rates of these methods are greater than that of OGTT measurement, a problem is posed in terms of discrimination capability. Further, adiponectin, which is a factor of adipokines that are attributable to visceral fat accumulation, has been also known to be correlated with IGT (refer to Nonpatent Literature 7); however, this finding has not yet achieved full reliability in terms of discrimination capability. Furthermore, although amino acids are known to vary in obesity and diabetes (refer to Nonpatent Literature 8 and Nonpatent Literature 9), a concept of IGT had not been introduced at that time yet, and hence, this finding was not intended to be used to discriminate IGT.

As a method for diagnosing a disease state using an amino acid in the blood, an amino index has been known as described in Patent Documents 1 and 2. However, in terms of subjects to be clinically diagnosed, the index is intended to discriminate between hepatitis C and hepatitis C-free in the subject in Patent Document 1, and it is intended to discriminate between healthy subjects and patients with ulcerative colitis as well as healthy subjects and patients with Crohn's disease in Patent Document 2.

The amino acid metabolism is considered to be affected in peripheral tissues due to insulin resistance attributable to visceral fat accumulation, and is considered to be strongly associated with glucose metabolism, lipid metabolism, inflammatory reactions, and redox regulatory mechanisms. Therefore, if an amino acid that varies specifically in, for example, the peripheral blood, in a group of subjects with IGT could be discovered, and an index formula employing a concentration parameter of the varied amino acid could be constructed, such findings are widely applicable as a simple and sensitive test method that reflects underlying metabolic alteration in the group of subjects with IGT.

Nonpatent Literature 1: Homepage of Ministry of Health, Labour and Welfare, Japan, a survey on the actuality of diabetes mellitus, 2002
Nonpatent Literature 2: The Expert Committee on the Diagnosis and Classification of Diabates Mellitus, Diabates Care, 20, 1183 (1997).
Nonpatent Literature 3: KUZUYA, Takeshi et al., Commission report on classification and diagnostic criteria for diabetes mellitus, Journal of the Japan Diabetes Society, 42, 3835 (1999).
Nonpatent Literature 4: Tominaga, M. et al., The Funagata Diabates Study, Diabates Care, 22, 920 (1999).
Nonpatent Literature 5: The Expert Committee on the Diagnosis and Classification of Diabetes Mellitus, Diabates Care, 26, 3160 (2003).
Nonpatent Literature 6: Ko, G. et al., Diabates Care, 21, 1221 (1998).
Nonpatent Literature 7: Wasim, H. et al., Cardiovasc Diabetol., 25, 10 (2006).
Nonpatent Literature 8: Felig, P., Marliss, E., et al., New Engl. J. Med., 281, 811 (1969).
Nonpatent Literature 9: Felig, P., Marliss, E., et al., Diabetes, 19, 727 (1970).
Patent Document 1: WO 2004/052191 Pamphlet
Patent Document 2: WO 2006/098192 Pamphlet

DISCLOSURE OF INVENTION

PROBLEM TO BE SOLVED BY THE INVENTION

However, there is a problem that there are reports on changes of amino acids in obesity or diabetes, and there is no report on a metabolic pattern of amino acids in peripheral blood in an IGT state. Specifically, there is also a problem that there is no report on application to a diagnostic method for a discrimination between 2 groups of an IGT and a NGT. There is a problem that technology of diagnosing the IGT state with a plurality of amino acids as explanatory variables is not developed and not practically used.

The present invention is made in view of the problem described above. An object of the present invention is to provide a method of evaluating IGT, an IGT-evaluating apparatus, an IGT-evaluating method, an IGT-evaluating system, an IGT-evaluating program, and a recording medium, which are capable of evaluating the IGT state accurately by utilizing concentrations of amino acids in blood. An object of the present invention is to provide a method of searching for prophylactic/ameliorating substance for IGT which is capable of searching for prophylactic/ameliorating substance for the IGT efficiently.

MEANS FOR SOLVING PROBLEMS

The present inventors have made extensive study for solving the problems described above, and as a result they have identified amino acids which are useful in the discrimination between the 2 groups of the IGT and the NGT (specifically, the amino acids varying with a statistically significant difference between the 2 groups of the IGT and the NGT), and have found that multivariate discriminants (correlation equations, index formulae) including concentrations of the identified amino acids as explanatory variables correlate significantly with a progress of the IGT state, and the present invention was thereby completed. The present invention is intended to discriminate between the IGT and the NGT. In the present invention, IGT and diabetes, in which OGTT is equal to or more than 140 mg/dl, and which are so-classified by the classification provided by American Diabetes Association in 1997, are subjected to evaluation, and these states are collectively called IGT in the present specification. Further, as described above, because IGT includes symptoms of hyperglycemia based on insulin resistance and accompanying risks of cardiovascular disorders, the present invention is also effective for evaluation and discrimination of these symptoms. Furthermore, the present invention can be used in combination with other biological metabolites, biological indices, and the like as an explanatory variable besides an amino acid. Moreover, the present invention enables selection of an existing animal model that partially reflects a state of IGT and an effective drug in an early stage in a clinical setting by utilizing information on a typical variation pattern in the amino acid concentration in IGT and an index formula corresponding to IGT.

To solve the problems and achieve the objects described above, a method of evaluating IGT according to one aspect of the present invention includes a measuring step of measuring amino acid concentration data on a concentration value of an amino acid in blood collected from a subject to be evaluated, and a concentration value criterion evaluating step of evaluating an impaired glucose tolerance state in the subject based on the amino acid concentration data of the subject measured at the measuring step.

Another aspect of the present invention is the method of evaluating IGT, wherein at the concentration value criterion evaluating step, the impaired glucose tolerance state in the subject is evaluated based on the concentration value of at least one of Glu, Gly, Ser, Ile, Val, Leu, Phe and Asp contained in the amino acid concentration data of the subject measured at the measuring step.

Still another aspect of the present invention is the method of evaluating IGT, wherein the concentration value criterion evaluating step further includes a concentration value criterion discriminating step of discriminating between an impaired glucose tolerance and a normal glucose tolerance in the subject based on the concentration value of at least one of Glu, Gly, Ser, Ile, Val, Leu, Phe and Asp contained in the amino acid concentration data of the subject measured at the measuring step.

Still another aspect of the present invention is the method of evaluating IGT, wherein at the concentration value criterion discriminating step, the discrimination between the impaired glucose tolerance and the normal glucose tolerance in the subject is conducted based on the concentration values of at least two of Glu, Gly, Ser, Ile, Val, Leu, Phe and Asp contained in the amino acid concentration data of the subject measured at the measuring step.

Still another aspect of the present invention is the method of evaluating IGT, wherein the concentration value criterion evaluating step further includes a discriminant value calculating step of calculating a discriminant value that is a value of a multivariate discriminant with a concentration of the amino acid as an explanatory variable, based on both the amino acid concentration data of the subject measured at the measuring step and the previously established multivariate discriminant, and a discriminant value criterion evaluating step of evaluating the impaired glucose tolerance state in the subject based on the discriminant value calculated at the discriminant value calculating step.

Still another aspect of the present invention is the method of evaluating IGT, wherein the multivariate discriminant contains at least one of Glu, Gly, Ser, Ile, Val, Leu, Phe and Asp as the explanatory variable, and wherein at the discriminant value calculating step, the discriminant value is calculated based on both the concentration value of at least one of Glu, Gly, Ser, Ile, Val, Leu, Phe and Asp contained in the amino acid concentration data of the subject measured at the measuring step and the multivariate discriminant.

Still another aspect of the present invention is the method of evaluating IGT, wherein the discriminant value criterion evaluating step further includes a discriminant value criterion discriminating step of discriminating between an impaired glucose tolerance and a normal glucose tolerance in the subject based on the discriminant value calculated at the discriminant value calculating step.

Still another aspect of the present invention is the method of evaluating IGT, wherein the multivariate discriminant contains at least two of Glu, Gly, Ser, Ile, Val, Leu, Phe and Asp as the explanatory variables, and wherein at the discriminant value calculating step, the discriminant value is calculated based on both the concentration values of at least two of Glu, Gly, Ser, Ile, Val, Leu, Phe and Asp contained in the amino acid concentration data of the subject measured at the measuring step and the multivariate discriminant.

Still another aspect of the present invention is the method of evaluating IGT, wherein the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant.

Still another aspect of the present invention is the method of evaluating IGT, wherein the multivariate discriminant is formula 1.

$$\text{Glu}/(\text{His}+\text{Cit})+(\text{Phe}+\text{Tyr})/\text{Gly} \qquad\qquad (\text{formula 1})$$

Still another aspect of the present invention is the method of evaluating IGT, wherein the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree.

Still another aspect of the present invention is the method of evaluating IGT, wherein the multivariate discriminant is the logistic regression equation containing at least Glu and Gly as the explanatory variables.

The present invention also relates to an IGT-evaluating apparatus, the IGT-evaluating apparatus according to one aspect of the present invention includes a control unit and a memory unit to evaluate an impaired glucose tolerance state in a subject to be evaluated. The control unit includes a discriminant value-calculating unit that calculates a discriminant value that is a value of a multivariate discriminant with a concentration of an amino acid as an explanatory variable, based on both previously obtained amino acid concentration data of the subject on a concentration value of the amino acid and the multivariate discriminant stored in the memory unit, and a discriminant value criterion-evaluating unit that evaluates the impaired glucose tolerance state in the subject based on the discriminant value calculated by the discriminant value-calculating unit.

Another aspect of the present invention is the IGT-evaluating apparatus, wherein the multivariate discriminant contains at least one of Glu, Gly, Ser, Ile, Val, Leu, Phe and Asp as the explanatory variable, and wherein the discriminant value-calculating unit calculates the discriminant value based on both the concentration value of at least one of Glu, Gly, Ser, Ile, Val, Leu, Phe and Asp contained in the previously obtained amino acid concentration data of the subject and the multivariate discriminant.

Still another aspect of the present invention is the IGT-evaluating apparatus, wherein the discriminant value criterion-evaluating unit further includes a discriminant value criterion-discriminating unit that discriminates between an impaired glucose tolerance and a normal glucose tolerance in the subject based on the discriminant value calculated by the discriminant value-calculating unit.

Still another aspect of the present invention is the IGT-evaluating apparatus, wherein the multivariate discriminant contains at least two of Glu, Gly, Ser, Ile, Val, Leu, Phe and Asp as the explanatory variables, and wherein the discriminant value-calculating unit calculates the discriminant value based on both the concentration values of at least two of Glu, Gly, Ser, Ile, Val, Leu, Phe and Asp contained in the previously obtained amino acid concentration data of the subject and the multivariate discriminant.

Still another aspect of the present invention is the IGT-evaluating apparatus, wherein the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant.

Still another aspect of the present invention is the IGT-evaluating apparatus, wherein the multivariate discriminant is formula 1.

$$\text{Glu}/(\text{His}+\text{Cit})+(\text{Phe}+\text{Tyr})/\text{Gly} \qquad\qquad (\text{formula 1})$$

Still another aspect of the present invention is the IGT-evaluating apparatus, wherein the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree.

Still another aspect of the present invention is the IGT-evaluating apparatus, wherein the multivariate discriminant is the logistic regression equation containing at least Glu and Gly as the explanatory variables.

Still another aspect of the present invention is the IGT-evaluating apparatus, wherein the control unit further includes a multivariate discriminant-preparing unit that prepares the multivariate discriminant stored in the memory unit, based on impaired glucose tolerance state information stored in the memory unit containing the amino acid concentration data and impaired glucose tolerance state index data on an index for indicating the impaired glucose tolerance state. The multivariate discriminant-preparing unit further includes a candidate multivariate discriminant-preparing unit that prepares a candidate multivariate discriminant that is a candidate of the multivariate discriminant, based on a predetermined discriminant-preparing method from the impaired glucose tolerance state information, a candidate multivariate discriminant-verifying unit that verifies the candidate multivariate discriminant prepared by the candidate multivariate discriminant-preparing unit, based on a predetermined verifying method, and an explanatory variable-selecting unit that selects the explanatory variable of the candidate multivariate discriminant based on a predetermined explanatory variable-selecting method from a verification result obtained by the candidate multivariate discriminant-verifying unit, thereby selecting a combination of the amino acid concentration data contained in the impaired glucose tolerance state information used in preparing the candidate multivariate discriminant. The multivariate discriminant-preparing unit prepares the multivariate discriminant by selecting the candidate multivariate discriminant used as the multivariate discriminant, from a plurality of the candidate multivariate discriminants, based on the verification results accumulated by repeatedly executing the candidate multivariate discriminant-preparing unit, the candidate multivariate discriminant-verifying unit, and the explanatory variable-selecting unit.

The present invention also relates to an IGT-evaluating method, one aspect of the present invention is the IGT-evaluating method of evaluating an impaired glucose tolerance state in a subject to be evaluated. The method is carried out with an information processing apparatus including a control unit and a memory unit. The method includes (i) a discriminant value calculating step of calculating a discriminant value that is a value of a multivariate discriminant with a concentration of an amino acid as an explanatory variable, based on both previously obtained amino acid concentration data of the subject on a concentration value of the amino acid and the multivariate discriminant stored in the memory unit, and (ii) a discriminant value criterion evaluating step of evaluating the impaired glucose tolerance state in the subject based on the discriminant value calculated at the discriminant value calculating step. The steps (i) and (ii) are executed by the control unit.

Another aspect of the present invention is the IGT-evaluating method, wherein the multivariate discriminant contains at least one of Glu, Gly, Ser, Ile, Val, Leu, Phe and Asp as the explanatory variable, and wherein at the discriminant value calculating step, the discriminant value is calculated based on both the concentration value of at least one of Glu, Gly, Ser, Ile, Val, Leu, Phe and Asp contained in the previously obtained amino acid concentration data of the subject and the multivariate discriminant.

Still another aspect of the present invention is the IGT-evaluating method, wherein the discriminant value criterion evaluating step further includes a discriminant value criterion discriminating step of discriminating between an impaired glucose tolerance and a normal glucose tolerance in the subject based on the discriminant value calculated at the discriminant value calculating step.

Still another aspect of the present invention is the IGT-evaluating method, wherein the multivariate discriminant contains at least two of Glu, Gly, Ser, Ile, Val, Leu, Phe and Asp as the explanatory variables, and wherein at the discriminant value calculating step, the discriminant value is calculated based on both the concentration values of at least two of Glu, Gly, Ser, Ile, Val, Leu, Phe and Asp contained in the previously obtained amino acid concentration data of the subject and the multivariate discriminant.

Still another aspect of the present invention is the IGT-evaluating method, wherein the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant.

Still another aspect of the present invention is the IGT-evaluating method, wherein the multivariate discriminant is formula 1.

$$Glu/(His+Cit)+(Phe+Tyr)/Gly \qquad \text{(formula 1)}$$

Still another aspect of the present invention is the IGT-evaluating method, wherein the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree.

Still another aspect of the present invention is the IGT-evaluating method, wherein the multivariate discriminant is the logistic regression equation containing at least Glu and Gly as the explanatory variables.

Still another aspect of the present invention is the IGT-evaluating method, wherein the method further includes a multivariate discriminant preparing step of preparing the multivariate discriminant stored in the memory unit, based on impaired glucose tolerance state information stored in the memory unit containing the amino acid concentration data and impaired glucose tolerance state index data on an index for indicating the impaired glucose tolerance state. The multivariate discriminant preparing step is executed by the control unit. The multivariate discriminant preparing step further includes a candidate multivariate discriminant preparing step of preparing a candidate multivariate discriminant that is a candidate of the multivariate discriminant, based on a predetermined discriminant-preparing method from the impaired glucose tolerance state information, a candidate multivariate discriminant verifying step of verifying the candidate multivariate discriminant prepared at the candidate multivariate discriminant preparing step, based on a predetermined verifying method, and an explanatory variable selecting step of selecting the explanatory variable of the candidate multivariate discriminant based on a predetermined explanatory variable-selecting method from a verification result obtained at the candidate multivariate discriminant verifying step, thereby selecting a combination of the amino acid concentration data contained in the impaired glucose tolerance state information used in preparing the candidate multivariate discriminant. At the multivariate discriminant preparing step, the multivariate discriminant is prepared by selecting the candidate multivariate discriminant used as the multivariate discriminant from a plurality of the candidate multivariate discriminants, based on the verification results accumulated by repeatedly executing the candidate multivariate discriminant preparing step, the candidate multivariate discriminant verifying step, and the explanatory variable selecting step.

The present invention also relates to an IGT-evaluating system, the IGT-evaluating system according to one aspect of the present invention includes an IGT-evaluating apparatus including a control unit and a memory unit to evaluate an impaired glucose tolerance state in a subject to be evaluated and an information communication terminal apparatus that provides amino acid concentration data of the subject on a concentration value of an amino acid connected to each other communicatively via a network. The information communication terminal apparatus includes an amino acid concentration data-sending unit that transmits the amino acid concentration data of the subject to the IGT-evaluating apparatus, and an evaluation result-receiving unit that receives an evaluation result on the impaired glucose tolerance state of the subject transmitted from the IGT-evaluating apparatus. The control unit of the IGT-evaluating apparatus includes an amino acid concentration data-receiving unit that receives the amino acid concentration data of the subject transmitted from the information communication terminal apparatus, a discriminant value-calculating unit that calculates a discriminant value that is a value of a multivariate discriminant with a concentration of the amino acid as an explanatory variable, based on both the amino acid concentration data of the subject received by the amino acid concentration data-receiving unit and the multivariate discriminant stored in the memory unit, a discriminant value criterion-evaluating unit that evaluates the impaired glucose tolerance state in the subject based on the discriminant value calculated by the discriminant value-calculating unit, and an evaluation result-sending unit that transmits the evaluation result of the subject obtained by the discriminant value criterion-evaluating unit to the information communication terminal apparatus.

Another aspect of the present invention is the IGT-evaluating system, wherein the multivariate discriminant contains at least one of Glu, Gly, Ser, Ile, Val, Leu, Phe and Asp as the explanatory variable, and wherein the discriminant value-calculating unit calculates the discriminant value based on both the concentration value of at least one of Glu, Gly, Ser, Ile, Val, Leu, Phe and Asp contained in the amino acid concentration data of the subject received by the amino acid concentration data-receiving unit and the multivariate discriminant.

Still another aspect of the present invention is the IGT-evaluating system, wherein the discriminant value criterion-evaluating unit further includes a discriminant value criterion-discriminating unit that discriminates between an impaired glucose tolerance and a normal glucose tolerance in the subject based on the discriminant value calculated by the discriminant value-calculating unit.

Still another aspect of the present invention is the IGT-evaluating system, wherein the multivariate discriminant contains at least two of Glu, Gly, Ser, Ile, Val, Leu, Phe and Asp as the explanatory variables, and wherein the discriminant value-calculating unit calculates the discriminant value based on both the concentration values of at least two of Glu, Gly, Ser, Ile, Val, Leu, Phe and Asp contained in the amino acid concentration data of the subject received by the amino acid concentration data-receiving unit and the multivariate discriminant.

Still another aspect of the present invention is the IGT-evaluating system, wherein the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant.

Still another aspect of the present invention is the IGT-evaluating system, wherein the multivariate discriminant is formula 1.

```
Glu/(His+Cit)+(Phe+Tyr)/Gly                    (formula 1)
```

Still another aspect of the present invention is the IGT-evaluating system, wherein the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree.

Still another aspect of the present invention is the IGT-evaluating system, wherein the multivariate discriminant is the logistic regression equation containing at least Glu and Gly as the explanatory variables.

Still another aspect of the present invention is the IGT-evaluating system, wherein the control unit of the IGT-evaluating apparatus further includes a multivariate discriminant-preparing unit that prepares the multivariate discriminant stored in the memory unit, based on impaired glucose tolerance state information stored in the memory unit containing the amino acid concentration data and impaired glucose tolerance state index data on an index for indicating the impaired glucose tolerance state. The multivariate discriminant-preparing unit further includes a candidate multivariate discriminant-preparing unit that prepares a candidate multivariate discriminant that is a candidate of the multivariate discriminant, based on a predetermined discriminant-preparing method from the impaired glucose tolerance state information, a candidate multivariate discriminant-verifying unit that verifies the candidate multivariate discriminant prepared by the candidate multivariate discriminant-preparing unit, based on a predetermined verifying method, and an explanatory variable-selecting unit that selects the explanatory variable of the candidate multivariate discriminant based on a predetermined explanatory variable-selecting method from a verification result obtained by the candidate multivariate discriminant-verifying unit, thereby selecting a combination of the amino acid concentration data contained in the impaired glucose tolerance state information used in preparing the candidate multivariate discriminant. The multivariate discriminant-preparing unit prepares the multivariate discriminant by selecting the candidate multivariate discriminant used as the multivariate discriminant, from a plurality of the candidate multivariate discriminants, based on the verification results accumulated by repeatedly executing the candidate multivariate discriminant-preparing unit, the candidate multivariate discriminant-verifying unit, and the explanatory variable-selecting unit.

The present invention also relates to an IGT-evaluating program product, one aspect of the present invention is the IGT-evaluating program product that makes an information processing apparatus including a control unit and a memory unit execute a method of evaluating an impaired glucose tolerance state in a subject to be evaluated. The method includes (i) a discriminant value calculating step of calculating a discriminant value that is a value of a multivariate discriminant with a concentration of an amino acid as an explanatory variable, based on both previously obtained amino acid concentration data of the subject on a concentration value of the amino acid and the multivariate discriminant stored in the memory unit, and (ii) a discriminant value criterion evaluating step of evaluating the impaired glucose tolerance state in the subject based on the discriminant value calculated at the discriminant value calculating step. The steps (i) and (ii) are executed by the control unit.

Another aspect of the present invention is the IGT-evaluating program product, wherein the multivariate discriminant contains at least one of Glu, Gly, Ser, Ile, Val, Leu, Phe and Asp as the explanatory variable, and wherein at the discriminant value calculating step, the discriminant value is calculated based on both the concentration value of at least one of Glu, Gly, Ser, Ile, Val, Leu, Phe and Asp contained in the previously obtained amino acid concentration data of the subject and the multivariate discriminant.

Still another aspect of the present invention is the IGT-evaluating program product, wherein the discriminant value criterion evaluating step further includes a discriminant value criterion discriminating step of discriminating between an impaired glucose tolerance and a normal glucose tolerance in the subject based on the discriminant value calculated at the discriminant value calculating step.

Still another aspect of the present invention is the IGT-evaluating program product, wherein the multivariate discriminant contains at least two of Glu, Gly, Ser, Ile, Val, Leu, Phe and Asp as the explanatory variables, and wherein at the discriminant value calculating step, the discriminant value is calculated based on both the concentration values of at least two of Glu, Gly, Ser, Ile, Val, Leu, Phe and Asp contained in the previously obtained amino acid concentration data of the subject and the multivariate discriminant.

Still another aspect of the present invention is the IGT-evaluating program product, wherein the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant.

Still another aspect of the present invention is the IGT-evaluating program product, wherein the multivariate discriminant is formula 1.

$$Glu/(His+Cit)+(Phe+Tyr)/Gly \qquad \text{(formula 1)}$$

Still another aspect of the present invention is the IGT-evaluating program product, wherein the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree.

Still another aspect of the present invention is the IGT-evaluating program product, wherein the multivariate discriminant is the logistic regression equation containing at least Glu and Gly as the explanatory variables.

Still another aspect of the present invention is the IGT-evaluating program product, wherein the method further includes a multivariate discriminant preparing step of preparing the multivariate discriminant stored in the memory unit, based on impaired glucose tolerance state information stored in the memory unit containing the amino acid concentration data and impaired glucose tolerance state index data on an index for indicating the impaired glucose tolerance state. The multivariate discriminant preparing step is executed by the control unit. The multivariate discriminant preparing step further includes a candidate multivariate discriminant preparing step of preparing a candidate multivariate discriminant that is a candidate of the multivariate discriminant, based on a predetermined discriminant-preparing method from the impaired glucose tolerance state information, a candidate multivariate discriminant verifying step of verifying the candidate multivariate discriminant prepared at the candidate multivariate discriminant preparing step, based on a predetermined verifying method, and an explanatory variable selecting step of selecting the explanatory variable of the candidate multivariate discriminant based on a predetermined explanatory variable-selecting method from a verification result obtained at the candidate multivariate discriminant verifying step, thereby selecting a combination of the amino acid concentration data contained in the impaired glucose tolerance state information used in preparing the candidate multivariate discriminant. At the multivariate discriminant preparing step, the multivariate discriminant is prepared by selecting the candidate multivariate discriminant used as the multivariate discriminant from a plurality of the candidate multivariate discriminants, based on the verification results accumulated by repeatedly executing the candidate multivariate discriminant preparing step, the candidate multivariate discriminant verifying step, and the explanatory variable selecting step.

The present invention also relates to a recording medium, the recording medium according to one aspect of the present invention includes the IGT-evaluating program product described above.

The present invention also relates to a method of searching for prophylactic/ameliorating substance for IGT. One aspect of the present invention is the method of searching for prophylactic/ameliorating substance for IGT, wherein the method includes a measuring step of measuring amino acid concentration data on a concentration value of an amino acid in blood collected from a subject to be evaluated to which a desired substance group consisting of one or more substances that prevent an impaired glucose tolerance or ameliorate an impaired glucose tolerance state has been administered, a concentration value criterion evaluating step of evaluating an impaired glucose tolerance state in the subject based on the amino acid concentration data measured at the measuring step, and a judging step of judging whether or not the desired substance group prevents the impaired glucose tolerance or ameliorates the impaired glucose tolerance state, based on an evaluation result obtained at the concentration value criterion evaluating step.

Another aspect of the present invention is the method of searching for prophylactic/ameliorating substance for IGT, wherein at the concentration value criterion evaluating step, the impaired glucose tolerance state in the subject is evaluated based on the concentration value of at least one of Glu, Gly, Ser, Ile, Val, Leu, Phe and Asp contained in the amino acid concentration data of the subject measured at the measuring step.

Still another aspect of the present invention is the method of searching for prophylactic/ameliorating substance for IGT, wherein the concentration value criterion evaluating step further includes a concentration value criterion discriminating step of discriminating between an impaired glucose tolerance and a normal glucose tolerance in the subject based on the concentration value of at least one of Glu, Gly, Ser, Ile, Val, Leu, Phe and Asp contained in the amino acid concentration data of the subject measured at the measuring step.

Still another aspect of the present invention is the method of searching for prophylactic/ameliorating substance for IGT, wherein at the concentration value criterion discriminating step, the discrimination between the impaired glucose tolerance and the normal glucose tolerance in the subject is conducted based on the concentration values of at least two of Glu, Gly, Ser, Ile, Val, Leu, Phe and Asp contained in the amino acid concentration data of the subject measured at the measuring step.

Still another aspect of the present invention is the method of searching for prophylactic/ameliorating substance for IGT, wherein the concentration value criterion evaluating step further includes a discriminant value calculating step of calculating a discriminant value that is a value of a multivariate discriminant with a concentration of the amino acid as an explanatory variable, based on both the amino acid concentration data of the subject measured at the measuring step and the previously established multivariate discriminant, and a discriminant value criterion evaluating step of evaluating the impaired glucose tolerance state in the subject based on the discriminant value calculated at the discriminant value calculating step.

Still another aspect of the present invention is the method of searching for prophylactic/ameliorating substance for IGT, wherein the multivariate discriminant contains at least one of Glu, Gly, Ser, Ile, Val, Leu, Phe and Asp as the explanatory variable, and wherein at the discriminant value calculating step, the discriminant value is calculated based on both the concentration value of at least one of Glu, Gly, Ser, Ile, Val, Leu, Phe and Asp contained in the amino acid concentration data of the subject measured at the measuring step and the multivariate discriminant.

Still another aspect of the present invention is the method of searching for prophylactic/ameliorating substance for IGT, wherein the discriminant value criterion evaluating step further includes a discriminant value criterion discriminating step of discriminating between an impaired glucose tolerance and a normal glucose tolerance in the subject based on the discriminant value calculated at the discriminant value calculating step.

Still another aspect of the present invention is the method of searching for prophylactic/ameliorating substance for IGT, wherein the multivariate discriminant contains at least two of Glu, Gly, Ser, Ile, Val, Leu, Phe and Asp as the explanatory variables, and wherein at the discriminant value calculating step, the discriminant value is calculated based on both the concentration values of at least two of Glu, Gly, Ser, Ile, Val, Leu, Phe and Asp contained in the amino acid concentration data of the subject measured at the measuring step and the multivariate discriminant.

Still another aspect of the present invention is the method of searching for prophylactic/ameliorating substance for IGT, wherein the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant.

Still another aspect of the present invention is the method of searching for prophylactic/ameliorating substance for IGT, wherein the multivariate discriminant is formula 1.

$$Glu/(His+Cit)+(Phe+Tyr)/Gly \qquad (formula\ 1)$$

Still another aspect of the present invention is the method of searching for prophylactic/ameliorating substance for IGT, wherein the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree.

Still another aspect of the present invention is the method of searching for prophylactic/ameliorating substance for IGT, wherein the multivariate discriminant is the logistic regression equation containing at least Glu and Gly as the explanatory variables.

EFFECTS OF THE INVENTION

According to the method of evaluating IGT of the present invention, amino acid concentration data on a concentration value of an amino acid in blood collected from a subject to be evaluated is measured, and an IGT state in the subject is evaluated based on the measured amino acid concentration data of the subject. Thus, concentrations of amino acids in blood can be utilized to bring about an effect of enabling an accurate evaluation of the IGT state.

According to the method of evaluating IGT of the present invention, the IGT state in the subject is evaluated based on the concentration value of at least one of Glu, Gly, Ser, Ile, Val, Leu, Phe and Asp contained in the measured amino acid concentration data of the subject. Thus, concentrations of amino acids which among amino acids in blood, are associated with the IGT state can be utilized to bring about an effect of enabling an accurate evaluation of the IGT state.

According to the method of evaluating IGT of the present invention, a discrimination between an IGT and a NGT in the subject is conducted based on the concentration value of at least one of Glu, Gly, Ser, Ile, Val, Leu, Phe and Asp contained in the measured amino acid concentration data of the subject. Thus, concentrations of amino acids which among amino acids in blood, are useful for discriminating between the 2 groups of the IGT and the NGT can be utilized to bring about an effect of enabling an accurate discrimination between the 2 groups of the IGT and the NGT.

According to the method of evaluating IGT of the present invention, the discrimination between the IGT and the NGT in the subject is conducted based on the concentration values of at least two of Glu, Gly, Ser, Ile, Val, Leu, Phe and Asp contained in the measured amino acid concentration data of the subject. Thus, concentrations of amino acids which among amino acids in blood, are useful for discriminating between the 2 groups of the IGT and the NGT can be utilized to bring about an effect of enabling an accurate discrimination between the 2 groups of the IGT and the NGT.

According to the method of evaluating IGT of the present invention, a discriminant value that is a value of a multivariate discriminant with a concentration of the amino acid as an explanatory variable is calculated based on both the measured amino acid concentration data of the subject and the previously established multivariate discriminant, and the IGT state in the subject is evaluated based on the calculated discriminant value. Thus, discriminant values obtained in multivariate discriminants with concentrations of amino acids as explanatory variables can be utilized to bring about an effect of enabling an accurate evaluation of the IGT state.

According to the method of evaluating IGT of the present invention, the discriminant value is calculated based on both the concentration value of at least one of Glu, Gly, Ser, Ile, Val, Leu, Phe and Asp contained in the measured amino acid concentration data of the subject and the multivariate discriminant containing at least one of Glu, Gly, Ser, Ile, Val, Leu, Phe and Asp as the explanatory variable. Thus, discriminant values obtained in multivariate discriminants which are correlated with the IGT state significantly can be utilized to bring about an effect of enabling an accurate evaluation of the IGT state.

According to the method of evaluating IGT of the present invention, a discrimination between an IGT and a NGT in the subject is conducted based on the calculated discriminant value. Thus, discriminant values obtained in multivariate discriminants useful for discriminating between the 2 groups of the IGT and the NGT can be utilized to bring about an effect of enabling an accurate discrimination between the 2 groups of the IGT and the NGT.

According to the method of evaluating IGT of the present invention, the discriminant value is calculated based on both the concentration values of at least two of Glu, Gly, Ser, Ile, Val, Leu, Phe and Asp contained in the measured amino acid concentration data of the subject and the multivariate discriminant containing at least two of Glu, Gly, Ser, Ile, Val, Leu, Phe and Asp as the explanatory variables. Thus, discriminant values obtained in multivariate discriminants useful for discriminating between the 2 groups of the IGT and the NGT can be utilized to bring about an effect of enabling an accurate discrimination between the 2 groups of the IGT and the NGT.

According to the method of evaluating IGT of the present invention, the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the method of evaluating IGT of the present invention, the multivariate discriminant is formula 1. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

$$Glu/(His+Cit)+(Phe+Tyr)/Gly \qquad (formula\ 1)$$

According to the method of evaluating IGT of the present invention, the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the method of evaluating IGT of the present invention, the multivariate discriminant is the logistic regression equation containing at least Glu and Gly as the explanatory variables. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the IGT-evaluating apparatus, the IGT-evaluating method, and the IGT-evaluating program of the present invention, a discriminant value that is a value of a multivariate discriminant with a concentration of an amino acid as an explanatory variable is calculated based on both previously obtained amino acid concentration data of the subject on a concentration value of the amino acid and the previously established multivariate discriminant, and an IGT state in the subject is evaluated based on the calculated discriminant value. Thus, discriminant values obtained in multivariate discriminants with concentrations of amino acids as explanatory variables can be utilized to bring about an effect of enabling an accurate evaluation of the IGT state.

According to the IGT-evaluating apparatus, the IGT-evaluating method, and the IGT-evaluating program of the present invention, the discriminant value is calculated based on both the concentration value of at least one of Glu, Gly, Ser, Ile, Val, Leu, Phe and Asp contained in the previously obtained amino acid concentration data of the subject and the multivariate discriminant containing at least one of Glu, Gly, Ser, Ile, Val, Leu, Phe and Asp as the explanatory variable. Thus, discriminant values obtained in multivariate discriminants which are correlated with the IGT state significantly can be utilized to bring about an effect of enabling an accurate evaluation of the IGT state.

According to the IGT-evaluating apparatus, the IGT-evaluating method, and the IGT-evaluating program of the present invention, a discrimination between an IGT and a NGT in the subject is conducted based on the calculated discriminant value. Thus, discriminant values obtained in multivariate discriminants useful for discriminating between the 2 groups of the IGT and the NGT can be utilized to bring about an effect of enabling an accurate discrimination between the 2 groups of the IGT and the NGT.

According to the IGT-evaluating apparatus, the IGT-evaluating method, and the IGT-evaluating program of the present invention, the discriminant value is calculated based on both the concentration values of at least two of Glu, Gly, Ser, Ile, Val, Leu, Phe and Asp contained in the previously obtained amino acid concentration data of the subject and the multivariate discriminant containing at least two of Glu, Gly, Ser, Ile, Val, Leu, Phe and Asp as the explanatory variables. Thus, discriminant values obtained in multivariate discriminants useful for discriminating between the 2 groups of the IGT and the NGT can be utilized to bring about an effect of enabling an accurate discrimination between the 2 groups of the IGT and the NGT.

According to the IGT-evaluating apparatus, the IGT-evaluating method, and the IGT-evaluating program of the present invention, the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the IGT-evaluating apparatus, the IGT-evaluating method, and the IGT-evaluating program of the present invention, the multivariate discriminant is formula 1. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

$$\mathrm{Glu/(His+Cit)+(Phe+Tyr)/Gly} \qquad \mathrm{(formula\ 1)}$$

According to the IGT-evaluating apparatus, the IGT-evaluating method, and the IGT-evaluating program of the present invention, the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the IGT-evaluating apparatus, the IGT-evaluating method, and the IGT-evaluating program of the present invention, the multivariate discriminant is the logistic regression equation containing at least Glu and Gly as the explanatory variables. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the IGT-evaluating apparatus, the IGT-evaluating method, and the IGT-evaluating program of the present invention, the multivariate discriminant stored in the memory unit is prepared based on IGT state information stored in the memory unit containing the amino acid concentration data and IGT state index data on an index for indicating the IGT state. Specifically, (1) a candidate multivariate discriminant that is a candidate of the multivariate discriminant is prepared based on a predetermined discriminant-preparing method from the IGT state information, (2) the prepared candidate multivariate discriminant is verified based on a predetermined verifying method, (3) the explanatory variable of the candidate multivariate discriminant is selected based on a predetermined explanatory variable-selecting method from a verification result obtained by executing (2), thereby selecting a combination of the amino acid concentration data contained in the IGT state information used in preparing the candidate multivariate discriminant, and (4) the candidate multivariate discriminant used as the multivariate discriminant is selected from a plurality of the candidate multivariate discriminants based on the verification results accumulated by repeatedly executing (1), (2) and (3), thereby preparing the multivariate discriminant. Thus, there can be brought about an effect of enabling a preparation of multivariate discriminants most appropriate for evaluating the IGT state (specifically, multivariate discriminants correlating significantly with the IGT state (more specifically, multivariate discriminants useful for discriminating between the 2 groups of the IGT and the NGT)).

According to the IGT-evaluating system of the present invention, an information communication terminal apparatus first transmits amino acid concentration data of a subject to be evaluated to an IGT-evaluating apparatus. The IGT-evaluating apparatus receives the amino acid concentration data of the subject transmitted from the information communication terminal apparatus, calculates a discriminant value that is a value of a multivariate discriminant with a concentration of an amino acid as an explanatory variable, based on both the received amino acid concentration data of the subject and the multivariate discriminant stored in a memory unit, and evaluates an IGT state in the subject based on the calculated discriminant value, and transmits an evaluation result on the IGT state of the subject to the information communication terminal apparatus. Then, the information communication terminal apparatus receives the evaluation result of the subject transmitted from the IGT-evaluating apparatus. Thus, discriminant values obtained in multivariate discriminants with concentrations of amino acids as explanatory variables can be utilized to bring about an effect of enabling an accurate evaluation of the IGT state.

According to the IGT-evaluating system of the present invention, the discriminant value is calculated based on both the concentration value of at least one of Glu, Gly, Ser, Ile, Val, Leu, Phe and Asp contained in the recieved amino acid concentration data of the subject and the multivariate discriminant containing at least one of Glu, Gly, Ser, Ile, Val, Leu, Phe and Asp as the explanatory variable. Thus, discriminant values obtained in multivariate discriminants which are correlated with the IGT state significantly can be utilized to bring about an effect of enabling an accurate evaluation of the IGT state.

According to the IGT-evaluating system of the present invention, a discrimination between an IGT and a NGT in the subject is conducted based on the calculated discriminant value. Thus, discriminant values obtained in multivariate discriminants useful for discriminating between the 2 groups of the IGT and the NGT can be utilized to bring about an effect of enabling an accurate discrimination between the 2 groups of the IGT and the NGT.

According to the IGT-evaluating system of the present invention, the discriminant value is calculated based on both the concentration values of at least two of Glu, Gly, Ser, Ile, Val, Leu, Phe and Asp contained in the recieved amino acid concentration data of the subject and the multivariate discriminant containing at least two of Glu, Gly, Ser, Ile, Val, Leu, Phe and Asp as the explanatory variables. Thus, discriminant values obtained in multivariate discriminants useful for discriminating between the 2 groups of the IGT and the NGT can be utilized to bring about an effect of enabling an accurate discrimination between the 2 groups of the IGT and the NGT.

According to the IGT-evaluating system of the present invention, the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the IGT-evaluating system of the present invention, the multivariate discriminant is formula 1.

Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

```
Glu/(His+Cit)+(Phe+Tyr)/Gly                    (formula 1)
```

According to the IGT-evaluating system of the present invention, the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the IGT-evaluating system of the present invention, the multivariate discriminant is the logistic regression equation containing at least Glu and Gly as the explanatory variables. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the IGT-evaluating system of the present invention, the multivariate discriminant stored in the memory unit is prepared based on IGT state information stored in the memory unit containing the amino acid concentration data and IGT state index data on an index for indicating the IGT state. Specifically, (1) a candidate multivariate discriminant that is a candidate of the multivariate discriminant is prepared based on a predetermined discriminant-preparing method from the IGT state information, (2) the prepared candidate multivariate discriminant is verified based on a predetermined verifying method, (3) the explanatory variable of the candidate multivariate discriminant is selected based on a predetermined explanatory variable-selecting method from a verification result obtained by executing (2), thereby selecting a combination of the amino acid concentration data contained in the IGT state information used in preparing the candidate multivariate discriminant, and (4) the candidate multivariate discriminant used as the multivariate discriminant is selected from a plurality of the candidate multivariate discriminants based on the verification results accumulated by repeatedly executing (1), (2) and (3), thereby preparing the multivariate discriminant. Thus, there can be brought about an effect of enabling a preparation of multivariate discriminants most appropriate for evaluating the IGT state (specifically, multivariate discriminants correlating significantly with the IGT state (more specifically, multivariate discriminants useful for discriminating between the 2 groups of the IGT and the NGT)).

According to the recording medium of the present invention, the IGT-evaluating program recorded on the recording medium is read and executed by the computer, thereby allowing the computer to execute the IGT-evaluating program, thus bringing about an effect of obtaining the same effect as in the IGT-evaluating program.

According to the method of searching for prophylactic/ameliorating substance for IGT of the present invention, amino acid concentration data on a concentration value of an amino acid is measured in blood collected from a subject to be evaluated to which a desired substance group has been administered, an IGT state in the subject is evaluated based on the measured amino acid concentration data, and whether or not the desired substance group prevents an IGT or ameliorates an IGT state is judged based on an evaluation result. Thus, the method of evaluating IGT capable of accurately evaluating the IGT state by utilizing concentrations of amino acids in blood can be used to bring about an effect of enabling an accurate search for a substance for preventing the IGT or ameliorating the IGT state. According to the method of searching for prophylactic/ameliorating substance for IGT of the present invention, information on amino acid concentration variation pattern typical of the IGT or a multivariate discriminant corresponding to the IGT can be used for selecting a clinically effective chemical at an early stage or an existing animal model partially reflecting the IGT state.

According to the method of searching for prophylactic/ameliorating substance for IGT of the present invention, the IGT state in the subject is evaluated based on the concentration value of at least one of Glu, Gly, Ser, Ile, Val, Leu, Phe and Asp contained in the measured amino acid concentration data of the subject. Thus, concentrations of amino acids which among amino acids in blood, are associated with the IGT state can be utilized to bring about an effect of enabling an accurate evaluation of the IGT state.

According to the method of searching for prophylactic/ameliorating substance for IGT of the present invention, a discrimination between an IGT and a NGT in the subject is conducted based on the concentration value of at least one of Glu, Gly, Ser, Ile, Val, Leu, Phe and Asp contained in the measured amino acid concentration data of the subject. Thus, concentrations of amino acids which among amino acids in blood, are useful for discriminating between the 2 groups of the IGT and the NGT can be utilized to bring about an effect of enabling an accurate discrimination between the 2 groups of the IGT and the NGT.

According to the method of searching for prophylactic/ameliorating substance for IGT of the present invention, the discrimination between the IGT and the NGT in the subject is conducted based on the concentration values of at least two of Glu, Gly, Ser, Ile, Val, Leu, Phe and Asp contained in the measured amino acid concentration data of the subject. Thus, concentrations of amino acids which among amino acids in blood, are useful for discriminating between the 2 groups of the IGT and the NGT can be utilized to bring about an effect of enabling an accurate discrimination between the 2 groups of the IGT and the NGT.

According to the method of searching for prophylactic/ameliorating substance for IGT of the present invention, a discriminant value that is a value of a multivariate discriminant with a concentration of the amino acid as an explanatory variable is calculated based on both the measured amino acid concentration data of the subject and the previously established multivariate discriminant, and the IGT state in the subject is evaluated based on the calculated discriminant value. Thus, discriminant values obtained in multivariate discriminants with concentrations of amino acids as explanatory variables can be utilized to bring about an effect of enabling an accurate evaluation of the IGT state.

According to the method of searching for prophylactic/ameliorating substance for IGT of the present invention, the discriminant value is calculated based on both the concentration value of at least one of Glu, Gly, Ser, Ile, Val, Leu, Phe and Asp contained in the measured amino acid concentration data of the subject and the multivariate discriminant containing at least one of Glu, Gly, Ser, Ile, Val, Leu, Phe and Asp as the explanatory variable. Thus, discriminant values obtained in multivariate discriminants which are correlated with the IGT state significantly can be utilized to bring about an effect of enabling an accurate evaluation of the IGT state.

According to the method of searching for prophylactic/ameliorating substance for IGT of the present invention, a discrimination between an IGT and a NGT in the subject is conducted based on the calculated discriminant value. Thus, discriminant values obtained in multivariate discriminants useful for discriminating between the 2 groups of the IGT and the NGT can be utilized to bring about an effect of enabling an accurate discrimination between the 2 groups of the IGT and the NGT.

According to the method of searching for prophylactic/ameliorating substance for IGT of the present invention, the discriminant value is calculated based on both the concentration values of at least two of Glu, Gly, Ser, Ile, Val, Leu, Phe and Asp contained in the measured amino acid concentration data of the subject and the multivariate discriminant containing at least two of Glu, Gly, Ser, Ile, Val, Leu, Phe and Asp as the explanatory variables. Thus, discriminant values obtained in multivariate discriminants useful for discriminating between the 2 groups of the IGT and the NGT can be utilized to bring about an effect of enabling an accurate discrimination between the 2 groups of the IGT and the NGT.

According to the method of searching for prophylactic/ameliorating substance for IGT of the present invention, the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the method of searching for prophylactic/ameliorating substance for IGT of the present invention, the multivariate discriminant is formula 1. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT. Glu/(His+Cit)+(Phe+Tyr)/Gly (formula 1)

According to the method of searching for prophylactic/ameliorating substance for IGT of the present invention, the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

According to the method of searching for prophylactic/ameliorating substance for IGT of the present invention, the multivariate discriminant is the logistic regression equation containing at least Glu and Gly as the explanatory variables. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

When the IGT state is evaluated (specifically, the discrimination between the IGT group and the NGT group is conducted) in the present invention, concentrations of other metabolites (biological metabolites), protein expression level, age and sex of the subject, biological indices or the like may be used in addition to the concentrations of the amino acids. When the IGT state is evaluated (specifically, the discrimination between the IGT group and the NGT group is conducted) in the present invention, concentrations of other metabolites (biological metabolites), protein expression level, age and sex of the subject, biological indices or the like may be used as the explanatory variables in the multivariate discriminants in addition to the concentrations of the amino acids.

The IGT includes symptoms of hyperglycemia based on insulin resistance and accompanying risks of cardiovascular disorders, and thus the present invention is also effective in evaluation or discrimination thereof.

BRIEF DESCRIPTION OF THE DRAWINGS

FIG. 1 is a principle configurational diagram showing a basic principle of the present invention;

FIG. 2 is a flowchart showing one example of a method of evaluating IGT according to a first embodiment;

FIG. 3 is a principle configurational diagram showing a basic principle of the present invention;

FIG. 4 is a diagram showing an example of an entire configuration of a present system;

FIG. 5 is a diagram showing another example of an entire configuration of the present system;

FIG. 6 is a block diagram showing an example of a configuration of an IGT-evaluating apparatus 100 in the present system;

FIG. 7 is a chart showing an example of information stored in a user information file 106a;

FIG. 8 is a chart showing an example of information stored in an amino acid concentration data file 106b;

FIG. 9 is a chart showing an example of information stored in an IGT state information file 106c;

FIG. 10 is a chart showing an example of information stored in a designated IGT state information file 106d;

FIG. 11 is a chart showing an example of information stored in a candidate multivariate discriminant file 106e1;

FIG. 12 is a chart showing an example of information stored in a verification result file 106e2;

FIG. 13 is a chart showing an example of information stored in a selected IGT state information file 106e3;

FIG. 14 is a chart showing an example of information stored in a multivariate discriminant file 106e4;

FIG. 15 is a chart showing an example of information stored in a discriminant value file 106f;

FIG. 16 is a chart showing an example of information stored in an evaluation result file 106g;

FIG. 17 is a block diagram showing a configuration of a multivariate discriminant-preparing part 102h;

FIG. 18 is a block diagram showing a configuration of a discriminant value criterion-evaluating part 102j;

FIG. 19 is a block diagram showing an example of a configuration of a client apparatus 200 in the present system;

FIG. 20 is a block diagram showing an example of a configuration of a database apparatus 400 in the present system;

FIG. 21 is a flowchart showing an example of an IGT evaluation service processing performed in the present system;

FIG. 22 is a flowchart showing an example of a multivariate discriminant-preparing processing performed in the IGT-evaluating apparatus 100 in the present system;

FIG. 23 is a principle configurational diagram showing a basic principle of the present invention;

FIG. 24 is a flowchart showing one example of a method of searching for prophylactic/ameliorating substance for IGT according to a third embodiment;

FIG. 25 is boxplots showing distributions of amino acid explanatory variables between 2 groups of NGT and IGT;

FIG. 26 is a graph showing ROC curve for an evaluation of a discrimination capability between 2 groups;

FIG. 27 is a graph showing ROC curve for an evaluation of a discrimination capability between 2 groups;

FIG. 28 is a chart showing a list of AUCs of ROC curves for an evaluation of a discrimination capability between 2 groups;

FIG. 29 is a chart showing a list of AUCs of ROC curves for an evaluation of a discrimination capability between 2 groups;

FIG. 30 is a chart showing a list of AUCs of ROC curves for an evaluation of a discrimination capability between 2 groups;

FIG. 31 is a chart showing a list of AUCs of ROC curves for an evaluation of a discrimination capability between 2 groups;

FIG. 32 is a chart showing a list of logistic regression equations;

FIG. 33 is a chart showing a list of logistic regression equations;

FIG. 34 is a diagram showing discriminatory conditions of a discrimination between 2 groups of NGT and IGT;

FIG. 35 is a chart showing a list of AUCs of ROC curves for an evaluation of a discrimination capability between 2 groups;

FIG. 36 is a chart showing a list of AUCs of ROC curves for an evaluation of a discrimination capability between 2 groups;

FIG. 37 is a chart showing a list of AUCs of ROC curves and fractional expressions with real coefficients;

FIG. 38 is a chart showing a list of AUCs of ROC curves and fractional expressions with real coefficients;

FIG. 39 is a chart showing a list of AUCs of ROC curves and logistic regression equations containing numerical coefficients; and

FIG. 40 is a chart showing a list of AUCs of ROC curves and logistic regression equations containing numerical coefficients.

EXPLANATION OF LETTERS OR NUMERALS

100 IGT-evaluating apparatus
102 Control device
    102a Request-interpreting part
    102b Browsing processing part
    102c Authentication-processing part
    102d Electronic mail-generating part
    102e Web page-generating part
    102f Receiving part
    102g IGT state information-designating part
    102h Multivariate discriminant-preparing part
    102h1 Candidate multivariate discriminant-preparing part
    102h2 Candidate multivariate discriminant-verifying part
    102h3 Explanatory variable-selecting part
    102i Discriminant value-calculating part
    102j Discriminant value criterion-evaluating part
    102j1 Discriminant value criterion- discriminating part
    102k Result outputting part
    102m Sending part
104 Communication interface
106 Memory device
    106a User information file
    106b Amino acid concentration data file
    106c IGT state information file
    106d Designated IGT state information file
    106e Multivariate discriminant-related information database
    106e1 Candidate multivariate discriminant file
    106e2 Verification result file
    106e3 Selected IGT state information file
    106e4 Multivariate discriminant file
    106f Discriminant value file
    106g Evaluation result file
108 Input/output interface
112 Input device
114 Output device
200 Client apparatus (information communication terminal apparatus)
300 Network
400 Database apparatus

BEST MODE(S) FOR CARRYING OUT THE INVENTION

Hereinafter, an embodiment (first embodiment) of the method of evaluating IGT of the present invention, an embodiment (second embodiment) of the IGT-evaluating apparatus, the IGT-evaluating method, the IGT-evaluating system, the IGT-evaluating program and the recording medium of the present invention, and an embodiment (third embodiment) of the method of searching for prophylactic/ameliorating substance for IGT of the present invention are described in detail with reference to the drawings. The present invention is not limited to these embodiments.

First Embodiment

1-1. Outline of the Invention

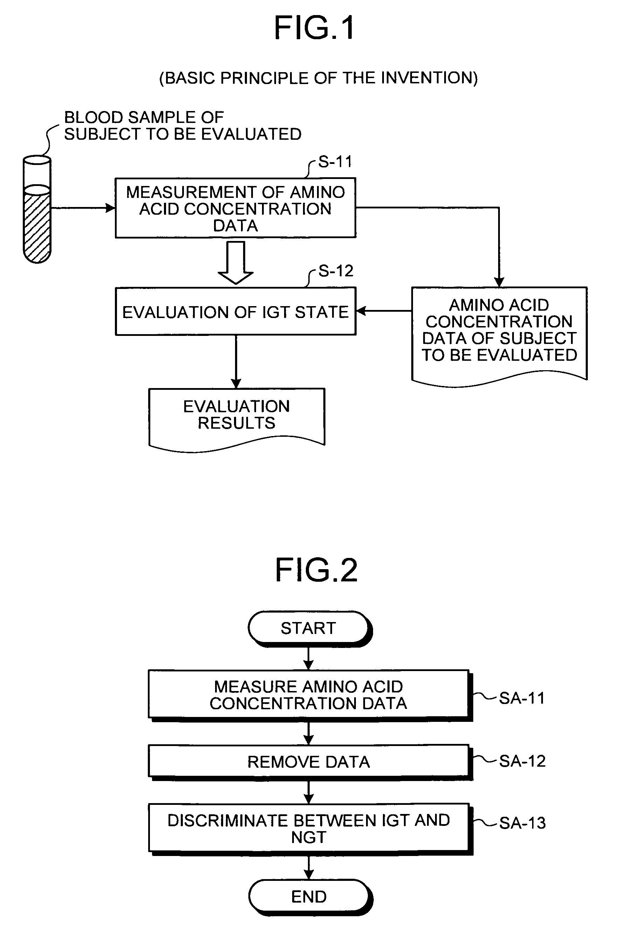

Here, an outline of the method of evaluating IGT of the present invention will be described with reference to FIG. 1. FIG. 1 is a principle configurational diagram showing a basic principle of the present invention.

Amino acid concentration data on a concentration value of an amino acid in blood collected from a subject (for example, an individual such as animal or human) to be evaluated are first measured (step S-11). Concentrations of amino acids in blood are analyzed in the following manner. A blood sample is collected in a heparin-treated tube, and then the blood plasma is separated by centrifugation of the collected blood sample. All blood plasma samples separated are frozen and stored at -70°C before a measurement of amino acid concentrations. Before the measurement of amino acid concentrations, the blood plasma samples are deproteinized by adding sulfosalicylic acid to a concentration of 3%. An amino acid analyzer by high-performance liquid chromatography (HPLC) by using ninhydrin reaction in post column is used for the measurement of amino acid concentrations. The unit of the amino acid concentration may be for example molar concentration, weight concentration, or these concentrations which are subjected to addition, subtraction, multiplication and division by an arbitrary constant.

An IGT state in the subject is evaluated based on the amino acid concentration data of the subject measured in the step S-11 (step S-12).

According to the present invention described above, the amino acid concentration data on the concentration value of the amino acid in blood collected from the subject is measured, and the IGT state in the subject is evaluated based on the measured amino acid concentration data of the subject. Thus, concentrations of amino acids in blood can be utilized to bring about an effect of enabling an accurate evaluation of the IGT state.

Before the step S-12 is executed, data such as defective and outliers may be removed from the amino acid concentration data of the subject measured in the step S-11. Thus, the IGT state can be more accurately evaluated.

In the step S-12, the IGT state in the subject may be evaluated based on the concentration value of at least one of Glu, Gly, Ser, Ile, Val, Leu, Phe and Asp contained in the amino acid concentration data of the subject measured in the step S-11. Thus, concentrations of amino acids which among amino acids in blood, are associated with the IGT state can be utilized to bring about an effect of enabling an accurate evaluation of the IGT state.

In the step S-12, a discrimination between an IGT and a NGT in the subject may be conducted based on the concentration value of at least one of Glu, Gly, Ser, Ile, Val, Leu, Phe and Asp contained in the amino acid concentration data of the subject measured in the step S-11. Specifically, the concentration value of at least one of Glu, Gly, Ser, Ile, Val, Leu, Phe and Asp may be compared with a previously established threshold (cutoff value), thereby discriminating between the IGT and the NGT in the subject. Thus, concentrations of amino acids which among amino acids in blood, are useful for discriminating between the 2 groups of the IGT and the NGT can be utilized to bring about an effect of enabling an accurate discrimination between the 2 groups of the IGT and the NGT.

In the step S-12, the discrimination between the IGT and the NGT in the subject may be conducted based on the concentration values of at least two of Glu, Gly, Ser, Ile, Val, Leu, Phe and Asp contained in the amino acid concentration data of the subject measured in the step S-11. Specifically, the concentration values of at least two of Glu, Gly, Ser, Ile, Val, Leu, Phe and Asp may be compared with a previously established threshold (cutoff value), thereby discriminating between the IGT and the NGT in the subject. Thus, concentrations of amino acids which among amino acids in blood, are useful for discriminating between the 2 groups of the IGT and the NGT can be utilized to bring about an effect of enabling an accurate discrimination between the 2 groups of the IGT and the NGT.

In the step S-12, a discriminant value that is a value of a multivariate discriminant with a concentration of the amino acid as an explanatory variable may be calculated based on both the amino acid concentration data of the subject measured in the step S-11 and the previously established multivariate discriminant, and the IGT state in the subject may be evaluated based on the calculated discriminant value. Thus, discriminant values obtained in multivariate discriminants with concentrations of amino acids as explanatory variables can be utilized to bring about an effect of enabling an accurate evaluation of the IGT state.

In the step S-12, the discriminant value may be calculated based on both the concentration value of at least one of Glu, Gly, Ser, Ile, Val, Leu, Phe and Asp contained in the amino acid concentration data of the subject measured in the step S-11 and the multivariate discriminant containing at least one of Glu, Gly, Ser, Ile, Val, Leu, Phe and Asp as the explanatory variable, and the IGT state in the subject may be evaluated based on the calculated discriminant value. Thus, discriminant values obtained in multivariate discriminants which are correlated with the IGT state significantly can be utilized to bring about an effect of enabling an accurate evaluation of the IGT state.

In the step S-12, the discrimination between the IGT and the NGT in the subject may be conducted based on the calculated discriminant value. Specifically, the discriminant value may be compared with a previously established threshold (cutoff value), thereby discriminating between the IGT and the NGT in the subject. Thus, discriminant values obtained in multivariate discriminants useful for discriminating between the 2 groups of the IGT and the NGT can be utilized to bring about an effect of enabling an accurate discrimination between the 2 groups of the IGT and the NGT.

In the step S-12, the discriminant value may be calculated based on both the concentration values of at least two of Glu, Gly, Ser, Ile, Val, Leu, Phe and Asp contained in the amino acid concentration data of the subject measured in the step S-11 and the multivariate discriminant containing at least two of Glu, Gly, Ser, Ile, Val, Leu, Phe and Asp as the explanatory variables, and the discrimination between the IGT and the NGT in the subject may be conducted based on the calculated discriminant value. Thus, discriminant values obtained in multivariate discriminants useful for discriminating between the 2 groups of the IGT and the NGT can be utilized to bring about an effect of enabling an accurate discrimination between the 2 groups of the IGT and the NGT.

the multivariate discriminant may be expressed by one fractional expression or the sum of a plurality of the fractional expressions, and may contain at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or may contain at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Specifically, the multivariate discriminant may be formula 1. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT. Glu/(His+Cit)+(Phe+Tyr)/Gly (formula 1)

the multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Specifically, the multivariate discriminant may be the logistic regression equation containing at least Glu and Gly as the explanatory variables. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

The multivariate discriminants described above can be prepared by a method described in International Publication WO 2004/052191 Pamphlet that is an international application filed by the present applicant or by a method (multivariate discriminant-preparing processing described in the second embodiment described later) described in International Publication WO 2006/098192 Pamphlet that is an international application filed by the present applicant. Any multivariate discriminants obtained by these methods can be preferably used in the evaluation of the IGT state, regardless of the unit of the amino acid concentration in the amino acid concentration data as input data.

In the fractional expression, the numerator of the fractional expression is expressed by the sum of the amino acids A, B, C etc. and the denominator of the fractional expression is expressed by the sum of the amino acids a, b, c etc. The fractional expression also includes the sum of the fractional expressions $\alpha$, $\beta$, $\gamma$ etc. (for example, $\alpha+\beta$) having such constitution. The fractional expression also includes divided fractional expressions. The amino acids used in the numerator or denominator may have suitable coefficients respectively. The amino acids used in the numerator or denominator may appear repeatedly. Each fractional expression may have a suitable coefficient. A value of a coefficient for each explanatory variable and a value for a constant term may be any real numbers. In combinations where explanatory variables in the numerator and explanatory variables in the denominator in the fractional expression are switched with each other, the positive (or negative) sign is generally reversed in correlation with objective explanatory variables, but because their correlation is maintained, such combinations can be assumed to be equivalent to one another in discrimination, and thus the fractional expression also includes combinations where explanatory variables in the numerator and explanatory variables in the denominator in the fractional expression are switched with each other.

The multivariate discriminant refers to a form of equation used generally in multivariate analysis and includes, for example, multiple regression equation, multiple logistic regression equation, linear discriminant function, Mahalanobis' generalized distance, canonical discriminant function, support vector machine, and decision tree. The multivariate discriminant also includes an equation shown by the sum of different forms of multivariate discriminants. In the multiple regression equation, multiple logistic regression equation and canonical discriminant function, a coefficient and constant term are added to each explanatory variable, and the coefficient and constant term in this case are preferably real numbers, more preferably values in the range of 99% confidence interval for the coefficient and constant term obtained from data for discrimination, more preferably in the range of 95% confidence interval for the coefficient and constant term obtained from data for discrimination. The value of each coefficient and the confidence interval thereof may be those multiplied by a real number, and the value of each constant term and the confidence interval thereof may be those having an arbitrary actual constant added or subtracted or those multiplied or divided by an arbitrary actual constant. When an expression such as a logistic regression, a linear discriminant, and a multiple regression analysis is used as an index, a linear transformation of the expression (addition of a constant and multiplication by a constant) and a monotonic increasing (decreasing) transformation (for example, a logit transformation) of the expression do not alter discrimination capability, and thus are equivalent. Therefore, the expression includes an expression that is subjected to a linear transformation and a monotonic increasing (decreasing) transformation.

When the IGT state is evaluated (specifically, the discrimination between the IGT and the NGT is conducted) in the present invention, concentrations of other metabolites (biological metabolites), protein expression level, age and sex of the subject, biological indices or the like may be used in addition to the concentrations of the amino acids. When the IGT state is evaluated (specifically, the discrimination between the IGT and the NGT is conducted) in the present invention, concentrations of other metabolites (biological metabolites), protein expression level, age and sex of the subject, biological indices or the like may be used as the explanatory variables in the multivariate discriminants in addition to the concentrations of the amino acids.

1-2. Method of evaluating IGT in accordance with the first embodiment

Herein, the method of evaluating IGT according to the first embodiment is described with reference to FIG. 2. FIG. 2 is a flowchart showing one example of the method of evaluating IGT according to the first embodiment.

The amino acid concentration data on the concentration values of the amino acids is measured from blood collected from an individual such as animal or human (step SA-11). The measurement of the concentration values of the amino acids is conducted by the method described above.

Data such as defective and outliers is then removed from the amino acid concentration data of the individual measured in the step SA-11 (step SA-12).

Then, the concentration value of at least one or at least two of Glu, Gly, Ser, Ile, Val, Leu, Phe and Asp contained in the amino acid concentration data of the individual from which the data such as the defective and outliers have been removed in the step SA-12 is compared with a previously established threshold (cutoff value), thereby discriminating between the IGT and the NGT in the individual (step SA-13).

1-3. Summary of the first embodiment and other embodiments

According to the method of evaluating IGT according to the first embodiment described in detail above, (1) the amino acid concentration data is measured from blood collected from the individual, (2) the data such as the defective and the outliers is removed from the measured amino acid concentration data of the individual, and (3) the concentration value of at least one or at least two of Glu, Gly, Ser, Ile, Val, Leu, Phe and Asp contained in the amino acid concentration data of the individual from which the data such as the defective and the outliers have been removed is compared with the previously established threshold (cutoff value), thereby discriminating between the IGT and the NGT in the individual. Thus, concentrations of amino acids which among amino acids in blood, are useful for discriminating between the 2 groups of the IGT and the NGT can be utilized to bring about an effect of enabling an accurate discrimination between the 2 groups of the IGT and the NGT.

In the step SA-13, (a) the discriminant value may be calculated based on both (i) the concentration value of at least one or at least two of Glu, Gly, Ser, Ile, Val, Leu, Phe and Asp contained in the amino acid concentration data of the individual from which the data such as the defective and the outliers have been removed in the step SA-12 and (ii) the multivariate discriminant containing at least one or at least two of Glu, Gly, Ser, Ile, Val, Leu, Phe and Asp as the explanatory variable, and (b) the calculated discriminant value may be compared with the previously established threshold (cutoff value), thereby discriminating between the IGT and the NGT in the individual. Thus, discriminant values obtained in multivariate discriminants useful for discriminating between the 2 groups of the IGT and the NGT can be utilized to bring about an effect of enabling an accurate discrimination between the 2 groups of the IGT and the NGT.

In the step SA-13, the multivariate discriminant may be expressed by one fractional expression or the sum of a plurality of the fractional expressions, and may contain at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or may contain at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Specifically, the multivariate discriminant may be formula 1. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

$$\mathtt{Glu/(His+Cit)+(Phe+Tyr)/Gly} \qquad \mathtt{(formula\ 1)}$$

In the step SA-13, the multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Specifically, the multivariate discriminant may be the logistic regression equation containing at least Glu and Gly as the explanatory variables. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

The multivariate discriminants described above can be prepared by a method described in International Publication WO 2004/052191 Pamphlet that is an international application filed by the present applicant or by a method (multivariate discriminant-preparing processing described in the second embodiment described later) described in International Publication WO 2006/098192 Pamphlet that is an international application filed by the present applicant. Any multivariate discriminants obtained by these methods can be preferably used in the evaluation of the IGT state, regardless of the unit of the amino acid concentration in the amino acid concentration data as input data.

Second Embodiment

2-1. Outline of the Invention

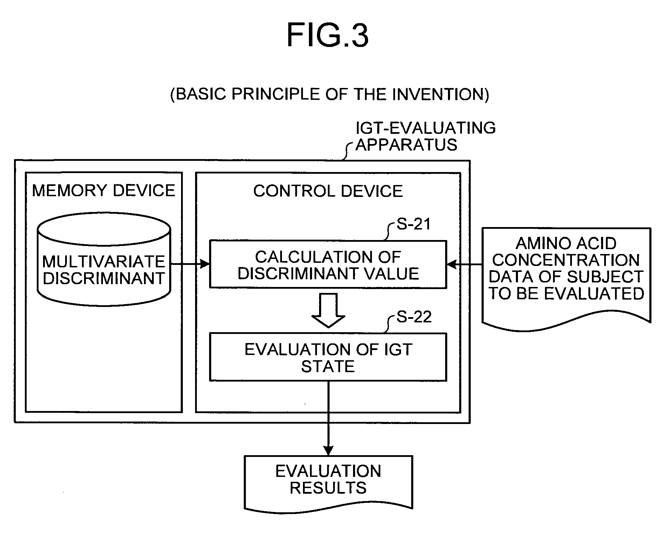

Herein, an outline of the IGT-evaluating apparatus, the IGT-evaluating method, the IGT-evaluating system, the IGT-evaluating program and the recording medium of the present invention are described in detail with reference to FIG. 3. FIG. 3 is a principle configurational diagram showing a basic principle of the present invention.

In the present invention, a discriminant value that is a value of a multivalent discriminant with a concentration of an amino acid as an explanatory variable is calculated in a control device based on both previously obtained amino acid concentration data of a subject to be evaluated (for example, an individual such as animal or human) and the multivariate discriminant stored in a memory device (step S-21).

In the present invention, an IGT state in the subject is evaluated in the control device based on the discriminant value calculated in the step S-21 (step S-22).

According to the present invention described above, the discriminant value is calculated based on both the previously obtained amino acid concentration data of the subject on the concentration value of the amino acid and the multivariate discriminant with the concentration of the amino acid as the explanatory variable stored in the memory device, and the IGT state in the subject is evaluated based on the calculated discriminant value. Thus, discriminant values obtained in multivariate discriminants with concentrations of amino acids as explanatory variables can be utilized to bring about an effect of enabling an accurate evaluation of the IGT state.

In the step S-21, the discriminant value may be calculated based on both the concentration value of at least one of Glu, Gly, Ser, Ile, Val, Leu, Phe and Asp contained in the previously obtained amino acid concentration data of the subject and the multivariate discriminant containing at least one of Glu, Gly, Ser, Ile, Val, Leu, Phe and Asp as the explanatory variable. Thus, discriminant values obtained in multivariate discriminants which are correlated with the IGT state significantly can be utilized to bring about an effect of enabling an accurate evaluation of the IGT state.

In the step S-22, a discrimination between an IGT and a NGT in the subject may be conducted based on the discriminant value calculated in the step S-21. Specifically, the discriminant value may be compared with a previously established threshold (cutoff value), thereby discriminating between the IGT and the NGT in the subject. Thus, discriminant values obtained in multivariate discriminants useful for discriminating between the 2 groups of the IGT and the NGT can be utilized to bring about an effect of enabling an accurate discrimination between the 2 groups of the IGT and the NGT.

In the step S-21, the discriminant value may be calculated based on both the concentration values of at least two of Glu, Gly, Ser, Ile, Val, Leu, Phe and Asp contained in the previously obtained amino acid concentration data of the subject and the multivariate discriminant containing at least two of Glu, Gly, Ser, Ile, Val, Leu, Phe and Asp as the explanatory variables. In the step S-22, the discrimination between the IGT and the NGT in the subject may be conducted based on the discriminant value calculated in the step S-21. Thus, discriminant values obtained in multivariate discriminants useful for discriminating between the 2 groups of the IGT and the NGT can be utilized to bring about an effect of enabling an accurate discrimination between the 2 groups of the IGT and the NGT.

The multivariate discriminant may be expressed by one fractional expression or the sum of a plurality of the fractional expressions, and may contain at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or may contain at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Specifically, the multivariate discriminant may be formula 1. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

```
Glu/(His+Cit)+(Phe+Tyr)/Gly                    (formula 1)
```

The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Specifically, the multivariate discriminant may be the logistic regression equation containing at least Glu and Gly as the explanatory variables. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

The multivariate discriminants described above can be prepared by a method described in International Publication WO 2004/052191 Pamphlet that is an international application filed by the present applicant or by a method (multivariate discriminant-preparing processing described later) described in International Publication WO 2006/098192 Pamphlet that is an international application filed by the present applicant. Any multivariate discriminants obtained by these methods can be preferably used in the evaluation of the IGT state, regardless of the unit of the amino acid concentration in the amino acid concentration data as input data.

In the fractional expression, the numerator of the fractional expression is expressed by the sum of the amino acids A, B, C etc. and the denominator of the fractional expression is expressed by the sum of the amino acids a, b, c etc. The fractional expression also includes the sum of the fractional expressions $\alpha$, $\beta$, $\gamma$ etc. (for example, $\alpha+\beta$) having such constitution. The fractional expression also includes divided fractional expressions. The amino acids used in the numerator or denominator may have suitable coefficients respectively. The amino acids used in the numerator or denominator may appear repeatedly. Each fractional expression may have a suitable coefficient. A value of a coefficient for each explanatory variable and a value for a constant term may be any real numbers. In combinations where explanatory variables in the numerator and explanatory variables in the denominator in the fractional expression are switched with each other, the positive (or negative) sign is generally reversed in correlation with objective explanatory variables, but because their correlation is maintained, such combinations can be assumed to be equivalent to one another in discrimination, and thus the fractional expression also includes combinations where explanatory variables in the numerator and explanatory variables in the denominator in the fractional expression are switched with each other.

The multivariate discriminant refers to a form of equation used generally in multivariate analysis and includes, for example, multiple regression equation, multiple logistic regression equation, linear discriminant function, Mahalanobis' generalized distance, canonical discriminant function, support vector machine, and decision tree. The multivariate discriminant also includes an equation shown by sum of different forms of the multivariate discriminants. In the multiple regression equation, multiple logistic regression equation and canonical discriminant function, a coefficient and constant term are added to each explanatory variable, and the coefficient and constant term in this case are preferably real numbers, more preferably values in the range of 99% confidence interval for the coefficient and constant term obtained from data for discrimination, more preferably in the range of 95% confidence interval for the coefficient and constant term obtained from data for discrimination. The value of each coefficient and the confidence interval thereof may be those multiplied by a real number, and the value of each constant term and the confidence interval thereof may be those having an arbitrary actual constant added or subtracted or those multiplied or divided by an arbitrary actual constant. When an expression such as a logistic regression, a linear discriminant, and a multiple regression analysis is used as an index, a linear transformation of the expression (addition of a constant and multiplication by a constant) and a monotonic increasing (decreasing) transformation (for example, a logit transformation) of the expression do not alter discrimination capability, and thus are equivalent. Therefore, the expression includes an expression that is subjected to a linear transformation and a monotonic increasing (decreasing) transformation.

When the IGT state is evaluated (specifically, the discrimination between the IGT and the NGT is conducted) in the present invention, concentrations of other metabolites (biological metabolites), protein expression level, age and sex of the subject, biological indices or the like may be used in addition to the concentrations of the amino acids. When the IGT state is evaluated (specifically, the discrimination between the IGT and the NGT is conducted) in the present invention, concentrations of other metabolites (biological metabolites), protein expression level, age and sex of the subject, biological indices or the like may be used as the explanatory variables in the multivariate discriminants in addition to the concentrations of the amino acids.

Here, the summary of the multivariate discriminant-preparing processing (steps 1 to 4) is described in detail.

First, a candidate multivariate discriminant (e.g., $y = a_1x_1 + a_2x_2 + ... + a_nx_n$, y: IGT state index data, $x_i$: amino acid concentration data, $a_i$: constant, i = 1,2, ..., n) that is a candidate of the multivariate discriminant is prepared in the control device based on a predetermined discriminant-preparing method from IGT state information stored in the memory device containing the amino acid concentration data and IGT state index data on an index for indicating the IGT state (step 1). Data containing defective and outliers may be removed in advance from the IGT state information.

In the step 1, a plurality of the candidate multivariate discriminants may be prepared from the IGT state information by using a plurality of the different discriminant-preparing methods (including those for multivariate analysis such as principal component analysis, discriminant analysis, support vector machine, multiple regression analysis, logistic regression analysis, k-means method, cluster analysis, and decision tree). Specifically, a plurality of the candidate multivariate discriminant groups may be prepared simultaneously and concurrently by using a plurality of different algorithms with the IGT state information which is multivariate data composed of the amino acid concentration data and the IGT state index data obtained by analyzing blood samples from a large number of NGT groups and IGT groups. For example, the two different candidate multivariate discriminants may be formed by performing discriminant analysis and logistic regression analysis simultaneously with the different algorithms. Alternatively, the candidate multivariate discriminant may be formed by converting the IGT state information with the candidate multivariate discriminant prepared by performing principal component analysis and then performing discriminant analysis of the converted IGT state information. In this way, it is possible to finally prepare the multivariate discriminant suitable for diagnostic condition.

The candidate multivariate discriminant prepared by principal component analysis is a linear expression consisting of amino acid explanatory variables maximizing the variance of all amino acid concentration data. The candidate multivariate discriminant prepared by discriminant analysis is a high-powered expression (including exponential and logarithmic expressions) consisting of amino acid explanatory variables minimizing the ratio of the sum of the variances in respective groups to the variance of all amino acid concentration data. The candidate multivariate discriminant prepared by using support vector machine is a high-powered expression (including kernel function) consisting of amino acid explanatory variables maximizing the boundary between groups. The candidate multivariate discriminant prepared by multiple regression analysis is a high-powered expression consisting of amino acid explanatory variables minimizing the sum of the distances from all amino acid concentration data. The candidate multivariate discriminant prepared by logistic regression analysis is a fraction expression having, as a component, the natural logarithm having a linear expression consisting of amino acid explanatory variables maximizing the likelihood as the exponent. The k-means method is a method of searching k pieces of neighboring amino acid concentration data in various groups, designating the group containing the greatest number of the neighboring points as its data-belonging group, and selecting the amino acid explanatory variable that makes the group to which input amino acid concentration data belong agree well with the designated group. The cluster analysis is a method of clustering (grouping) the points closest in entire amino acid concentration data. The decision tree is a method of ordering amino acid explanatory variables and predicting the group of amino acid concentration data from the pattern possibly held by the higher-ordered amino acid explanatory variable.

Returning to the description of the multivariate discriminant-preparing processing, the candidate multivariate discriminant prepared in the step 1 is verified (mutually verified) in the control device based on a predetermined verifying method (step 2). The verification of the candidate multivariate discriminant is performed on each other to each candidate multivariate discriminant prepared in the step 1.

In the step 2, at least one of discrimination rate, sensitivity, specificity, information criterion, and the like of the candidate multivariate discriminant may be verified by at least one of the bootstrap method, holdout method, leave-one-out method, and the like. In this way, it is possible to prepare the candidate multivariate discriminant higher in predictability or reliability, by taking the IGT state information and the diagnostic condition into consideration.

The discrimination rate is the rate of the IGT states judged correct according to the present invention in all input data. The sensitivity is the rate of the IGT states judged correct according to the present invention in the IGT states declared in the input data. The specificity is the rate of the IGT states judged correct according to the present invention in the NGT declared in the input data. The information criterion is the sum of the number of the amino acid explanatory variables in the candidate multivariate discriminant prepared in the step 1 and the difference in number between the IGT states evaluated according to the present invention and those declared in input data. The predictability is the average of the discrimination rate, sensitivity, or specificity obtained by repeating verification of the candidate multivariate discriminant. Alternatively, the reliability is the variance of the discrimination rate, sensitivity, or specificity obtained by repeating verification of the candidate multivariate discriminant.

Returning to the description of the multivariate discriminant-preparing processing, a combination of the amino acid concentration data contained in the IGT state information used in preparing the candidate multivariate discriminant is selected by selecting the explanatory variable of the candidate multivariate discriminant in the control device based on a predetermined explanatory variable-selecting method from the verification result obtained in the step 2 (step 3). The selection of the amino acid explanatory variable is performed on each candidate multivariate discriminant prepared in the step 1. In this way, it is possible to select the amino acid explanatory variable of the candidate multivariate discriminant properly. The step 1 is executed once again by using the IGT state information including the amino acid concentration data selected in the step 3.

In the step 3, the amino acid explanatory variable of the candidate multivariate discriminant may be selected based on at least one of the stepwise method, best path method, local search method, and genetic algorithm from the verification result obtained in the step 2.

The best path method is a method of selecting an amino acid explanatory variable by optimizing an evaluation index of the candidate multivariate discriminant while eliminating the amino acid explanatory variables contained in the candidate multivariate discriminant one by one.

Returning to the description of the multivariate discriminant-preparing processing, the steps 1, 2 and 3 are repeatedly performed in the control device, and based on verification results thus accumulated, the candidate multivariate discriminant used as the multivariate discriminant is selected from a plurality of the candidate multivariate discriminants, thereby preparing the multivariate discriminant (step 4). In the selection of the candidate multivariate discriminants, there are cases where the optimum multivariate discriminant is selected from the candidate multivariate discriminants prepared in the same discriminant-preparing method or the optimum multivariate discriminant is selected from all candidate multivariate discriminants.

As described above, in the multivariate discriminant-preparing processing, the processing for the preparation of the candidate multivariate discriminants, the verification of the candidate multivariate discriminants, and the selection of the explanatory variables in the candidate multivariate discriminants are performed based on the IGT state information in a series of operations in a systematized manner, whereby the optimum multivariate discriminant for the evaluation of the IGT state can be prepared. In other words, in the multivariate discriminant-preparing processing, the amino acid concentration is used in multivariate statistical analysis, and for selecting the optimum and robust combination of the explanatory variables, the explanatory variable-selecting method is combined with cross-validation to extract the multivariate discriminant having high diagnosis performance. Logistic regression equation, linear discriminant, discriminant prepared by support vector machine, discriminant prepared by Mahalanobis' generalized distance method, equation prepared by multiple regression analysis, discriminant prepared by cluster analysis, and the like can be used in the multivariate discriminant.

2-2. System configuration

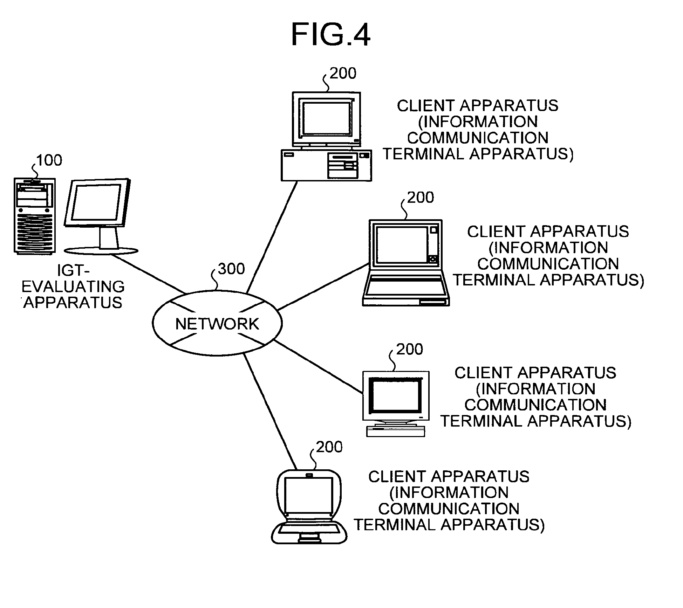

Hereinafter, a configuration of the IGT-evaluating system according to the second embodiment (hereinafter referred to sometimes as the present system) will be described with reference to FIGS. 4 to 20. This system is merely one example, and the present invention is not limited thereto.

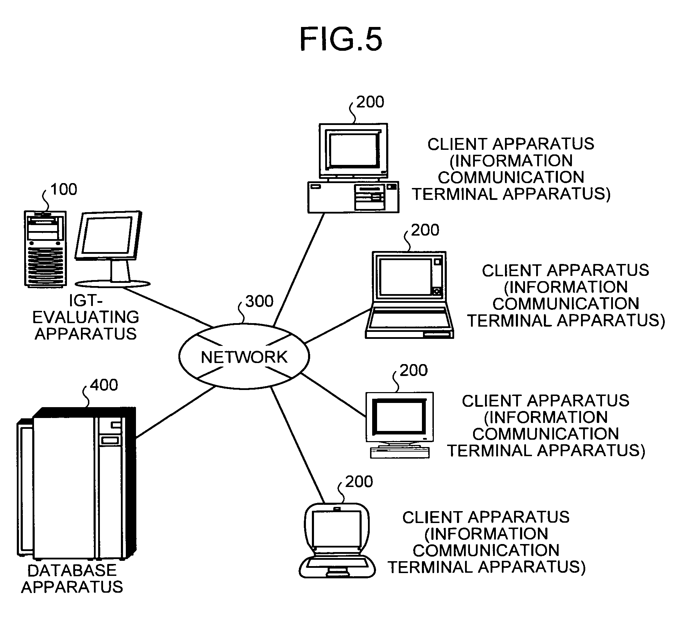

First, an entire configuration of the present system will be described with reference to FIGS. 4 and 5. FIG. 4 is a diagram showing an example of the entire configuration of the present system. FIG. 5 is a diagram showing another example of the entire configuration of the present system. As shown in FIG. 4, the present system is constituted in which the IGT-evaluating apparatus 100 that evaluates the IGT state in the subject, and the client apparatus 200 (corresponding to the information communication terminal apparatus of the present invention) that provides the amino acid concentration data of the subject on the concentration values of the amino acids, are communicatively connected to each other via a network 300.

In the present system as shown in FIG. 5, in addition to the IGT-evaluating apparatus 100 and the client apparatus 200, the database apparatus 400 storing, for example, the IGT state information used in preparing the multivariate discriminant and the multivariate discriminant used in evaluating the IGT state in the IGT-evaluating apparatus 100, may be communicatively connected via the network 300. In this configuration, the information on the IGT state etc. are provided via the network 300 from the IGT-evaluating apparatus 100 to the client apparatuses 200 and the database apparatus 400, or from the client apparatuses 200 and the database apparatus 400 to the IGT-evaluating apparatus 100. The "information on the IGT state" is information on measured values of particular items of the IGT state of organisms including human. The information on the IGT state is generated in the IGT-evaluating apparatus 100, client apparatus 200, and other apparatuses (e.g., various measuring apparatuses) and stored mainly in the database apparatus 400.

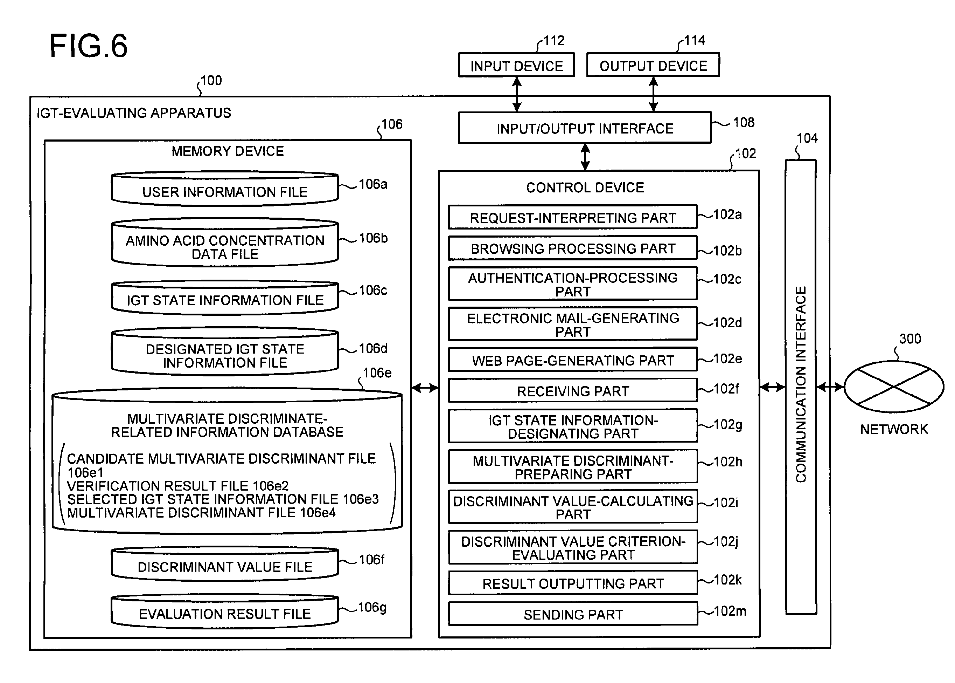

Now, a configuration of the IGT-evaluating apparatus 100 in the present system will be described with reference to FIGS. 6 to 18. FIG. 6 is a block diagram showing an example of the configuration of the IGT-evaluating apparatus 100 in the present system, showing conceptually only the region relevant to the present invention.

The IGT-evaluating apparatus 100 includes (a) a control device 102, such as CPU (Central Processing Unit), that integrally controls the IGT-evaluating apparatus 100, (b) a communication interface 104 that connects the IGT-evaluating apparatus 100 to the network 300 communicatively via communication apparatuses such as a router and wired or wireless communication lines such as a private line, (c) a memory device 106 that stores various databases, tables, files and others, and (d) an input/output interface 108 connected to an input device 112 and an output device 114, and these parts are connected to each other communicatively via any communication channel. The IGT-evaluating apparatus 100 may be present together with various analyzers (e.g., an amino acid analyzer) in a same housing. A typical configuration of disintegration/integration of the IGT-evaluating apparatus 100 is not limited to that shown in the figure, and all or a part of it may be disintegrated or integrated functionally or physically in any unit, for example, according to various loads applied. For example, a part of the processing may be performed via CGI (Common Gateway Interface).

The memory device 106 is a storage means, and examples thereof include memory apparatus such as RAM (Random Access Memory) and ROM (Read Only Memory), a fixed disk drive such as a hard disk, a flexible disk, an optical disk, and the like. The memory device 106 stores computer programs giving instructions to the CPU for various processings, together with OS (Operating System). As shown in the figure, the memory device 106 stores the user information file 106a, the amino acid concentration data file 106b, the IGT state information file 106c, the designated IGT state information file 106d, a multivariate discriminant-related information database 106e, the discriminant value file 106f, and the evaluation result file 106g.

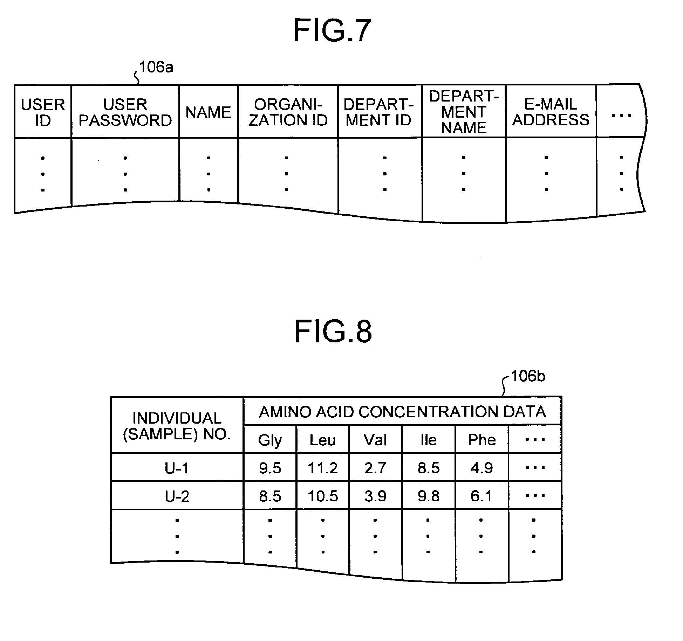

The user information file 106a stores user information on users. FIG. 7 is a chart showing an example of information stored in the user information file 106a. As shown in FIG. 7, the information stored in the user information file 106a includes user ID (identification) for identifying a user uniquely, user password for authentication of the user, user name, organization ID for uniquely identifying an organization of the user, department ID for uniquely identifying a department of the user organization, department name, and electronic mail address of the user that are correlated to one another.

Returning to FIG. 6, the amino acid concentration data file 106b stores the amino acid concentration data on the concentration values of the amino acids. FIG. 8 is a chart showing an example of information stored in the amino acid concentration data file 106b. As shown in FIG. 8, the information stored in the amino acid concentration data file 106b includes individual number for uniquely identifying an individual (sample) as a subject to be evaluated and amino acid concentration data that are correlated to one another. In FIG. 8, the amino acid concentration data are assumed to be numerical values, i.e., on a continuous scale, but the amino acid concentration data may be expressed on a nominal scale or an ordinal scale. In the case of the nominal or ordinal scale, any number may be allocated to each state for analysis. The amino acid concentration data may be combined with other biological information (e.g., biological metabolites such as glucose, lipid, protein, peptide, mineral and hormone, and biological indices such as blood glucose level, blood pressure level, sex, age, hepatic disease index, dietary habit, drinking habit, exercise habit, obesity level and disease history).

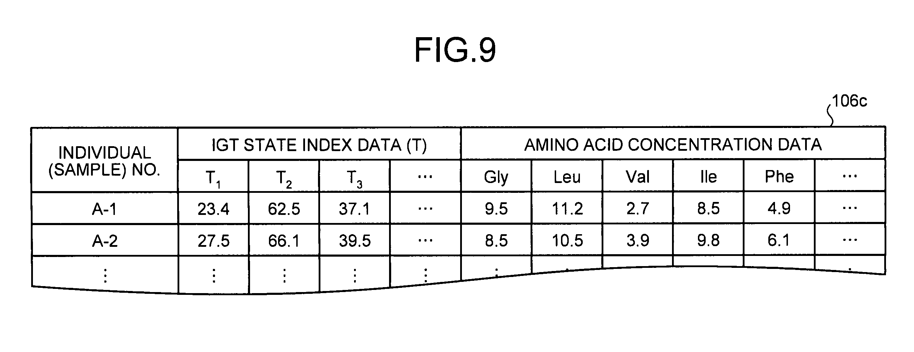

Returning to FIG. 6, the IGT state information file 106c stores the IGT state information used in preparing the multivariate discriminant. FIG. 9 is a chart showing an example of information stored in the IGT state information file 106c. As shown in FIG. 9, the information stored in the IGT state information file 106c includes individual number, IGT state index data (T) corresponding to an IGT state index (index $T_1$, index $T_2$, index $T_3$ ...), and amino acid concentration data that are correlated to one another. In FIG. 9, the IGT state index data and the amino acid concentration data are assumed to be numerical values, i.e., on a continuous scale, but the IGT state index data and the amino acid concentration data may be expressed on a nominal scale or an ordinal scale. In the case of nominal or ordinal scale, any number may be allocated to each state for analysis. The IGT state index data is a single known condition index serving as a marker of the IGT state, and numerical data may be used.

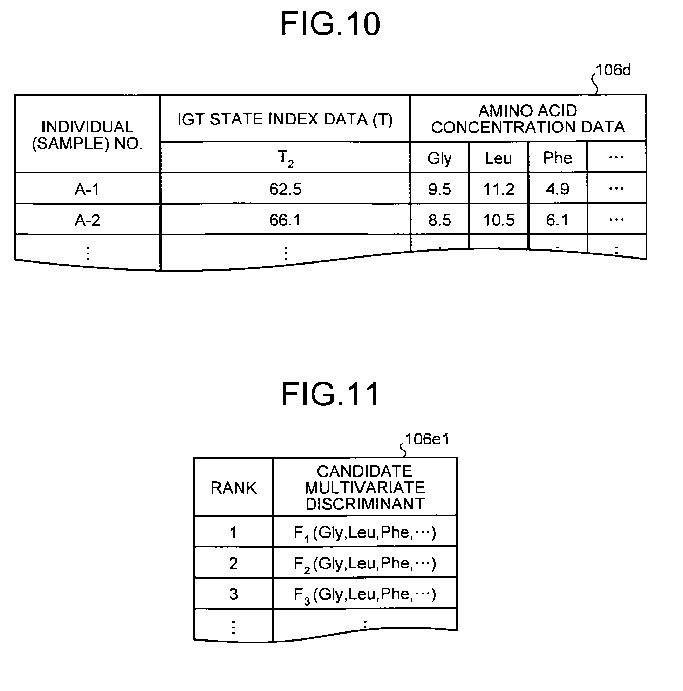

Returning to FIG. 6, the designated IGT state information file 106d stores the IGT state information designated in an IGT state information-designating part 102g described below. FIG. 10 is a chart showing an example of information stored in the designated IGT state information file 106d. As shown in FIG. 10, the information stored in the designated IGT state information file 106d includes individual number, designated IGT state index data, and designated amino acid concentration data that are correlated to one another.

Returning to FIG. 6, the multivariate discriminant-related information database 106e is composed of (i) the candidate multivariate discriminant file 106e1 storing the candidate multivariate discriminants prepared in a candidate multivariate discriminant-preparing part 102h1 described below, (ii) the verification result file 106e2 storing the verification results obtained in a candidate multivariate discriminant-verifying part 102h2 described below, (iii) the selected IGT state information file 106e3 storing the IGT state information containing the combination of the amino acid concentration data selected in an explanatory variable-selecting part 102h3 described below, and (iv) the multivariate discriminant file 106e4 storing the multivariate discriminants prepared in the multivariate discriminant-preparing part 102h described below.

The candidate multivariate discriminant file 106e1 stores the candidate multivariate discriminants prepared in the candidate multivariate discriminant-preparing part 102h1 described below. FIG. 11 is a chart showing an example of information stored in the candidate multivariate discriminant file 106e1. As shown in FIG. 11, the information stored in the candidate multivariate discriminant file 106e1 includes rank, and candidate multivariate discriminant (e.g., $F_1$ (Gly, Leu, Phe, ...), $F_2$ (Gly, Leu, Phe, ...), or $F_3$ (Gly, Leu, Phe, ...) in FIG. 11) that are correlated to each other.

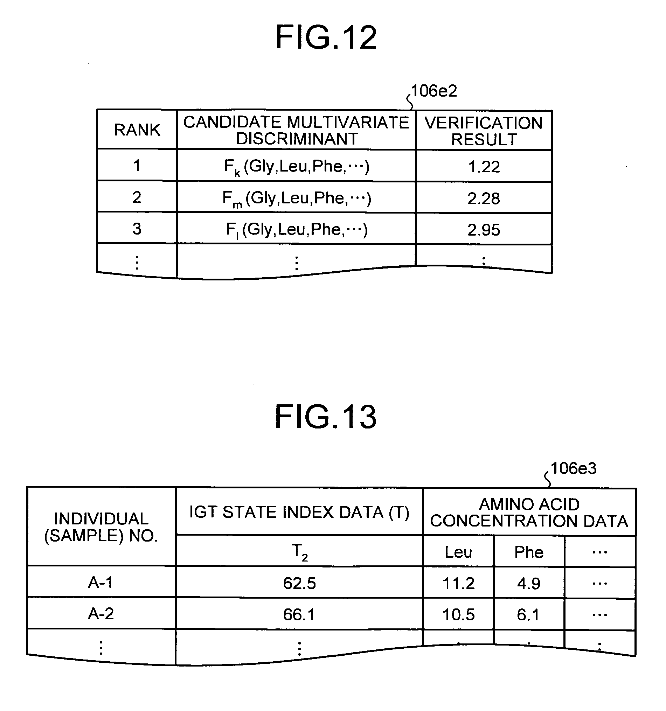

Returning to FIG. 6, the verification result file 106e2 stores the verification results obtained in the candidate multivariate discriminant-verifying part 102h2 described below. FIG. 12 is a chart showing an example of information stored in the verification result file 106e2. As shown in FIG. 12, the information stored in the verification result file 106e2 includes rank, candidate multivariate discriminant (e.g., $F_k$ (Gly, Leu, Phe, ...), $F_m$ (Gly, Leu, Phe, ...), $F_l$ (Gly, Leu, Phe, ...) in FIG. 12), and verification result of each candidate multivariate discriminant (e.g., evaluation value of each candidate multivariate discriminant) that are correlated to one another.

Returning to FIG. 6, the selected IGT state information file 106e3 stores the IGT state information including the combination of the amino acid concentration data corresponding to the explanatory variables selected in the explanatory variable-selecting part 102h3 described below. FIG. 13 is a chart showing an example of information stored in the selected IGT state information file 106e3. As shown in FIG. 13, the information stored in the selected IGT state information file 106e3 includes individual number, IGT state index data designated in the IGT state information-designating part 102g described below, and amino acid concentration data selected in the explanatory variable-selecting part 102h3 described below that are correlated to one another.

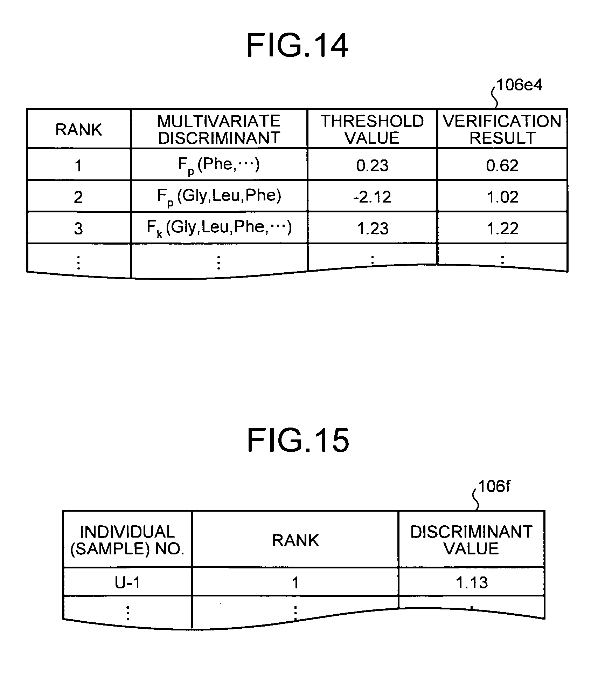

Returning to FIG. 6, the multivariate discriminant file 106e4 stores the multivariate discriminants prepared in the multivariate discriminant-preparing part 102h described below. FIG. 14 is a chart showing an example of information stored in the multivariate discriminant file 106e4. As shown in FIG. 14, the information stored in the multivariate discriminant file 106e4 includes rank, multivariate discriminant (e.g., Fp (Phe, ...), Fp (Gly, Leu, Phe), $F_k$ (Gly, Leu, Phe, ...) in FIG. 14), threshold corresponding to each discriminant-preparing method, and verification result of each multivariate discriminant (e.g., evaluation value of each multivariate discriminant) that are correlated to one another.

Returning to FIG. 6, the discriminant value file 106f stores the discriminant values calculated in a discriminant value-calculating part 102i described below. FIG. 15 is a chart showing an example of information stored in the discriminant value file 106f. As shown in FIG. 15, the information stored in the discriminant value file 106f includes individual number for uniquely identifying the individual (sample) as the subject, rank (number for uniquely identifying the multivariate discriminant), and discriminant value that are correlated to one another.

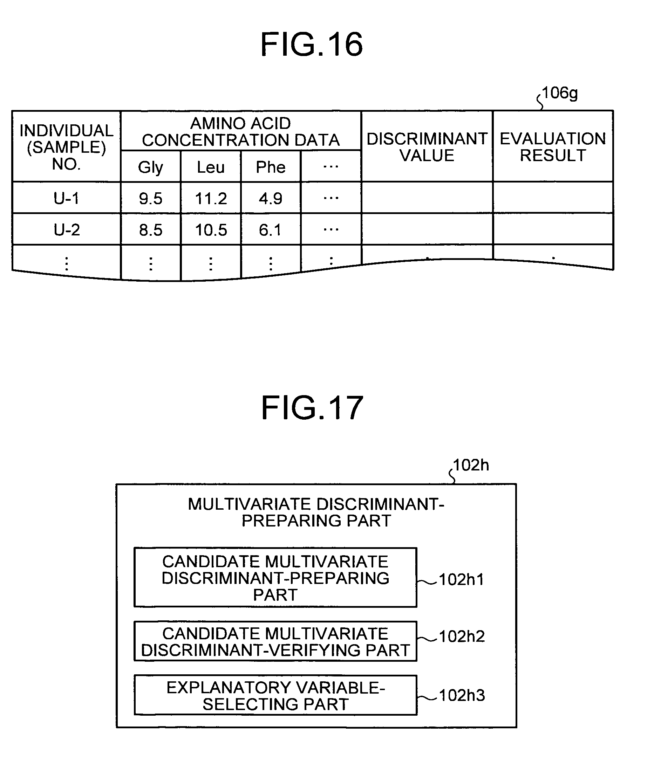

Returning to FIG. 6, the evaluation result file 106g stores the evaluation results obtained in the discriminant value criterion-evaluating part 102j described below (specifically the discrimination results obtained in a discriminant value criterion-discriminating part 102j1 described below). FIG. 16 is a chart showing an example of information stored in the evaluation result file 106g. The information stored in the evaluation result file 106g includes individual number for uniquely identifying the individual (sample) as the subject, previously obtained amino acid concentration data of the subject, discriminant value calculated in the multivariate discriminant, and evaluation result on the IGT state (specifically, discrimination result on the discrimination between the IGT and the NGT) that are correlated to one another.

Returning to FIG. 6, the memory device 106 stores various Web data for providing the client apparatuses 200 with web site information, CGI programs, and others as information other than the information described above. The Web data include data for displaying various Web pages described below and others, and the data are generated as, for example, HTML (HyperText Markup Language) or XML (Extensible Markup Language) text files. Files for components and files for operation for generation of the Web data, other temporary files, and the like are also stored in the memory device 106. In addition, the memory device 106 may store as needed sound files of sounds for transmission to the client apparatuses 200 in WAVE format or AIFF (Audio Interchange File Format) format and image files of still images or motion pictures in JPEG (Joint Photographic Experts Group) format or MPEG2 (Moving Picture Experts Group phase 2) format.

The communication interface 104 allows communication between the IGT-evaluating apparatus 100 and the network 300 (or a communication apparatus such as a router). Thus, the communication interface 104 has a function to communicate data via a communication line with other terminals.

The input/output interface 108 is connected to the input device 112 and the output device 114. A monitor (including a home television), a speaker, or a printer may be used as the output device 114 (hereinafter, the output device 114 may be described as a monitor 114). A keyboard, a mouse, a microphone, or a monitor functioning as a pointing device together with a mouse may be used as the input device 112.

The control device 102 has an internal memory storing control programs such as OS (Operating System), programs for various processing procedures, and other needed data, and performs various information processings according to these programs. As shown in the figure, the control device 102 includes mainly a request-interpreting part 102a, a browsing processing part 102b, an authentication-processing part 102c, an electronic mail-generating part 102d, a Web page-generating part 102e, a receiving part 102f, the IGT state information-designating part 102g, the multivariate discriminant-preparing part 102h, the discriminant value-calculating part 102i, the discriminant value criterion-evaluating part 102j, a result outputting part 102k, and a sending part 102m. The control device 102 performs data processings such as removal of data including defective, removal of data including many outliers, and removal of explanatory variables for the defective-including data in the IGT state information transmitted from the database apparatus 400 and in the amino acid concentration data transmitted from the client apparatus 200.

The request-interpreting part 102a interprets requests transmitted from the client apparatus 200 or the database apparatus 400 and sends the requests to other parts in the control device 102 according to results of interpreting the requests. Upon receiving browsing requests for various screens transmitted from the client apparatus 200, the browsing processing part 102b generates and transmits Web data for these screens. Upon receiving authentication requests transmitted from the client apparatus 200 or the database apparatus 400, the authentication-processing part 102c performs authentication. The electronic mail-generating part 102d generates electronic mails including various kinds of information. The Web page-generating part 102e generates Web pages for users to browse with the client apparatus 200.

The receiving part 102f receives, via the network 300, information (specifically, the amino acid concentration data, the IGT state information, the multivariate discriminant etc.) transmitted from the client apparatus 200 or the database apparatus 400. The IGT state information-designating part 102g designates objective IGT state index data and objective amino acid concentration data in preparing the multivariate discriminant.

The multivariate discriminant-preparing part 102h generates the multivariate discriminants based on the IGT state information received in the receiving part 102f and the IGT state information designated in the IGT state information-designating part 102g. Specifically, the multivariate discriminant-preparing part 102h prepares the multivariate discriminant by selecting the candidate multivariate discriminant to be used as the multivariate discriminant from a plurality of the candidate multivariate discriminants, based on verification results accumulated by repeating processings in the candidate multivariate discriminant-preparing part 102h1, the candidate multivariate discriminant-verifying part 102h2, and the explanatory variable-selecting part 102h3 from the IGT state information.

If the multivariate discriminant is stored previously in a predetermined region of the memory device 106, the multivariate discriminant-preparing part 102h may prepares the multivariate discriminant by selecting the desired multivariate discriminant out of the memory device 106. Alternatively, the multivariate discriminant-preparing part 102h may prepare the multivariate discriminant by selecting and downloading the desired multivariate discriminant from the multivariate discriminants previously stored in another computer apparatus (e.g., the database apparatus 400).

Hereinafter, a configuration of the multivariate discriminant-preparing part 102h will be described with reference to FIG. 17. FIG. 17 is a block diagram showing the configuration of the multivariate discriminant-preparing part 102h, and only a part in the configuration related to the present invention is shown conceptually. The multivariate discriminant-preparing part 102h has the candidate multivariate discriminant-preparing part 102h1, the candidate multivariate discriminant-verifying part 102h2, and the explanatory variable-selecting part 102h3, additionally. The candidate multivariate discriminant-preparing part 102h1 prepares the candidate multivariate discriminant that is a candidate of the multivariate discriminant, from the IGT state information based on a predetermined discriminant-preparing method. The candidate multivariate discriminant-preparing part 102h1 may prepare a plurality of the candidate multivariate discriminants from the IGT state information, by using a plurality of the different discriminant-preparing methods. The candidate multivariate discriminant-verifying part 102h2 verifies the candidate multivariate discriminants prepared in the candidate multivariate discriminant-preparing part 102h1 based on a predetermined verifying method. The candidate multivariate discriminant-verifying part 102h2 may verify at least one of the discrimination rate, sensitivity, specificity, and information criterion of the candidate multivariate discriminants based on at least one of the bootstrap method, holdout method, and leave-one-out method. The explanatory variable-selecting part 102h3 selects the combination of the amino acid concentration data contained in the IGT state information used in preparing the candidate multivariate discriminant, by selecting the explanatory variables of the candidate multivariate discriminant based on a predetermined explanatory variable-selecting method from the verification results obtained in the candidate multivariate discriminant-verifying part 102h2. The explanatory variable-selecting part 102h3 may select the explanatory variables of the candidate multivariate discriminant based on at least one of the stepwise method, best path method, local search method, and genetic algorithm from the verification results.

Returning to FIG. 6, the discriminant value-calculating part 102i calculates the discriminant value that is a value of the multivariate discriminant, based on the amino acid concentration data (for example, the concentration value of at least one or at least two of Glu, Gly, Ser, Ile, Val, Leu, Phe and Asp) of the subject received in the receiving part 102f and the multivariate discriminant (for example, the multivariate discriminant containing at least one or at least two of Glu, Gly, Ser, Ile, Val, Leu, Phe and Asp as the explanatory variable) prepared in the multivariate discriminant-preparing part 102h.

The multivariate discriminant may be expressed by one fractional expression or the sum of a plurality of the fractional expressions, and may contain at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or may contain at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Specifically, the multivariate discriminant may be formula 1.

$$\text{Glu/(His+Cit)+(Phe+Tyr)/Gly} \qquad\qquad \text{(formula 1)}$$

The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Specifically, the multivariate discriminant may be the logistic regression equation containing at least Glu and Gly as the explanatory variables.

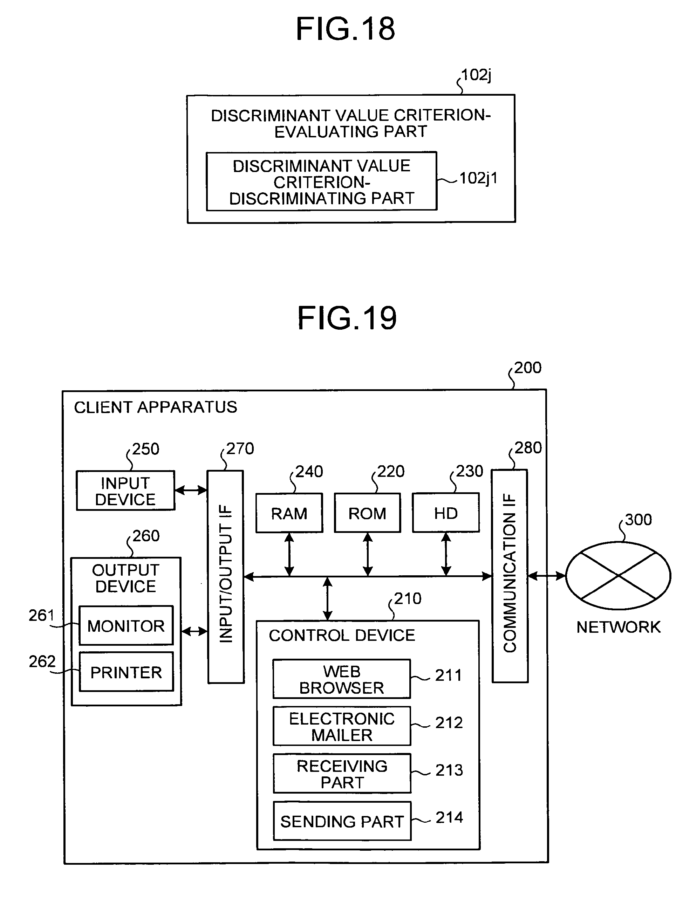

The discriminant value criterion-evaluating part 102j evaluates the IGT state in the subject based on the discriminant value calculated in the discriminant value-calculating part 102i. The discriminant value criterion-evaluating part 102j further includes the discriminant value criterion-discriminating part 102j1. Now, a configuration of the discriminant value criterion-evaluating part 102j will be described with reference to FIG. 18. FIG. 18 is a block diagram showing the configuration of the discriminant value criterion-evaluating part 102j, and only a part in the configuration related to the present invention is shown conceptually. The discriminant value criterion-discriminating part 102j1 discriminates between the IGT and the NGT in the subject based on the discriminant value. Specifically, the discriminant value criterion-discriminating part 102j1 compares the discriminant value with a previously established threshold (cutoff value), thereby discriminating between the IGT and the NGT in the subject.

Returning to FIG. 6, the result outputting part 102k outputs, into the output device 114, the processing results in each processing part in the control device 102 (the evaluation results obtained in the discriminant value criterion-evaluating part 102j (specifically the discrimination results obtained in the discriminant value criterion-discriminating part 102j1)) etc.

The sending part 102m transmits the evaluation results to the client apparatus 200 that is a sender of the amino acid concentration data of the subject, and transmits the multivariate discriminants prepared in the IGT-evaluating apparatus 100 and the evaluation results to the database apparatus 400.

Hereinafter, a configuration of the client apparatus 200 in the present system will be described with reference to FIG. 19. FIG. 19 is a block diagram showing an example of the configuration of the client apparatus 200 in the present system, and only the part in the configuration relevant to the present invention is shown conceptually.

The client apparatus 200 includes a control device 210, ROM 220, HD (Hard Disk) 230, RAM 240, an input device 250, an output device 260, an input/output IF 270, and a communication IF 280 that are connected communicatively to one another through a communication channel.

The control device 210 has a Web browser 211, an electronic mailer 212, a receiving part 213, and a sending part 214. The Web browser 211 performs browsing processings of interpreting Web data and displaying the interpreted Web data on a monitor 261 described below. The Web browser 211 may have various plug-in softwares such as stream player having functions to receive, display and feedback streaming screen images. The electronic mailer 212 sends and receives electronic mails using a particular protocol (e.g., SMTP (Simple Mail Transfer Protocol) or POP3 (Post Office Protocol version 3)). The receiving part 213 receives various kinds of information such as the evaluation results transmitted from the IGT-evaluating apparatus 100, via the communication IF 280. The sending part 214 sends various kinds of information such as the amino acid concentration data of the subject, via the communication IF 280 to the IGT-evaluating apparatus 100.

The input device 250 is for example a keyboard, a mouse, or a microphone. The monitor 261 described below also functions as a pointing device together with a mouse. The output device 260 is an output means for outputting information received via the communication IF 280, and includes the monitor 261 (including a home television) and a printer 262. In addition, the output device 260 may have a speaker or the like additionally. The input/output IF 270 is connected to the input device 250 and the output device 260.

The communication IF 280 connects the client apparatus 200 to the network 300 (or communication apparatus such as router) communicatively. In other words, the client apparatuses 200 are connected to the network 300 via a communication apparatus such as a modem, TA (Terminal Adapter) or a router, and a telephone line, or a private line. In this way, the client apparatuses 200 can access to the IGT-evaluating apparatus 100 by using a particular protocol.

The client apparatus 200 may be realized by installing softwares (including programs, data and others) for a Web data-browsing function and an electronic mail-processing function to an information processing apparatus (for example, an information processing terminal such as a known personal computer, a workstation, a family computer, Internet TV (Television), PHS (Personal Handyphone System) terminal, a mobile phone terminal, a mobile unit communication terminal or PDA (Personal Digital Assistants)) connected as needed with peripheral devices such as a printer, a monitor, and a image scanner.

All or a part of processings of the control device 210 in the client apparatus 200 may be performed by CPU and programs read and executed by the CPU. Computer programs for giving instructions to the CPU and executing various processings together with OS (Operating System) are recorded in the ROM 220 or HD 230. The computer programs, which are executed as they are loaded in the RAM 240, constitute the control device 210 with the CPU. The computer programs may be stored in application program servers connected via any network to the client apparatus 200, and the client apparatus 200 may download all or a part of them as needed. All or any part of processings of the control device 210 may be realized by hardwares such as wired-logic.

Hereinafter, the network 300 in the present system will be described with reference to FIGS. 4 and 5. The network 300 has a function to connect the IGT-evaluating apparatus 100, the client apparatuses 200, and the database apparatus 400 mutually, communicatively to one another, and is for example the Internet, an intranet, or LAN (Local Area Network (including both wired/wireless)). The network 300 may be VAN (Value Added Network), a personal computer communication network, a public telephone network (including both analog and digital), a leased line network (including both analog and digital), CATV (Community Antenna Television) network, a portable switched network or a portable packet-switched network (including IMT2000 (International Mobile Telecommunication 2000) system, GSM (Global System for Mobile Communications) system, PDC (Personal Digital Cellular)/PDC-P system, and the like), a wireless calling network, a local wireless network such as Bluetooth (registered trademark), PHS network, a satellite communication network (including CS (Communication Satellite), BS (Broadcasting Satellite), ISDB (Integrated Services Digital Broadcasting), and the like), or the like.

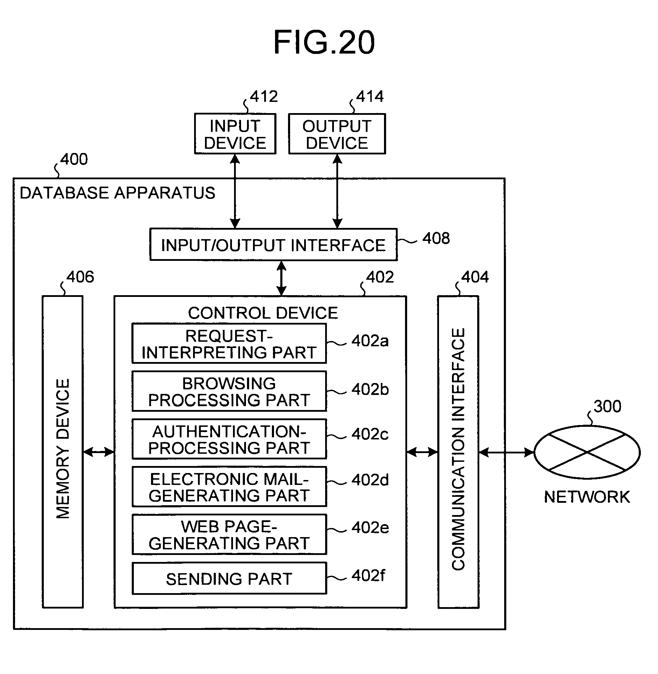

Hereinafter, a configuration of the database apparatus 400 in the present system will be described with reference to FIG. 20. FIG. 20 is a block diagram showing an example of the configuration of the database apparatus 400 in the present system, showing conceptually only the region relevant to the present invention.

The database apparatus 400 has functions to store, for example, the IGT state information used in preparing the multivariate discriminants in the IGT-evaluating apparatus 100 or in the database apparatus 400, the multivariate discriminants prepared in the IGT-evaluating apparatus 100, and the evaluation results obtained in the IGT-evaluating apparatus 100. As shown in FIG. 20, the database apparatus 400 includes (a) a control device 402, such as CPU, which integrally controls the entire database apparatus 400, (b) a communication interface 404 connecting the database apparatus 400 to the network 300 communicatively via communication apparatuses such as a router and via wired or wireless communication circuits such as a private line, (c) a memory device 406 storing various databases, tables and files (for example, files for Web pages), and (d) an input/output interface 408 connected to an input device 412 and an output device 414, and these parts are connected communicatively to each other via any communication channel.

The memory device 406 is a storage means, and may be, for example, memory apparatus such as RAM and ROM, a fixed disk drive such as a hard disk, a flexible disk, an optical disk, and the like. The memory device 406 stores various programs used in various processings. The communication interface 404 allows communication between the database apparatus 400 and the network 300 (or a communication apparatus such as a router). Thus, the communication interface 404 has a function to communicate data via a communication line with other terminals. The input/output interface 408 is connected to the input device 412 and the output device 414. A monitor (including a home television), a speaker, or a printer may be used as the output device 414 (hereinafter, the output device 414 may be described as a monitor 414). A keyboard, a mouse, a microphone, or a monitor functioning as a pointing device together with a mouse may be used as the input device 412.

The control device 402 has an internal memory storing control programs such as OS (Operating System), programs for various processing procedures, and other needed data, and performs various information processings according to these programs. As shown in the figure, the control device 402 includes mainly a request-interpreting part 402a, a browsing processing part 402b, an authentication-processing part 402c, an electronic mail-generating part 402d, a Web page-generating part 402e, and a sending part 402f.

The request-interpreting part 402a interprets requests transmitted from the IGT-evaluating apparatus 100 and sends the requests to other parts in the control device 402 according to results of interpreting the requests. Upon receiving browsing requests for various screens transmitted from the IGT-evaluating apparatus 100, the browsing processing part 402b generates and transmits Web data for these screens. Upon receiving authentication requests transmitted from the IGT-evaluating apparatus 100, the authentication-processing part 402c performs authentication. The electronic mail-generating part 402d generates electronic mails including various kinds of information. The Web page-generating part 402e generates Web pages for users to browse with the client apparatus 200. The sending part 402f transmits various kinds of information such as the IGT state information and the multivariate discriminants to the IGT-evaluating apparatus 100.

2-3. Processing in the present system

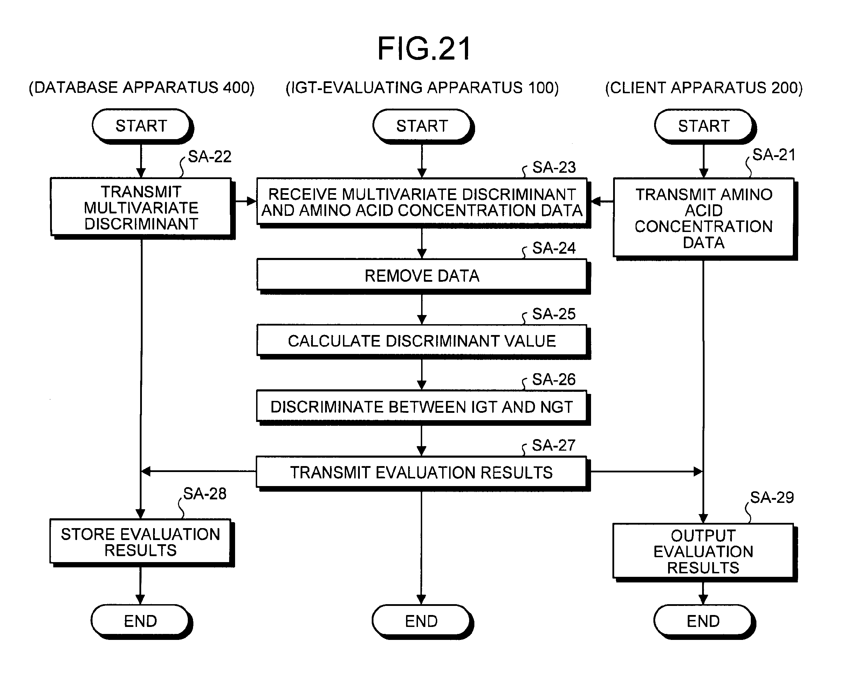

Here, an example of an IGT evaluation service processing performed in the present system constituted as described above will be described with reference to FIG. 21. FIG. 21 is a flowchart showing the example of the IGT evaluation service processing.

The amino acid concentration data used in the present processing is data concerning the concentration values of the amino acids obtained by analyzing blood previously collected from an individual. Hereinafter, the method of analyzing blood amino acid will be described briefly. First, a blood sample is collected in a heparin-treated tube, and then the blood plasma is separated by centrifugation of the tube. All blood plasma samples separated are frozen and stored at -70°C before a measurement of an amino acid concentration. Before the measurement of the amino acid concentration, the blood plasma samples are deproteinized by adding sulfosalicylic acid to a concentration of 3%. An amino acid analyzer by high-performance liquid chromatography (HPLC) by using ninhydrin reaction in post column is used for the measurement of the amino acid concentration.

First, the client apparatus 200 accesses the IGT-evaluating apparatus 100 when the user specifies the Web site address (such as URL) provided from the IGT-evaluating apparatus 100, via the input device 250 on the screen displaying the Web browser 211. Specifically, when the user instructs an update of the Web browser 211 screen on the client apparatus 200, the Web browser 211 sends the Web site address provided from the IGT-evaluating apparatus 100 by a particular protocol to the IGT-evaluating apparatus 100, thereby transmitting requests demanding a transmission of Web page corresponding to an amino acid concentration data transmission screen to the IGT-evaluating apparatus 100 based on a routing of the address.

Then, upon receiving the requests transmitted from the client apparatus 200, the request-interpreting part 102a in the IGT-evaluating apparatus 100 analyzes the transmitted requests and sends the requests to other parts in the control device 102 according to analysis results. Specifically, when the transmitted requests are requests to send the Web page corresponding to the amino acid concentration data transmission screen, mainly the browsing processing part 102b in the IGT-evaluating apparatus 100 obtains the Web data for displaying the Web page stored in a predetermined region of the memory device 106 and sends the obtained Web data to the client apparatus 200. More specifically, upon receiving the requests to transmit the Web page corresponding to the amino acid concentration data transmission screen by the user, the control device 102 in the IGT-evaluating apparatus 100 demands inputs of user ID and user password from the user. If the user ID and password are input, the authentication-processing part 102c in the IGT-evaluating apparatus 100 examines the input user ID and password by comparing them with the user ID and user password stored in the user information file 106a for authentication. Only when the user is authenticated, the browsing processing part 102b in the IGT-evaluating apparatus 100 sends the Web data for displaying the Web page corresponding to the amino acid concentration data transmission screen to the client apparatus 200. The client apparatus 200 is identified with the IP (Internet Protocol) address transmitted from the client apparatus 200 together with the transmission requests.

Then, the client apparatus 200 receives, in the receiving part 213, the Web data (for displaying the Web page corresponding to the amino acid concentration data transmission screen) transmitted from the IGT-evaluating apparatus 100, interprets the received Web data with the Web browser 211, and displays the amino acid concentration data transmission screen on the monitor 261.

When the user inputs and selects, via the input device 250, for example the amino acid concentration data of the individual on the amino acid concentration data transmission screen displayed on the monitor 261, the sending part 214 in the client apparatus 200 transmits an identifier for identifying input information and selected items to the IGT-evaluating apparatus 100, thereby transmitting the amino acid concentration data of the individual as the subject to the IGT-evaluating apparatus 100 (step SA-21). In the step SA-21, the transmission of the amino acid concentration data may be realized for example by using an existing file transfer technology such as FTP (File Transfer Protocol).

Then, the request-interpreting part 102a in the IGT-evaluating apparatus 100 interprets the identifier transmitted from the client apparatus 200 thereby interpreting the requests from the client apparatus 200, and requests the database apparatus 400 to send the multivariate discriminants for an evaluation of the IGT state (specifically, for a discrimination between the 2 groups of the IGT and the NGT) containing at least one or at least two of Glu, Gly, Ser, Ile, Val, Leu, Phe and Asp as the explanatory variable.

Then, the request-interpreting part 402a in the database apparatus 400 interprets the transmission requests from the IGT-evaluating apparatus 100 and transmits, to the IGT-evaluating apparatus 100, the multivariate discriminant (for example, the updated newest multivariate discriminant) stored in a predetermined memory region of the memory device 406 containing at least one or at least two of Glu, Gly, Ser, Ile, Val, Leu, Phe and Asp as the explanatory variable (step SA-22).

In the step SA-22, the multivariate discriminant transmitted to the IGT-evaluating apparatus 100 may be expressed by one fractional expression or the sum of a plurality of the fractional expressions, and may contain at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or may contain at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Specifically, the multivariate discriminant may be formula 1.

$$Glu/(His+Cit)+(Phe+Tyr)/Gly \qquad (formula\ 1)$$

In the step SA-22, the multivariate discriminant transmitted to the IGT-evaluating apparatus 100 may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Specifically, the multivariate discriminant may be the logistic regression equation containing at least Glu and Gly as the explanatory variables.

The IGT-evaluating apparatus 100 receives, in the receiving part 102f, the amino acid concentration data of the individual transmitted from the client apparatuses 200 and the multivariate discriminant transmitted from the database apparatus 400, and stores the received amino acid concentration data in a predetermined memory region of the amino acid concentration data file 106b and the received multivariate discriminant in a predetermined memory region of the multivariate discriminant file 106e4 (step SA-23).

Then, the control device 102 in the IGT-evaluating apparatus 100 removes data such as defective and outliers from the amino acid concentration data of the individual received in the step SA-23 (step SA-24).

Then, the discriminant value-calculating part 102i in the IGT-evaluating apparatus 100 calculates the discriminant value based on both the multivariate discriminant received in the step SA-23 and the concentration value of at least one or at least two of Glu, Gly, Ser, Ile, Val, Leu, Phe and Asp contained in the amino acid concentration data of the individual from which the data such as the defective and outliers have been removed in the step SA-24 (step SA-25).

Then, the discriminant value criterion-discriminating part 102j1 in the IGT-evaluating apparatus 100 compares the discriminant value calculated in the step SA-25 with a previously established threshold (cutoff value), thereby discriminating between the IGT and the NGT in the individual, and stores the discrimination results in a predetermined memory region of the evaluation result file 106g (step SA-26).

Then, the sending part 102m in the IGT-evaluating apparatus 100 sends, to the client apparatus 200 that has sent the amino acid concentration data and to the database apparatus 400, the discrimination results (the discrimination results on the discrimination between the IGT and the NGT) obtained in the step SA-26 (step SA-27). Specifically, the IGT-evaluating apparatus 100 first generates a Web page for displaying the discrimination results in the Web page-generating part 102e and stores the Web data corresponding to the generated Web page in a predetermined memory region of the memory device 106. Then, the user is authenticated as described above by inputting a predetermined URL (Uniform Resource Locator) into the Web browser 211 of the client apparatus 200 via the input device 250, and the client apparatus 200 sends a Web page browsing request to the IGT-evaluating apparatus 100. The IGT-evaluating apparatus 100 then interprets the browsing request transmitted from the client apparatus 200 in the browsing processing part 102b and reads the Web data corresponding to the Web page for displaying the discrimination results, out of the predetermined memory region of the memory device 106. The sending part 102m in the IGT-evaluating apparatus 100 then sends the read-out Web data to the client apparatus 200 and simultaneously sends the Web data or the discrimination results to the database apparatus 400.

In the step SA-27, the control device 102 in the IGT-evaluating apparatus 100 may notify the discrimination results to the user client apparatus 200 by electronic mail. Specifically, the electronic mail-generating part 102d in the IGT-evaluating apparatus 100 first acquires the user electronic mail address by referencing the user information stored in the user information file 106a based on the user ID and the like at the transmission timing. The electronic mail-generating part 102d in the IGT-evaluating apparatus 100 then generates electronic mail data with the acquired electronic mail address as its mail address, including the user name and the discrimination results. The sending part 102m in the IGT-evaluating apparatus 100 then transmits the generated electronic mail data to the user client apparatus 200.

Also in the step SA-27, the IGT-evaluating apparatus 100 may send the discrimination results to the user client apparatus 200 by using, for example, an existing file transfer technology such as FTP.

Returning to FIG. 21, the control device 402 in the database apparatus 400 receives the discrimination results or the Web data transmitted from the IGT-evaluating apparatus 100 and stores (accumulates) the received discrimination results or the received Web data in a predetermined memory region of the memory device 406 (step SA-28).

The receiving part 213 in the client apparatus 200 receives the Web data transmitted from the IGT-evaluating apparatus 100, and the received Web data is interpreted with the Web browser 211, to display on the monitor 261 the Web page screen displaying the discrimination results of the individual (step SA-29). When the discrimination results are sent from the IGT-evaluating apparatus 100 by electronic mail, the electronic mail transmitted from the IGT-evaluating apparatus 100 is received at any timing, and the received electronic mail is displayed on the monitor 261 with the known function of the electronic mailer 212 in the client apparatus 200.

In this way, the user can confirm the discrimination results on the discrimination of the 2 groups of the IGT and the NGT in the individual, by browsing the Web page displayed on the monitor 261. The user can print out the contents of the Web page displayed on the monitor 261 by the printer 262.

When the discrimination results are transmitted by electronic mail from the IGT-evaluating apparatus 100, the user reads the electronic mail displayed on the monitor 261, whereby the user can confirm the discrimination results on the discrimination of the 2 groups of the IGT and the NGT in the individual. The user may print out the contents of the electronic mail displayed on the monitor 261 by the printer 262.

Given the foregoing description, the explanation of the IGT evaluation service processing is finished.

2-4. Summary of the second embodiment and other embodiments

According to the IGT-evaluating system described above in detail, the client apparatus 200 sends the amino acid concentration data of the individual to the IGT-evaluating apparatus 100. Upon receiving the requests from the IGT-evaluating apparatus 100, the database apparatus 400 transmits the multivariate discriminant for the discrimination of the 2 groups of the IGT and the NGT, to the IGT-evaluating apparatus 100. By the IGT-evaluating apparatus 100, (1) the amino acid concentration data transmitted from the client apparatus 200 is received and the multivariate discriminant transmitted from the database apparatus 400 is received simultaneously, (2) the discriminant values are calculated based on the received amino acid concentration data and the received multivariate discriminant, (3) the calculated discriminant values are compared with the previously established threshold, thereby discriminating between the IGT and the NGT in the individual, and (4) the discrimination results are transmitted to the client apparatus 200 and database apparatus 400. Then, the client apparatus 200 receives and displays the discrimination results transmitted from the IGT-evaluating apparatus 100, and the database apparatus 400 receives and stores the discrimination results transmitted from the IGT-evaluating apparatus 100. Thus, the discriminant values obtained in the multivariate discriminants useful for discriminating between the 2 groups of the IGT and the NGT can be utilized to bring about an effect of enabling an accurate discrimination between the 2 groups of the IGT and the NGT.

According to the IGT-evaluating system, the multivariate discriminant may be expressed by one fractional expression or the sum of a plurality of the fractional expressions, and may contain at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or may contain at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Specifically, the multivariate discriminant may be formula 1. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

$$Glu/(His+Cit)+(Phe+Tyr)/Gly \qquad (formula\ 1)$$

According to the IGT-evaluating system, the multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Specifically, the multivariate discriminant may be the logistic regression equation containing at least Glu and Gly as the explanatory variables. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

The multivariate discriminants described above can be prepared by a method described in International Publication WO 2004/052191 Pamphlet that is an international application filed by the present applicant or by a method (multivariate discriminant-preparing processing described later) described in International Publication WO 2006/098192 Pamphlet that is an international application filed by the present applicant. Any multivariate discriminants obtained by these methods can be preferably used in the evaluation of the IGT state, regardless of the unit of the amino acid concentration in the amino acid concentration data as input data.

In addition to the second embodiment described above, the IGT-evaluating apparatus, the IGT-evaluating method, the IGT-evaluating system, the IGT-evaluating program product and the recording medium according to the present invention can be practiced in various different embodiments within the technological scope of the claims. For example, among the processings described in the second embodiment above, all or a part of the processings described above as performed automatically may be performed manually, and all or a part of the manually conducted processings may be performed automatically by known methods. In addition, the processing procedure, control procedure, specific name, various registered data, information including parameters such as retrieval condition, screen, and database configuration shown in the description above or drawings may be modified arbitrarily, unless specified otherwise. For example, the components of the IGT-evaluating apparatus 100 shown in the figures are conceptual and functional and may not be the same physically as those shown in the figure. In addition, all or an arbitrary part of the operational function of each component and each device in the IGT-evaluating apparatus 100 (in particular, the operational functions executed in the control device 102) may be executed by the CPU (Central Processing Unit) or the programs executed by the CPU, and may be realized as wired-logic hardware.

The "program" is a data processing method written in any language or by any description method and may be of any format such as source code or binary code. The "program" may be not limited to a program configured singly, and may include a program configured decentrally as a plurality of modules or libraries, and a program to achieve the function together with a different program such as OS (Operating System). The program is stored on a recording medium and read mechanically as needed by the IGT-evaluating apparatus 100. Any well-known configuration or procedure may be used as specific configuration, reading procedure, installation procedure after reading, and the like for reading the programs recorded on the recording medium in each apparatus.

The "recording media" includes any "portable physical media", "fixed physical media", and "communication media". Examples of the "portable physical media" include flexible disk, magnetic optical disk, ROM, EPROM (Erasable Programmable Read Only Memory), EEPROM (Electronically Erasable and Programmable Read Only Memory), CD-ROM (Compact Disk Read Only Memory), MO (Magneto-Optical disk), DVD (Digital Versatile Disk), and the like. Examples of the "fixed physical media" include ROM, RAM, HD, and the like which are installed in various computer systems. The "communication media" for example stores the program for a short period of time such as communication line and carrier wave when the program is transmitted via a network such as LAN (Local Area Network), WAN (Wide Area Network), or the Internet.

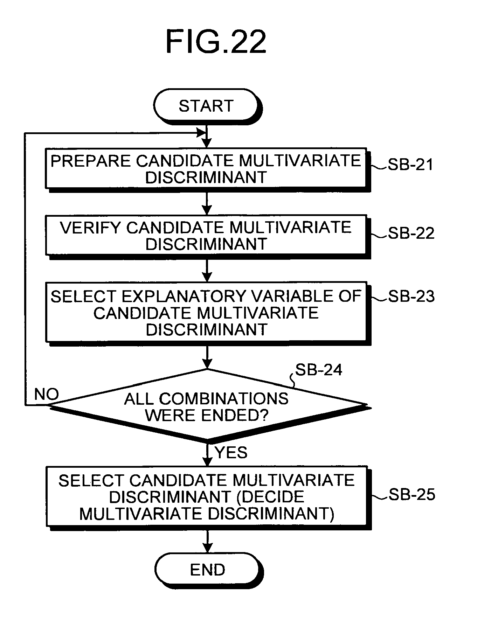

Finally, an example of the multivariate discriminant-preparing processing performed in the IGT-evaluating apparatus 100 is described in detail with reference to FIG. 22. FIG. 22 is a flowchart showing an example of the multivariate discriminant-preparing processing. The multivariate discriminant-preparing processing may be performed in the database apparatus 400 handling the IGT state information.

In the present description, the IGT-evaluating apparatus 100 stores the IGT state information previously obtained from the database apparatus 400 in a predetermined memory region of the IGT state information file 106c. The IGT-evaluating apparatus 100 shall store, in a predetermined memory region of the designated IGT state information file 106d, the IGT state information including the IGT state index data and amino acid concentration data designated previously in the IGT state information-designating part 102g.

The candidate multivariate discriminant-preparing part 102h1 in the multivariate discriminant-preparing part 102h first prepares the candidate multivariate discriminant according to a predetermined discriminant-preparing method from the IGT state information stored in a predetermine memory region of the designated IGT state information file 106d, and stores the prepared candidate multivariate discriminate in a predetermined memory region of the candidate multivariate discriminant file 106e1 (step SB-21). Specifically, the candidate multivariate discriminant-preparing part 102h1 in the multivariate discriminant-preparing part 102h first selects a desired method out of a plurality of different discriminant-preparing methods (including those for multivariate analysis such as principal component analysis, discriminant analysis, support vector machine, multiple regression analysis, logistic regression analysis, k-means method, cluster analysis, and decision tree) and determines the form of the candidate multivariate discriminant to be prepared based on the selected discriminant-preparing method. The candidate multivariate discriminant-preparing part 102h1 in the multivariate discriminant-preparing part 102h then performs various calculation corresponding to the selected function-selecting method (e.g., average or variance), based on the IGT state information. The candidate multivariate discriminant-preparing part 102h1 in the multivariate discriminant-preparing part 102h then determines the parameters for the calculation result and the determined candidate multivariate discriminant. In this way, the candidate multivariate discriminant is generated based on the selected discriminant-preparing method. When candidate multivariate discriminants are generated simultaneously and concurrently (in parallel) by using a plurality of different discriminant-preparing methods in combination, the processings described above may be executed concurrently for each selected discriminant-preparing method. Alternatively when candidate multivariate discriminants are generated in series by using a plurality of different discriminant-preparing methods in combination, for example, candidate multivariate discriminants may be generated by converting the IGT state information with the candidate multivariate discriminant prepared by performing principal component analysis and performing discriminant analysis of the converted IGT state information.

The candidate multivariate discriminant-verifying part 102h2 in the multivariate discriminant-preparing part 102h verifies (mutually verifies) the candidate multivariate discriminant prepared in the step SB-21 according to a predetermined verifying method and stores the verification result in a predetermined memory region of the verification result file 106e2 (step SB-22). Specifically, the candidate multivariate discriminant-verifying part 102h2 in the multivariate discriminant-preparing part 102h first generates the verification data to be used in verification of the candidate multivariate discriminant, based on the IGT state information stored in a predetermined memory region of the designated IGT state information file 106d, and verifies the candidate multivariate discriminant according to the generated verification data. If a plurality of candidate multivariate discriminants are generated by using a plurality of different discriminant-preparing methods in the step SB-21, the candidate multivariate discriminant-verifying part 102h2 in the multivariate discriminant-preparing part 102h verifies each candidate multivariate discriminant corresponding to each discriminant-preparing method according to a predetermined verifying method. Here in the step SB-22, at least one of the discrimination rate, sensitivity, specificity, information criterion, and the like of the candidate multivariate discriminant may be verified based on at least one of the bootstrap method, holdout method, leave-one-out method, and the like. Thus, it is possible to select the candidate multivariate discriminant higher in predictability or reliability, by taking the IGT state information and the diagnostic condition into consideration.

Then, the explanatory variable-selecting part 102h3 in the multivariate discriminant-preparing part 102h selects the combination of the amino acid concentration data contained in the IGT state information used in preparing the candidate multivariate discriminant by selecting the explanatory variable of the candidate multivariate discriminant from the verification result obtained in the step SB-22 according to a predetermined explanatory variable-selecting method, and stores the IGT state information including the selected combination of the amino acid concentration data in a predetermined memory region of the selected IGT state information file 106e3 (step SB-23). When a plurality of candidate multivariate discriminants are generated by using a plurality of different discriminant-preparing methods in the step SB-21 and each candidate multivariate discriminant corresponding to each discriminant-preparing method is verified according to a predetermined verifying method in the step SB-22, the explanatory variable-selecting part 102h3 in the multivariate discriminant-preparing part 102h selects the explanatory variable of the candidate multivariate discriminant for each candidate multivariate discriminant corresponding to the verification result obtained in the step SB-22, according to a predetermined explanatory variable-selecting method in the step SB-23. Here in the step SB-23, the explanatory variable of the candidate multivariate discriminant may be selected from the verification results according to at least one of the stepwise method, best path method, local search method, and genetic algorithm. The best path method is a method of selecting an explanatory variable by optimizing an evaluation index of the candidate multivariate discriminant while eliminating the explanatory variables contained in the candidate multivariate discriminant one by one. In the step SB-23, the explanatory variable-selecting part 102h3 in the multivariate discriminant-preparing part 102h may select the combination of the amino acid concentration data based on the IGT state information stored in a predetermined memory region of the designated IGT state information file 106d.

The multivariate discriminant-preparing part 102h then judges whether all combinations of the amino acid concentration data contained in the IGT state information stored in a predetermined memory region of the designated IGT state information file 106d are processed, and if the judgment result is "End" (Yes in step SB-24), the processing advances to the next step (step SB-25), and if the judgment result is not "End" (No in step SB-24), it returns to the step SB-21. The multivariate discriminant-preparing part 102h judges whether the processing is performed a predetermined number of times, and if the judgment result is "End" (Yes in step SB-24), the processing may advance to the next step (step SB-25), and if the judgment result is not "End" (No in step SB-24), it may return to the step SB-21. The multivariate discriminant-preparing part 102h may judge whether the combination of the amino acid concentration data selected in the step SB-23 is the same as the combination of the amino acid concentration data contained in the IGT state information stored in a predetermined memory region of the designated IGT state information file 106d or the combination of the amino acid concentration data selected in the previous step SB-23, and if the judgment result is "the same" (Yes in step SB-24), the processing may advance to the next step (step SB-25) and if the judgment result is not "the same" (No in step SB-24), it may return to step SB-21. If the verification result is specifically the evaluation value for each multivariate discriminant, the multivariate discriminant-preparing part 102h may advance to the step SB-25 or return to the step SB-21, based on the comparison of the evaluation value with a particular threshold corresponding to each discriminant-preparing method.

Then, the multivariate discriminant-preparing part 102h determines the multivariate discriminant by selecting the candidate multivariate discriminant used as the multivariate discriminant based on the verification results from a plurality of the candidate multivariate discriminants, and stores the determined multivariate discriminant (the selected candidate multivariate discriminant) in particular memory region of the multivariate discriminant file 106e4 (step SB-25). Here, in the step SB-25, for example, there are cases where the optimal multivariate discriminant is selected from the candidate multivariate discriminants prepared in the same discriminant-preparing method or the optimal multivariate discriminant is selected from all candidate multivariate discriminants.

Given the foregoing description, the explanation of the multivariate discriminant-preparing processing is finished.

Third Embodiment

3-1. Outline of the Invention

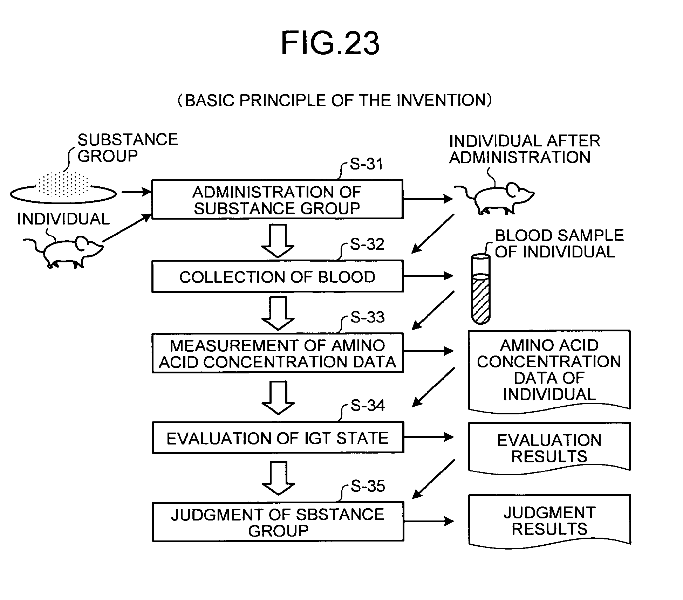

Herein, the method of searching for prophylactic/ameliorating substance for IGT of the present invention is described in detail with reference to FIG. 23. FIG. 23 is a principle configurational diagram showing a basic principle of the present invention.

First, a desired substance group consisting of one or more substances is administered to a subject to be evaluated (for example, an individual such as an animal or a human) (step S-31). For example, a suitable combination of an existing drug, amino acid, food and supplement capable of administration to humans (for example, a suitable combination of a drug, supplement and anti-obesity drug that are known to be effective in amelioration of various symptoms of IGT) may be administered over a predetermined period (for example in the range of 1 day to 12 months) in a predetermined amount at predetermined frequency and timing (for example 3 times per day, after food) by a predetermined administration method (for example, oral administration). The administration method, dose, and dosage form may be suitably combined depending on the condition of a patient. The dosage form may be determined based on known techniques. The dose is not particularly limited, and for example, a drug containing 1 $\mu$g to 100 g active ingredient may be given.

From the subject administered with the substance group in the step S-31, blood is then collected (step S-32).

Amino acid concentration data on concentration values of amino acids are measured from the blood collected in the step S-32 (step S-33). The concentrations of amino acids in blood may be analyzed in the following manner. A blood sample is collected in a heparin-treated tube, and then the blood plasma is separated by centrifugation of the collected blood sample. All blood plasma samples separated are frozen and stored at -70°C before a measurement of an amino acid concentration. Before the measurement of the amino acid concentration, the blood plasma samples are defrosted, and the defrosted blood plasma samples are deproteinized by adding sulfosalicylic acid to a concentration of 3%. The concentration values of various amino acids are measured by analyzing the deproteinized blood plasma samples by an amino acid analyzer by high-performance liquid chromatography (HPLC) by using ninhydrin reaction in post column.

Then, an IGT state in the subject is evaluated based on the amino acid concentration data of the subject measured in the step S-33 (step S-34).

Then, whether or not the substance group administered in the step S-31 prevents the IGT or ameliorates the IGT state is judged based on an evaluation result in the step S-34 (step S-35).

When a judgment result in the step S-35 is "preventive or ameliorative", the substance group administered in the step S-31 is searched as one preventing the IGT or ameliorating the IGT state.

According to the present invention, (1) the desired substance group is administered to the subject, (2) blood is collected from the subject to which the desired substance group has been administered, (3) the amino acid concentration data on the concentration values of the amino acids is measured, (4) the IGT state in the subject is evaluated based on the measured amino acid concentration data, and (5) it is judged whether or not the desired substance group prevents the IGT or ameliorates the IGT state based on the evaluation results. Thus, the method of evaluating IGT capable of accurately evaluating the IGT state by utilizing the concentrations of the amino acids in blood can be used to bring about an effect of enabling an accurate search for substances for preventing the IGT or ameliorating the IGT state.

Before the step S-34 is executed, data such as defective and outliers may be removed from the amino acid concentration data. Thereby, the IGT state can be more accurately evaluated.

In the step S-34, the IGT state in the subject may be evaluated based on the concentration value of at least one of Glu, Gly, Ser, Ile, Val, Leu, Phe and Asp contained in the amino acid concentration data of the subject measured in the step S-33. Thus, the concentrations of the amino acids which among amino acids in blood, are associated with the IGT state can be utilized to bring about an effect of enabling an accurate evaluation of the IGT state.

In the step S-34, a discrimination between the IGT and a NGT in the subject may be conducted based on the concentration value of at least one of Glu, Gly, Ser, Ile, Val, Leu, Phe and Asp contained in the amino acid concentration data of the subject measured in the step S-33. Specifically, the concentration value of at least one of Glu, Gly, Ser, Ile, Val, Leu, Phe and Asp may be compared with a previously established threshold (cutoff value), thereby discriminating between the IGT and the NGT in the subject. Thus, the concentrations of the amino acids which among amino acids in blood, are useful for discriminating between the 2 groups of the IGT and the NGT can be utilized to bring about an effect of enabling an accurate discrimination between the 2 groups of the IGT and the NGT.

In the step S-34, the discrimination between the IGT and the NGT in the subject may be conducted based on the concentration values of at least two of Glu, Gly, Ser, Ile, Val, Leu, Phe and Asp contained in the amino acid concentration data of the subject measured in the step S-33. Specifically, the concentration values of at least two of Glu, Gly, Ser, Ile, Val, Leu, Phe and Asp may be compared with a previously established threshold (cutoff value), thereby discriminating between the IGT and the NGT in the subject. Thus, the concentrations of the amino acids which among amino acids in blood, are useful for discriminating between the 2 groups of the IGT and the NGT can be utilized to bring about an effect of enabling an accurate discrimination between the 2 groups of the IGT and the NGT.

In the step S-34, a discriminant value that is a value of a multivariate discriminant with a concentration of the amino acid as an explanatory variable may be calculated based on both the amino acid concentration data of the subject measured in the step S-33 and the previously established multivariate discriminant, and the IGT state in the subject may be evaluated based on the calculated discriminant value. Thus, the discriminant values obtained in the multivariate discriminants with the concentrations of the amino acids as the explanatory variables can be utilized to bring about an effect of enabling an accurate evaluation of the IGT state.

In the step S-34, the discriminant value may be calculated based on both the concentration value of at least one of Glu, Gly, Ser, Ile, Val, Leu, Phe and Asp contained in the amino acid concentration data of the subject measured in the step S-33 and the multivariate discriminant containing at least one of Glu, Gly, Ser, Ile, Val, Leu, Phe and Asp as the explanatory variable, and the IGT state in the subject may be evaluated based on the calculated discriminant value. Thus, the discriminant values obtained in the multivariate discriminants which are correlated with the IGT state significantly can be utilized to bring about an effect of enabling an accurate evaluation of the IGT state.

In the step S-34, the discrimination between the IGT and the NGT in the subject may be conducted based on the calculated discriminant value. Specifically, the discriminant value may be compared with a previously established threshold (cutoff value), thereby discriminating between the IGT and the NGT in the subject. Thus, the discriminant values obtained in the multivariate discriminants useful for discriminating between the 2 groups of the IGT and the NGT can be utilized to bring about an effect of enabling an accurate discrimination between the 2 groups of the IGT and the NGT.

In the step S-34, the discriminant value may be calculated based on both the concentration values of at least two of Glu, Gly, Ser, Ile, Val, Leu, Phe and Asp contained in the amino acid concentration data of the subject measured in the step S-33 and the multivariate discriminant containing at least two of Glu, Gly, Ser, Ile, Val, Leu, Phe and Asp as the explanatory variables, and the discrimination between the IGT and the NGT in the subject may be conducted based on the calculated discriminant value. Thus, the discriminant values obtained in the multivariate discriminants useful for discriminating between the 2 groups of the IGT and the NGT can be utilized to bring about an effect of enabling an accurate discrimination between the 2 groups of the IGT and the NGT.

The multivariate discriminant may be expressed by one fractional expression or the sum of a plurality of the fractional expressions, and may contain at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or may contain at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Specifically, the multivariate discriminant may be formula 1. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT. Glu/(His+Cit)+(Phe+Tyr)/Gly (formula 1)

The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Specifically, the multivariate discriminant may be the logistic regression equation containing at least Glu and Gly as the explanatory variables. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

The multivariate discriminants described above can be prepared by a method described in International Publication WO 2004/052191 Pamphlet that is an international application filed by the present applicant or by a method (multivariate discriminant-preparing processing described in the second embodiment described above) described in International Publication WO 2006/098192 Pamphlet that is an international application filed by the present applicant. Any multivariate discriminants obtained by these methods can be preferably used in the evaluation of the IGT state, regardless of the unit of the amino acid concentration in the amino acid concentration data as input data.

In the fractional expression, the numerator of the fractional expression is expressed by the sum of the amino acids A, B, C etc. and the denominator of the fractional expression is expressed by the sum of the amino acids a, b, c etc. The fractional expression also includes the sum of the fractional expressions $\alpha$, $\beta$, $\gamma$ etc. (for example, $\alpha+\beta$) having such constitution. The fractional expression also includes divided fractional expressions. The amino acids used in the numerator or denominator may have suitable coefficients respectively. The amino acids used in the numerator or denominator may appear repeatedly. Each fractional expression may have a suitable coefficient. A value of a coefficient for each explanatory variable and a value for a constant term may be any real numbers. In combinations where explanatory variables in the numerator and explanatory variables in the denominator in the fractional expression are switched with each other, the positive (or negative) sign is generally reversed in correlation with objective explanatory variables, but because their correlation is maintained, such combinations can be assumed to be equivalent to one another in discrimination, and thus the fractional expression also includes combinations where explanatory variables in the numerator and explanatory variables in the denominator in the fractional expression are switched with each other.

The multivariate discriminant refers to a form of equation used generally in multivariate analysis and includes, for example, multiple regression equation, multiple logistic regression equation, linear discriminant function, Mahalanobis' generalized distance, canonical discriminant function, support vector machine, and decision tree. The multivariate discriminant also includes an equation shown by the sum of different forms of multivariate discriminants. In the multiple regression equation, multiple logistic regression equation and canonical discriminant function, a coefficient and constant term are added to each explanatory variable, and the coefficient and constant term in this case are preferably real numbers, more preferably values in the range of 99% confidence interval for the coefficient and constant term obtained from data for discrimination, more preferably in the range of 95% confidence interval for the coefficient and constant term obtained from data for discrimination. The value of each coefficient and the confidence interval thereof may be those multiplied by a real number, and the value of each constant term and the confidence interval thereof may be those having an arbitrary actual constant added or subtracted or those multiplied or divided by an arbitrary actual constant. When an expression such as a logistic regression, a linear discriminant, and a multiple regression analysis is used as an index, a linear transformation of the expression (addition of a constant and multiplication by a constant) and a monotonic increasing (decreasing) transformation (for example, a logit transformation) of the expression do not alter discrimination capability, and thus are equivalent. Therefore, the expression includes an expression that is subjected to a linear transformation and a monotonic increasing (decreasing) transformation.

When the IGT state is evaluated (specifically, the discrimination between the IGT and the NGT is conducted) in the present invention, concentrations of other metabolites (biological metabolites), protein expression level, age and sex of the subject, biological indices or the like may be used in addition to the concentrations of the amino acids. When the IGT state is evaluated (specifically, the discrimination between the IGT and the NGT is conducted) in the present invention, concentrations of other metabolites (biological metabolites), protein expression level, age and sex of the subject, biological indices or the like may be used as the explanatory variables in the multivariate discriminants in addition to the concentrations of the amino acids.

3-2. An example of the method of searching for prophylactic/ameliorating substance for IGT according to the third embodiment

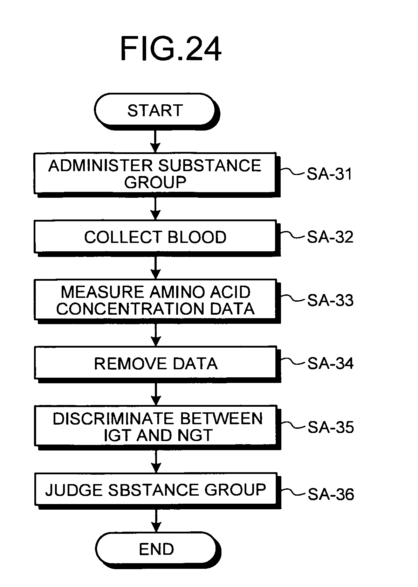

Here, an example of the method of searching for prophylactic/ameliorating substance for IGT according to the third embodiment is described with reference to FIG. 24. FIG. 24 is a flowchart showing an example of the method of searching for prophylactic/ameliorating substance for IGT according to the third embodiment.

First, a desired substance group consisting of one or more substances is administered to an individual such as an animal or a human with the IGT (step SA-31).

From the individual administered with the substance group in the step S-31, blood is then collected (step SA-32).

From the blood collected in the step S-32, the amino acid concentration data on the concentration values of the amino acids are measured (step SA-33). The measurement of the concentration values of the amino acids is conducted by the method described above.

From the amino acid concentration data of the individual measured in the step S-33, data such as defective and outliers is then removed (step SA-34).

Then, the concentration value of at least one or at least two of Glu, Gly, Ser, Ile, Val, Leu, Phe and Asp contained in the amino acid concentration data of the individual from which the data such as the defective and outliers was removed in the step SA-34 is compared with a previously established threshold (cutoff value), thereby discriminating between the IGT and the NGT in the individual (step SA-35).

Based on the discrimination results in the step SA-35, it is then judged whether or not the substance group administered in the step SA-31 prevents the IGT or ameliorates the IGT state (step SA-36).

When the judgment result obtained in the step SA-36 is "preventive or ameliorative", the substance group administered in the step SA-31 is searched as one preventing the IGT or ameliorating the IGT state. The substances searched by the searching method of the present embodiment include, for example, an amino acid group of Glu, Gly, Ser, Ile, Val, Leu, Phe and Asp.

3-3. Summary of the third embodiment and other embodiments

According to the method of searching for prophylactic/ameliorating substance for IGT according to the third embodiment described in detail above, (1) the desired substance group is administered to the individual, (2) the blood is collected from the individual administered with the substance group, (3) the amino acid concentration data are measured from the collected blood, (4) the data such as the defective and outliers is removed from the measured amino acid concentration data of the individual, (5) the concentration value of at least one or at least two of Glu, Gly, Ser, Ile, Val, Leu, Phe and Asp contained in the amino acid concentration data of the individual from which the data such as the defective and outliers was removed is compared with the previously established threshold (cutoff value), thereby discriminating between the IGT and the NGT in the individual, and (6) based on the discrimination result, it is judged whether or not the administered substance group prevents the IGT or ameliorates the IGT state. Thus, the method of evaluating IGT of the first embodiment described above capable of accurately evaluating the IGT state by utilizing the concentrations of the amino acids in blood can be used to bring about an effect of enabling an accurate search for the substance for preventing the IGT or ameliorating the IGT state.

In the step SA-35, (a) the discriminant value may be calculated based on both (i) the concentration value of at least one or at least two of Glu, Gly, Ser, Ile, Val, Leu, Phe and Asp contained in the amino acid concentration data of the individual from which the data such as the defective and outliers have been removed in the step SA-34 and (ii) the multivariate discriminant containing at least one or at least two of Glu, Gly, Ser, Ile, Val, Leu, Phe and Asp as the explanatory variable, and (b) the calculated discriminant value may be compared with the previously established threshold (cutoff value), thereby discriminating between the IGT and the NGT in the individual. Thus, the discriminant values obtained in the multivariate discriminants useful for discriminating between the 2 groups of the IGT and the NGT can be utilized to bring about an effect of enabling an accurate discrimination between the 2 groups of the IGT and the NGT.

In the step SA-35, the multivariate discriminant may be expressed by one fractional expression or the sum of a plurality of the fractional expressions, and may contain at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or may contain at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant. Specifically, the multivariate discriminant may be formula 1. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

$$\mathtt{Glu/(His+Cit)+(Phe+Tyr)/Gly} \qquad \mathtt{(formula\ 1)}$$

In the step SA-35, the multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree. Specifically, the multivariate discriminant may be the logistic regression equation containing at least Glu and Gly as the explanatory variables. Thus, discriminant values obtained in multivariate discriminants useful particularly for discriminating between the 2 groups of the IGT and the NGT can be utilized to bring about an effect of enabling a more accurate discrimination between the 2 groups of the IGT and the NGT.

The multivariate discriminants described above can be prepared by a method described in International Publication WO 2004/052191 Pamphlet that is an international application filed by the present applicant or by a method (multivariate discriminant-preparing processing described in the second embodiment described above) described in International Publication WO 2006/098192 Pamphlet that is an international application filed by the present applicant. Any multivariate discriminants obtained by these methods can be preferably used in the evaluation of the IGT state, regardless of the unit of the amino acid concentration in the amino acid concentration data as input data.

In the method of searching for prophylactic/ameliorating substance for IGT according to the third embodiment, substances that restore normal values to the concentration values of the amino acid group containing at least one or at least two of Glu, Gly, Ser, Ile, Val, Leu, Phe and Asp or the discriminant values of each of the multivariate discriminants, can be selected by the method of evaluating IGT in the first embodiment described above or by the IGT-evaluating apparatus in the second embodiment described above.

In the method of searching for prophylactic/ameliorating substance for IGT in the third embodiment, "searching for prophylactic/ameliorating substance" includes not only discovery of a novel substance effective in preventing and ameliorating the IGT, but also (1) new discovery of use of a known substance in preventing and ameliorating the IGT, (2) discovery of a novel composition consisting of a combination of existing drugs and supplements having efficacy expectable for prevention and amelioration of the IGT, (3) discovery of the suitable usage, dose and combination described above to form them into a kit, (4) presentation of a prophylactic and therapeutic menu including a diet, exercise etc., and (5) presentation of a necessary change in menu for each individual by monitoring the effect of the prophylactic and therapeutic menu.

Example 1

From among those who received a consultation for OGTT in a comprehensive medical examination, 304 subjects whose FPG were less than 110 mg/dl were classified into a NGT (normal glucose tolerance) group (less than 140 mg/dl, 248 subjects) and an IGT group (equal to or more than 140 mg/dl, 56 patients) based on the diagnostic criteria associated with a 2-hour OGTT level. The blood amino acid concentrations were measured by the amino acid analysis method described above from each of the blood samples collected from the subjects. Those who were receiving treatment for disease such as hypertension and hyperlipidemia were not included in the 304 subjects.

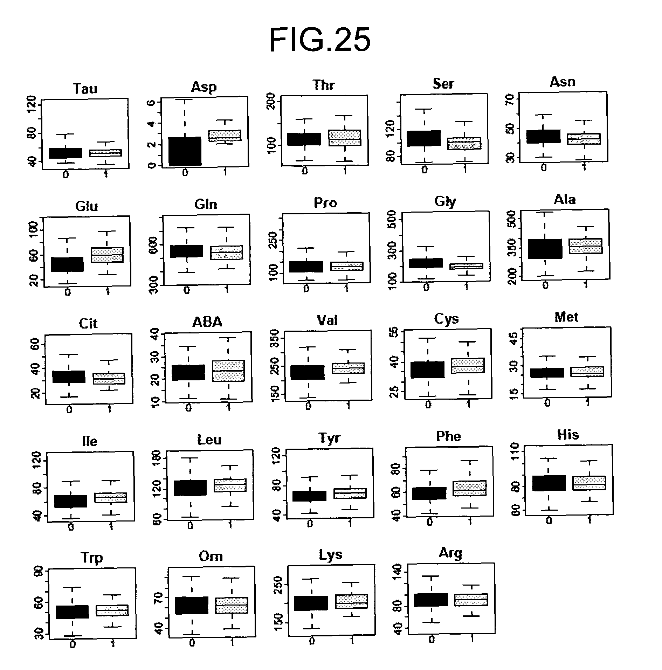

In FIG. 25, boxplots showing a distribution of the amino acid explanatory variables between the 2 groups of the NGT and the IGT are represented. In FIG. 25, the numeral reference "0" indicated in the horizontal axis of the boxplot represents the NGT group, while the numeral reference "1" represents the IGT group. In the figure, the abbreviation "ABA" represents $\alpha$-amino butyric acid, the abbreviation "Cys" represents cystine, and the abbreviation "Cit" represents citrulline. For the purpose of a discrimination between the 2 groups, Welch's t-test was carried out between the 2 groups.

In the IGT group as compared with the NGT group, Glu, Ile, Val, Leu, Phe and Asp were significantly increased (significant difference probability $P < 0.05$), and Gly and Ser were significantly decreased. It became clear that the amino acid explanatory variables Glu, Gly, Ser, Ile, Val, Leu, Phe and Asp have an ability for the discrimination between the 2 groups.

Example 2

The sample data used in Example 1 were used. An index formula that would maximize the discrimination capability between the 2 groups of the NGT and the IGT, was thoroughly searched using a method described in International Publication WO 2004/052191 Pamphlet. As a result, an index formula "(Glu)/(His+Cit)+(Phe+Tyr)/(Gly)" was obtained as an example of a plurality of multivariate discriminants having equivalent capabilities.

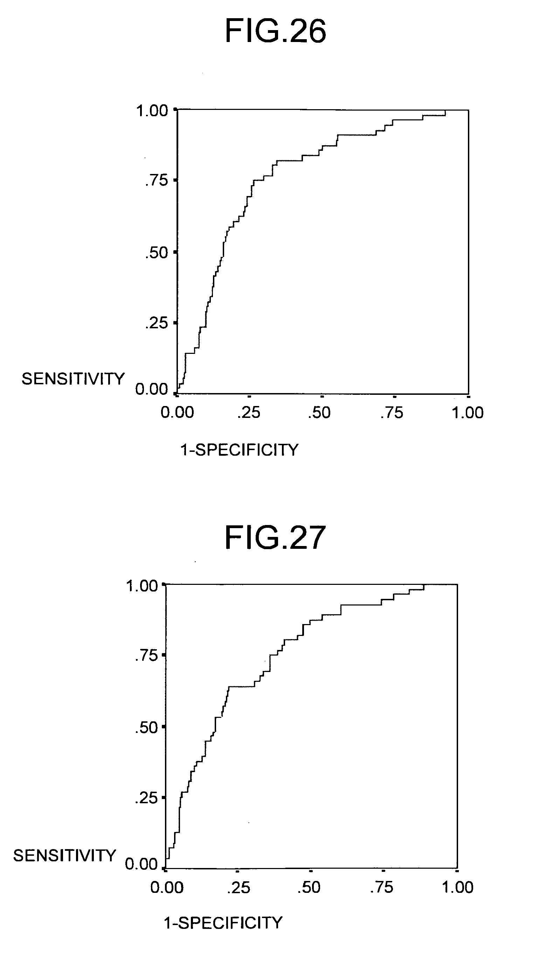

Diagnostic capability (discrimination capability) of the index formula between the 2 groups was evaluated by an AUC (area under the curve) of ROC curve (receiver operating characteristic curve) (Fig. 26). As a result, an AUC of 0.78 (95% confidence interval was 0.70 to 0.83) was obtained.

Example 3

The sample data used in Example 1 were used. An index that would maximize the discrimination capability between the 2 groups of the NGT and the IGT was thoroughly searched using a method of searching a multivariate discriminant described in International Publication WO 2006/098192 Pamphlet. As the method of searching the multivariate discriminant, a multivariate discriminant given by a logistic regression, a linear discrimination, a support vector machine, Mahalanobis' generalized distance method, and the like can be used. When multivariate discriminants were thoroughly searched by using a stepwise logistic analysis as an example, a logistic regression equation composed of Glu and Gly (numerical coefficients of amino acid explanatory variables Glu and Gly and constant terms were, $0.039 \pm 0.010$, $-0.013 \pm 0.005$, and $-0.788 \pm 1.187$, in sequence) was obtained as an index formula.

Diagnostic capability (discrimination capability) of the index formula between the 2 groups was evaluated by the AUC of the ROC curve (Fig. 27). As a result, an AUC of 0.75 (95% confidence interval was 0.69 to 0.82) was obtained, based on which the index formula was found to be a useful index with high discrimination capability.

An optimal cut-off value for discrimination between the 2 groups performed by the index formula was obtained (specifically, a cut-off value was obtained with respect to an explanatory variable obtained by subjecting a probability given by a logistic analysis to a logit transformation). As a result, when prevalence was provided as 0.18, the cut-off value was -0.64, and sensitivity was 64%, specificity was 71%, a positive predictive value was 33%, a negative predictive value was 90%, and efficiency was 69%, based on which the index formula was found to be a useful index with high discrimination capability.

As an example other than an index formula obtained by a logistic analysis, multivariate discriminants given by a linear discrimination, a support vector machine, and Mahalanobis' generalized distance method were studied using the same data as above. Subsequently, diagnostic capability of each discriminant between the 2 groups was evaluated. As a result, in sequence, an AUC of ROC curve was 0.74 with respect to an index formula having Glu, Gly, and Met as explanatory variables in a linear discrimination; an error rate was 17.8% with respect to an index formula having Glu, Gln, Gly, and Ala as explanatory variables in a support vector machine; and an error rate was 18.7% with respect to an index formula having Asp, Glu, Gly, Cys, Trp, and Arg as explanatory variables in Mahalanobis' generalized distance method, based on which each index formula was found to be a useful index with high discrimination capability similarly to the index formula obtained by a logistic regression.

Example 4

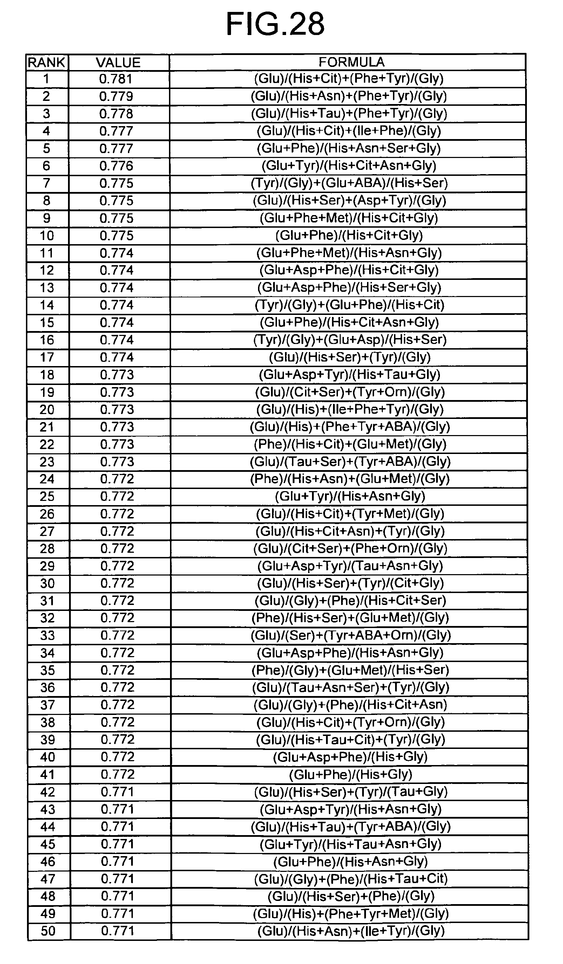

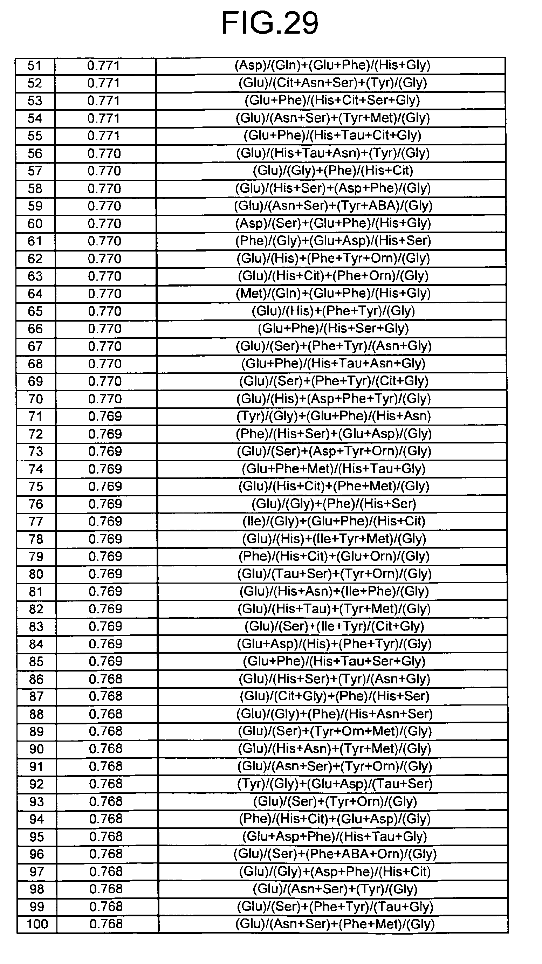

The sample data used in Example 1 were used. An index that would maximize the discrimination capability between the 2 groups of the NGT and the IGT was thoroughly searched using a method described in International Publication WO 2004/052191 Pamphlet. As a result, a plurality of index formulae having an equivalent capability was obtained. In FIG. 28 and 29, lists of AUCs of ROC curves with respect to diagnostic capability (discrimination capability) of the index formula between the 2 groups are represented.

Example 5

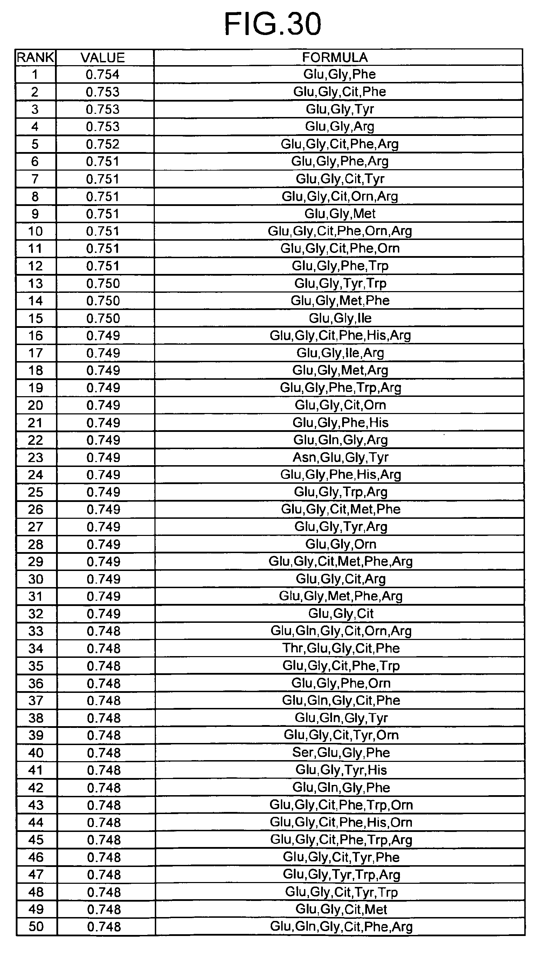

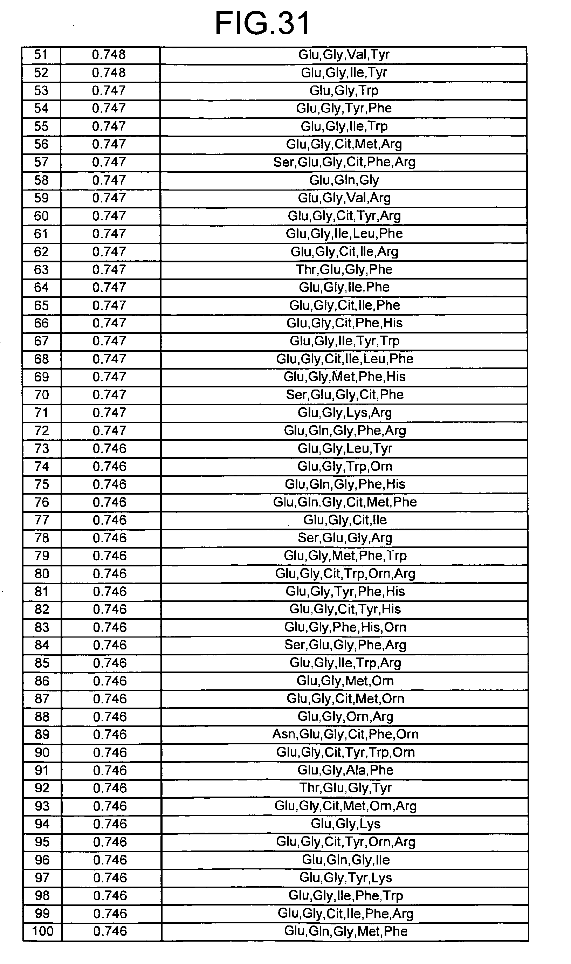

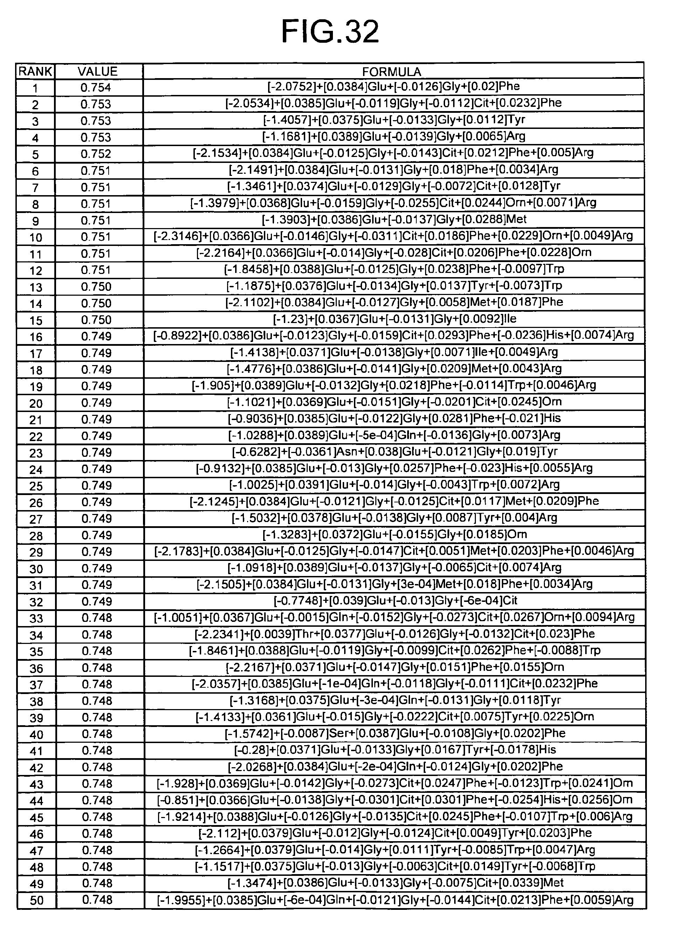

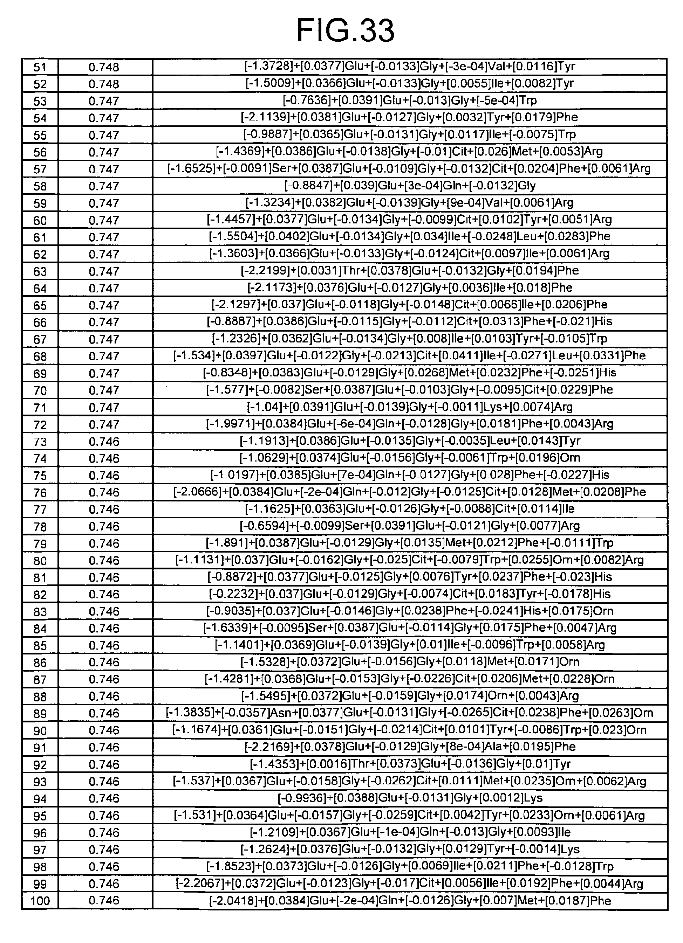

The sample data used in Example 1 were used. An index that would maximize the discrimination capability between the 2 groups of the NGT and the IGT was searched by a logistic analysis using a method described in International Publication WO 2006/098192 Pamphlet. As a result, a plurality of index formulae having an equivalent capability was obtained. In FIGS. 30 and 31, lists of AUCs of ROC curves with respect to diagnostic capability (discrimination capability) of the index formula between the 2 groups are represented. In FIGS. 32 and 33, lists of logistic regression equations including numerical coefficients are represented.

Example 6

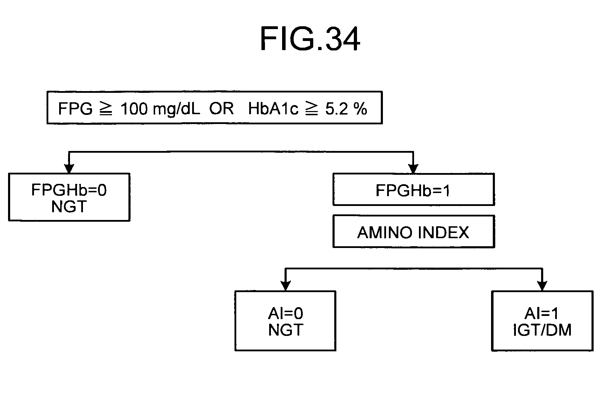

The sample data used in Example 1 were used. A 2-step discrimination shown in FIG. 34 was conducted for discrimination between the 2 groups of the NGT and the IGT. Specifically, in a first step, "those whose FPG were equal to or more than 100 mg/dl or those whose hemoglobin Alc (HbAlc) were equal to or more than 5.2%" were discriminated as positive (FPGHb=1) and others were discriminated as negative (FPGHb=0). Then, in a second step, with respect to the group of the subjects discriminated as positive (FPGHb=1) in the first step, an index formula for discrimination between the 2 groups of the NGT and the IGT was calculated by stepwise logistic analysis using a method described in International Publication WO 2006/098192 Pamphlet. As a result, a logistic regression equation composed of Glu and Gly as an index formula was obtained. Those who were found to be positive (AI=1) by the index formula were discriminated to have Impaired glucose tolerance (IGT/DM). Then, those who were found to be negative (FPGHb=0) in the first step or negative (AI=0) in the second step were discriminated as normal glucose tolerance (NGT).

Regarding discrimination between the 2 groups of the NGT and the IGT, sensitivity was 75%, specificity was 46%, a positive predictive value was 24%, a negative predictive value was 89%, and efficiency was 51% as obtained by the first step alone. However, once the second step was added, sensitivity was 68%, specificity was 77%, a positive predictive value was 40%, a negative predictive value was 91%, and efficiency was 75%. As results, the index formula obtained in the second step was found to be a useful index with high discrimination capability.

Example 7

From among those who received a consultation for OGTT in a comprehensive medical examination that was conducted at a different time from Example 1, 90 subjects whose FPG were less than 110 mg/dl were classified into a NGT group (less than 140 mg/dl, 60 subjects) and an IGT group (equal to or more than 140 mg/dl, 30 patients) based on the diagnostic criteria associated with a 2-hour OGTT level. Those who were receiving treatment for a disease such as hypertension and hyperlipidemia were not included in the 90 subjects. Then, a blood amino acid concentration was measured in a sample taken from each subject by an amino acid analysis method employing a LC-MS method, which was different from the ninhydrin method described herein.
A technical content of the LC-MS method employed herein was described in International Publication WO 2003/069328 Pamphlet, which was directed to an analytical method and an analytical reagent for an amino functional compound, and in International Publication WO 2005/116629 Pamphlet, which was directed to an analytical method and an apparatus for an amino functional compound.

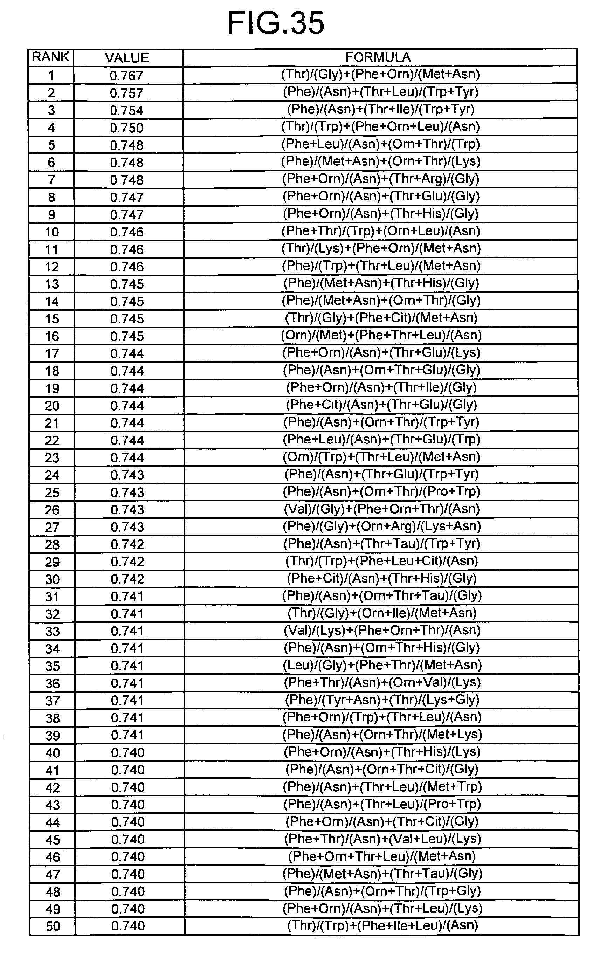

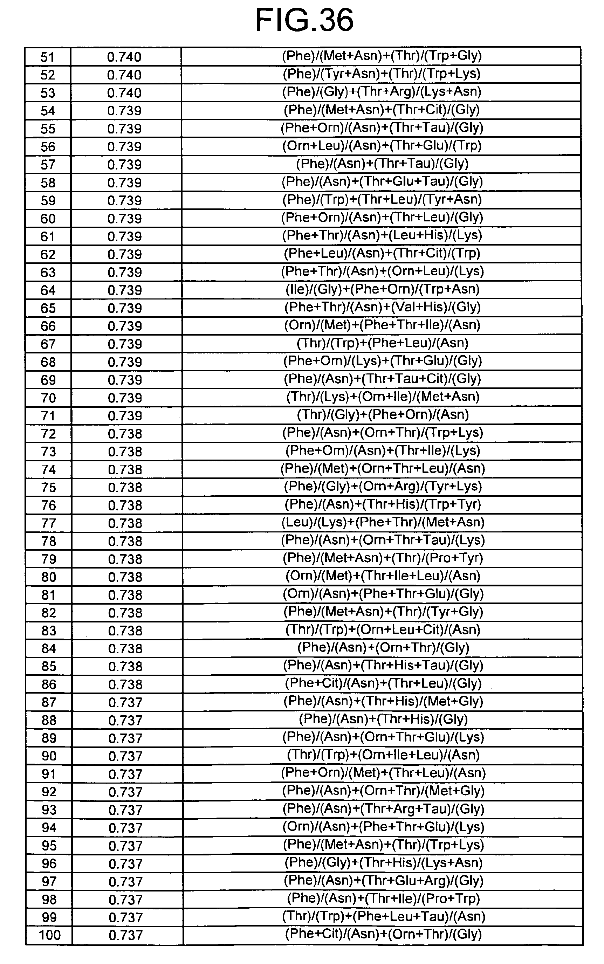

An index that would maximize the discrimination capability between the 2 groups of the NGT and the IGT was thoroughly searched using the measured sample data and a method described in International Publication WO 2004/052191 Pamphlet. As a result, a plurality of index formulae that were expressed as fractional expressions and had an equivalent capability were obtained. In FIGS. 35 and 36, lists of AUCs of ROC curves with respect to diagnostic capability (discrimination capability) of the index formulae between the 2 groups are represented.

Example 8

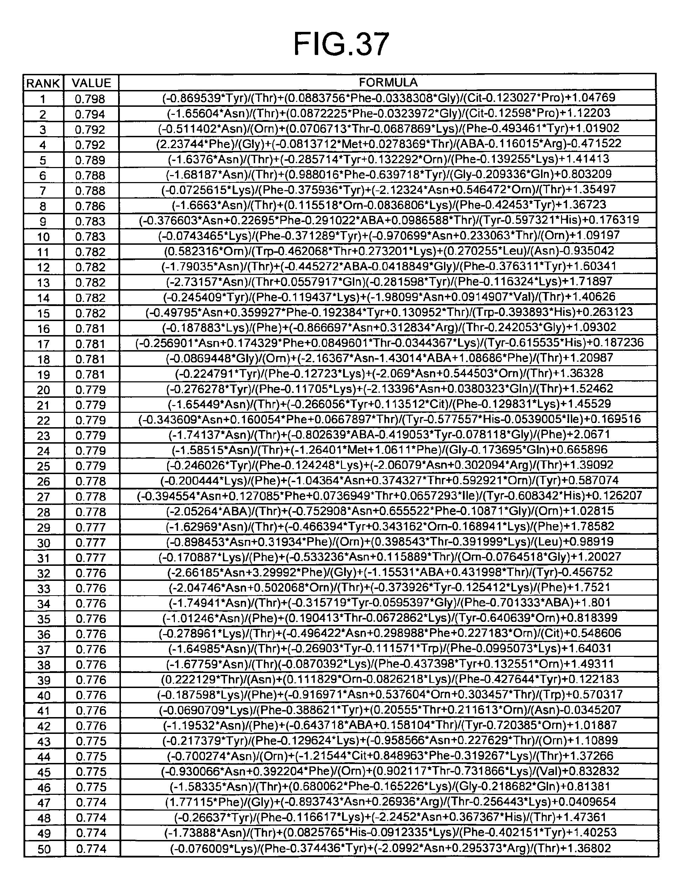

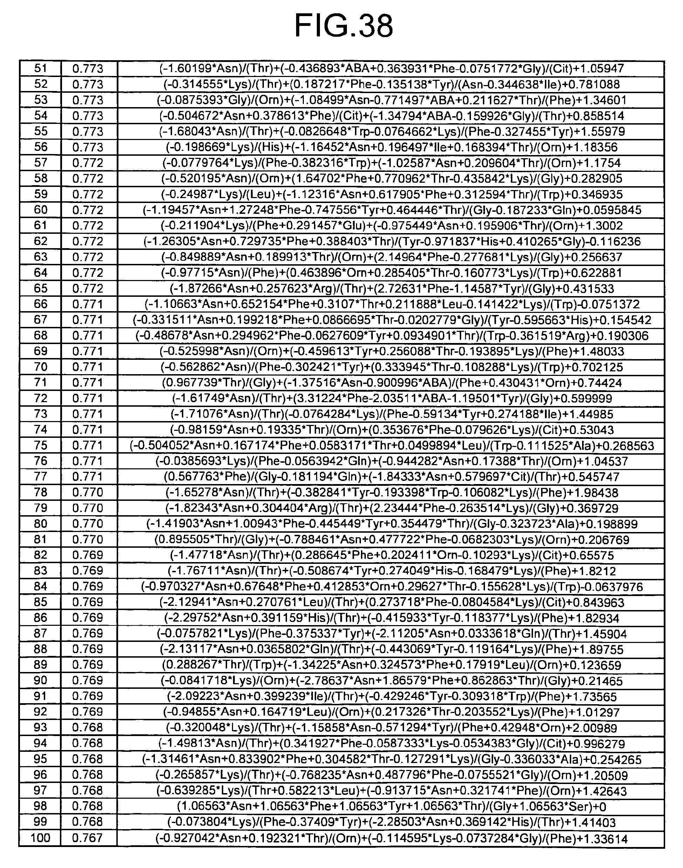

The sample data used in Example 7 were used. An index that would maximize the discrimination capability between the 2 groups of the NGT and the IGT was thoroughly searched using a method described in International Publication WO 2004/052191 Pamphlet. As a result, a plurality of index formulae that were expressed as fractional expressions with real coefficients and had an equivalent capability were obtained. In FIGS. 37 and 38, lists of AUCs of ROC curves and the fractional expressions with real coefficients with respect to diagnostic capability (discrimination capability) of the index formulae between the 2 groups are represented.

Example 9

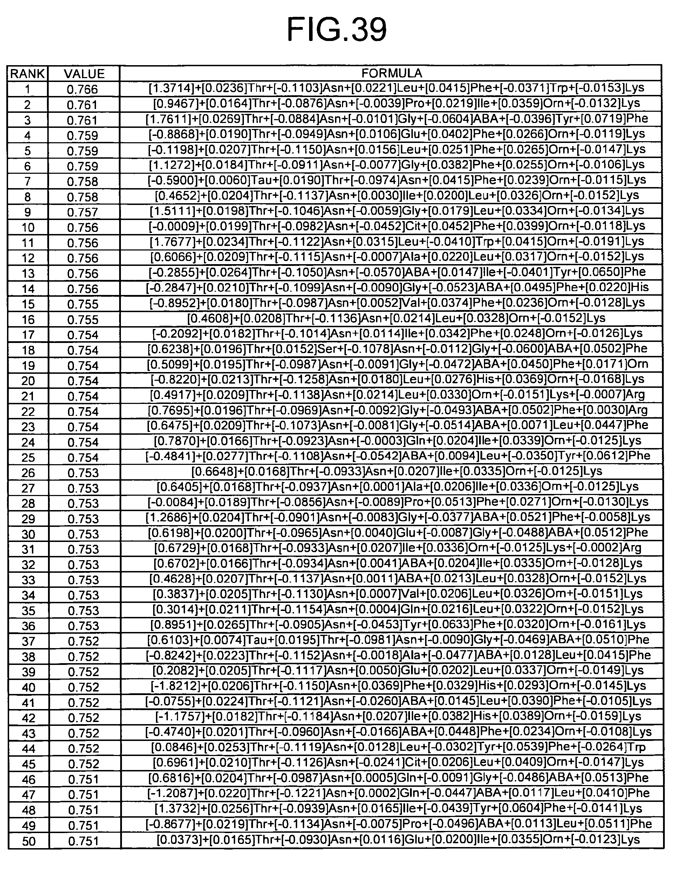

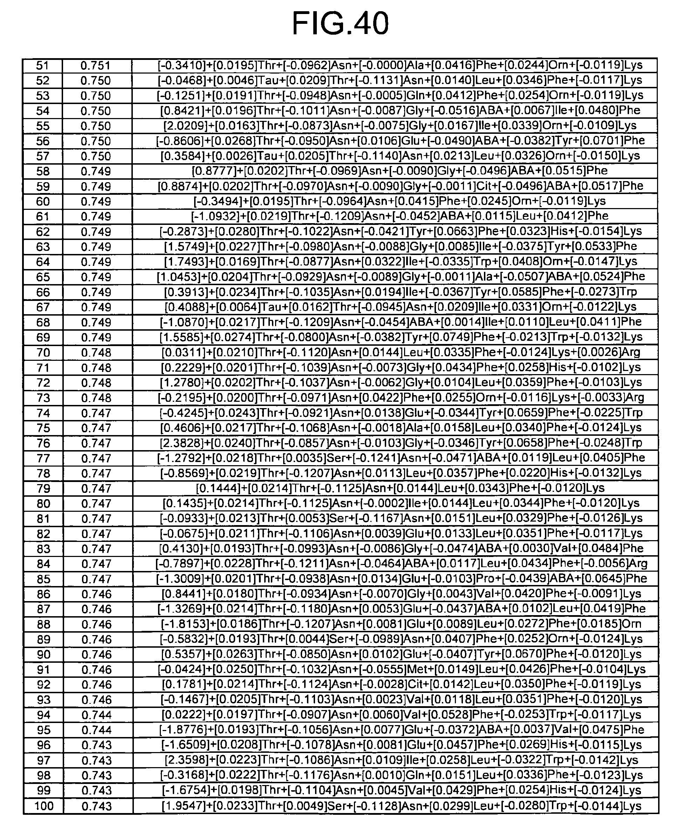

The sample data used in Example 7 were used. An index that would maximize the discrimination capability between the 2 groups of the NGT and the IGT was searched by a logistic analysis using a method described in International Publication WO 2006/098192 Pamphlet. As a result, a plurality of index formulae having an equivalent capability was obtained. In FIGS. 39 and 40, lists of AUCs of ROC curves and logistic regression equations including numerical coefficients with respect to diagnostic capability (discrimination capability) of the index formulae between the 2 groups are represented.

INDUSTRIAL APPLICABILITY

As described above, the method of evaluating IGT, the IGT-evaluating apparatus, the IGT-evaluating method, the IGT-evaluating system, the IGT-evaluating program, the recording medium, and the method of searching for prophylactic/ameliorating substance for IGT according to the present invention may be practiced widely in many industrial fields, in particular in pharmaceutical, food, and medical fields, and are extremely useful in the field of bioinformatics, for example, performing prediction of progression of the IGT state, disease risk prediction, and proteome or metabolome analysis.

**Claims**

1. A method of evaluating IGT, comprising:

   a measuring step of measuring amino acid concentration data on a concentration value of an amino acid in blood collected from a subject to be evaluated; and
   a concentration value criterion evaluating step of evaluating an impaired glucose tolerance state in the subject based on the amino acid concentration data of the subject measured at the measuring step.

2. The method of evaluating IGT according to claim 1, wherein at the concentration value criterion evaluating step, the impaired glucose tolerance state in the subject is evaluated based on the concentration value of at least one of Glu, Gly, Ser, Ile, Val, Leu, Phe and Asp contained in the amino acid concentration data of the subject measured at the measuring step.

3. The method of evaluating IGT according to claim 2, wherein the concentration value criterion evaluating step further includes a concentration value criterion discriminating step of discriminating between an impaired glucose tolerance and a normal glucose tolerance in the subject based on the concentration value of at least one of Glu, Gly, Ser, Ile, Val, Leu, Phe and Asp contained in the amino acid concentration data of the subject measured at the measuring step.

4. The method of evaluating IGT according to claim 3, wherein at the concentration value criterion discriminating step, the discrimination between the impaired glucose tolerance and the normal glucose tolerance in the subject is conducted based on the concentration values of at least two of Glu, Gly, Ser, Ile, Val, Leu, Phe and Asp contained in the amino acid concentration data of the subject measured at the measuring step.

5. The method of evaluating IGT according to claim 1, wherein the concentration value criterion evaluating step further includes:

   a discriminant value calculating step of calculating a discriminant value that is a value of a multivariate discriminant with a concentration of the amino acid as an explanatory variable, based on both the amino acid concentration data of the subject measured at the measuring step and the previously established multivariate discriminant; and
   a discriminant value criterion evaluating step of evaluating the impaired glucose tolerance state in the subject based on the discriminant value calculated at the discriminant value calculating step.

6. The method of evaluating IGT according to claim 5, wherein the multivariate discriminant contains at least one of Glu, Gly, Ser, Ile, Val, Leu, Phe and Asp as the explanatory variable, and
   wherein at the discriminant value calculating step, the discriminant value is calculated based on both the concentration value of at least one of Glu, Gly, Ser, Ile, Val, Leu, Phe and Asp contained in the amino acid concentration data of the subject measured at the measuring step and the multivariate discriminant.

7. The method of evaluating IGT according to claim 6, wherein the discriminant value criterion evaluating step further includes a discriminant value criterion discriminating step of discriminating between an impaired glucose tolerance and a normal glucose tolerance in the subject based on the discriminant value calculated at the discriminant value calculating step.

8. The method of evaluating IGT according to claim 7, wherein the multivariate discriminant contains at least two of Glu, Gly, Ser, Ile, Val, Leu, Phe and Asp as the explanatory variables, and
   wherein at the discriminant value calculating step, the discriminant value is calculated based on both the concentration values of at least two of Glu, Gly, Ser, Ile, Val, Leu, Phe and Asp contained in the amino acid concentration data of the subject measured at the measuring step and the multivariate discriminant.

9. The method of evaluating IGT according to claim 8, wherein the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant.

10. The method of evaluating IGT according to claim 9, wherein the multivariate discriminant is formula 1:

```
Glu/(His+Cit)+(Phe+Tyr)/Gly            (formula 1)
```

.

11. The method of evaluating IGT according to claim 8, wherein the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree.

12. The method of evaluating IGT according to claim 11, wherein the multivariate discriminant is the logistic regression equation containing at least Glu and Gly as the explanatory variables.

13. An IGT-evaluating apparatus comprising a control unit and a memory unit to evaluate an impaired glucose tolerance state in a subject to be evaluated, wherein the control unit includes:

a discriminant value-calculating unit that calculates a discriminant value that is a value of a multivariate discriminant with a concentration of an amino acid as an explanatory variable, based on both previously obtained amino acid concentration data of the subject on a concentration value of the amino acid and the multivariate discriminant stored in the memory unit; and
a discriminant value criterion-evaluating unit that evaluates the impaired glucose tolerance state in the subject based on the discriminant value calculated by the discriminant value-calculating unit.

14. The IGT-evaluating apparatus according to claim 13, wherein the multivariate discriminant contains at least one of Glu, Gly, Ser, Ile, Val, Leu, Phe and Asp as the explanatory variable, and
wherein the discriminant value-calculating unit calculates the discriminant value based on both the concentration value of at least one of Glu, Gly, Ser, Ile, Val, Leu, Phe and Asp contained in the previously obtained amino acid concentration data of the subject and the multivariate discriminant.

15. The IGT-evaluating apparatus according to claim 14, wherein the discriminant value criterion-evaluating unit further includes a discriminant value criterion-discriminating unit that discriminates between an impaired glucose tolerance and a normal glucose tolerance in the subject based on the discriminant value calculated by the discriminant value-calculating unit.

16. The IGT-evaluating apparatus according to claim 15, wherein the multivariate discriminant contains at least two of Glu, Gly, Ser, Ile, Val, Leu, Phe and Asp as the explanatory variables, and
wherein the discriminant value-calculating unit calculates the discriminant value based on both the concentration values of at least two of Glu, Gly, Ser, Ile, Val, Leu, Phe and Asp contained in the previously obtained amino acid concentration data of the subject and the multivariate discriminant.

17. The IGT-evaluating apparatus according to claim 16, wherein the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant.

18. The IGT-evaluating apparatus according to claim 17, wherein the multivariate discriminant is formula 1:

```
Glu/(His+Cit)+(Phe+Tyr)/Gly                    (formula 1)
```

19. The IGT-evaluating apparatus according to claim 16, wherein the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree.

20. The IGT-evaluating apparatus according to claim 19, wherein the multivariate discriminant is the logistic regression equation containing at least Glu and Gly as the explanatory variables.

21. The IGT-evaluating apparatus according to any one of claims 13 to 20, wherein the control unit further includes a multivariate discriminant-preparing unit that prepares the multivariate discriminant stored in the memory unit, based on impaired glucose tolerance state information stored in the memory unit containing the amino acid concentration data and impaired glucose tolerance state index data on an index for indicating the impaired glucose tolerance state, wherein the multivariate discriminant-preparing unit further includes:

   a candidate multivariate discriminant-preparing unit that prepares a candidate multivariate discriminant that is a candidate of the multivariate discriminant, based on a predetermined discriminant-preparing method from the impaired glucose tolerance state information;
   a candidate multivariate discriminant-verifying unit that verifies the candidate multivariate discriminant prepared by the candidate multivariate discriminant-preparing unit, based on a predetermined verifying method; and
   an explanatory variable-selecting unit that selects the explanatory variable of the candidate multivariate discriminant based on a predetermined explanatory variable-selecting method from a verification result obtained by the candidate multivariate discriminant-verifying unit, thereby selecting a combination of the amino acid concentration data contained in the impaired glucose tolerance state information used in preparing the candidate multivariate discriminant, and
   wherein the multivariate discriminant-preparing unit prepares the multivariate discriminant by selecting the candidate multivariate discriminant used as the multivariate discriminant, from a plurality of the candidate multivariate discriminants, based on the verification results accumulated by repeatedly executing the candidate multivariate discriminant-preparing unit, the candidate multivariate discriminant-verifying unit, and the explanatory variable-selecting unit.

22. An IGT-evaluating method of evaluating an impaired glucose tolerance state in a subject to be evaluated, the method is carried out with an information processing apparatus including a control unit and a memory unit, the method comprising:

   (i) a discriminant value calculating step of calculating a discriminant value that is a value of a multivariate discriminant with a concentration of an amino acid as an explanatory variable, based on both previously obtained amino acid concentration data of the subject on a concentration value of the amino acid and the multivariate discriminant stored in the memory unit; and
   (ii) a discriminant value criterion evaluating step of evaluating the impaired glucose tolerance state in the subject based on the discriminant value calculated at the discriminant value calculating step,
   wherein the steps (i) and (ii) are executed by the control unit.

23. The IGT-evaluating method according to claim 22, wherein the multivariate discriminant contains at least one of Glu, Gly, Ser, Ile, Val, Leu, Phe and Asp as the explanatory variable, and
   wherein at the discriminant value calculating step, the discriminant value is calculated based on both the concentration value of at least one of Glu, Gly, Ser, Ile, Val, Leu, Phe and Asp contained in the previously obtained amino acid concentration data of the subject and the multivariate discriminant.

24. The IGT-evaluating method according to claim 23, wherein the discriminant value criterion evaluating step further includes a discriminant value criterion discriminating step of discriminating between an impaired glucose tolerance and a normal glucose tolerance in the subject based on the discriminant value calculated at the discriminant value calculating step.

25. The IGT-evaluating method according to claim 24, wherein the multivariate discriminant contains at least two of Glu, Gly, Ser, Ile, Val, Leu, Phe and Asp as the explanatory variables, and
wherein at the discriminant value calculating step, the discriminant value is calculated based on both the concentration values of at least two of Glu, Gly, Ser, Ile, Val, Leu, Phe and Asp contained in the previously obtained amino acid concentration data of the subject and the multivariate discriminant.

26. The IGT-evaluating method according to claim 25, wherein the multivariate discriminant is expressed by one fractional expression or the sum of a plurality of the fractional expressions, and contains at least one of Glu, Ile, Val, Leu, Phe and Asp as the explanatory variable in the numerator in the fractional expression constituting the multivariate discriminant, and at least one of Gly and Ser as the explanatory variable in the denominator in the fractional expression constituting the multivariate discriminant, or contains at least one of Gly and Ser as the explanatory variable in the numerator in the fractional expression constituting the multivariate discriminant, and at least one of Glu, Ile, Val, Leu, Phe and Asp as the explanatory variable in the denominator in the fractional expression constituting the multivariate discriminant.

27. The IGT-evaluating method according to claim 26, wherein the multivariate discriminant is formula 1:

$$Glu/(His+Cit)+(Phe+Tyr)/Gly \qquad (formula\ 1)$$

.

28. The IGT-evaluating method according to claim 25, wherein the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis, and a discriminant prepared by a decision tree.

29. The IGT-evaluating method according to claim 28, wherein the multivariate discriminant is the logistic regression equation containing at least Glu and Gly as the explanatory variables.

30. The IGT-evaluating method according to any one of claims 22 to 29, wherein the method further includes a multivariate discriminant preparing step of preparing the multivariate discriminant stored in the memory unit, based on impaired glucose tolerance state information stored in the memory unit containing the amino acid concentration data and impaired glucose tolerance state index data on an index for indicating the impaired glucose tolerance state, that is executed by the control unit,
wherein the multivariate discriminant preparing step further includes:

a candidate multivariate discriminant preparing step of preparing a candidate multivariate discriminant that is a candidate of the multivariate discriminant, based on a predetermined discriminant-preparing method from the impaired glucose tolerance state information;
a candidate multivariate discriminant verifying step of verifying the candidate multivariate discriminant prepared at the candidate multivariate discriminant preparing step, based on a predetermined verifying method; and
an explanatory variable selecting step of selecting the explanatory variable of the candidate multivariate discriminant based on a predetermined explanatory variable-selecting method from a verification result obtained at the candidate multivariate discriminant verifying step, thereby selecting a combination of the amino acid concentration data contained in the impaired glucose tolerance state information used in preparing the candidate multivariate discriminant, and
wherein at the multivariate discriminant preparing step, the multivariate discriminant is prepared by selecting the candidate multivariate discriminant used as the multivariate discriminant from a plurality of the candidate multivariate discriminants, based on the verification results accumulated by repeatedly executing the candidate multivariate discriminant preparing step, the candidate multivariate discriminant verifying step, and the explanatory variable selecting step.

**31.** An IGT-evaluating system comprising an IGT-evaluating apparatus including a control unit and a memory unit to evaluate an impaired glucose tolerance state in a subject to be evaluated and an information communication terminal apparatus that provides amino acid concentration data of the subject on a concentration value of an amino acid connected to each other communicatively via a network,

wherein the information communication terminal apparatus includes:

an amino acid concentration data-sending unit that transmits the amino acid concentration data of the subject to the IGT-evaluating apparatus; and

an evaluation result-receiving unit that receives an evaluation result on the impaired glucose tolerance state of the subject transmitted from the IGT-evaluating apparatus,

wherein the control unit of the IGT-evaluating apparatus includes:

an amino acid concentration data-receiving unit that receives the amino acid concentration data of the subject transmitted from the information communication terminal apparatus;

a discriminant value-calculating unit that calculates a discriminant value that is a value of a multivariate discriminant with a concentration of the amino acid as an explanatory variable, based on both the amino acid concentration data of the subject received by the amino acid concentration data-receiving unit and the multivariate discriminant stored in the memory unit;

a discriminant value criterion-evaluating unit that evaluates the impaired glucose tolerance state in the subject based on the discriminant value calculated by the discriminant value-calculating unit; and

an evaluation result-sending unit that transmits the evaluation result of the subject obtained by the discriminant value criterion-evaluating unit to the information communication terminal apparatus.

**32.** An IGT-evaluating program product that makes an information processing apparatus including a control unit and a memory unit execute a method of evaluating an impaired glucose tolerance state in a subject to be evaluated, the method comprising:

(i) a discriminant value calculating step of calculating a discriminant value that is a value of a multivariate discriminant with a concentration of an amino acid as an explanatory variable, based on both previously obtained amino acid concentration data of the subject on a concentration value of the amino acid and the multivariate discriminant stored in the memory unit; and

(ii) a discriminant value criterion evaluating step of evaluating the impaired glucose tolerance state in the subject based on the discriminant value calculated at the discriminant value calculating step,

wherein the steps (i) and (ii) are executed by the control unit.

**33.** A computer-readable recording medium, comprising the IGT-evaluating program product according to claim 32 recorded thereon.

**34.** A method of searching for prophylactic/ameliorating substance for IGT, comprising:

a measuring step of measuring amino acid concentration data on a concentration value of an amino acid in blood collected from a subject to be evaluated to which a desired substance group consisting of one or more substances that prevent an impaired glucose tolerance or ameliorate an impaired glucose tolerance state has been administered;

a concentration value criterion evaluating step of evaluating an impaired glucose tolerance state in the subject based on the amino acid concentration data measured at the measuring step; and

a judging step of judging whether or not the desired substance group prevents the impaired glucose tolerance or ameliorates the impaired glucose tolerance state, based on an evaluation result obtained at the concentration value criterion evaluating step.

# FIG.1

(BASIC PRINCIPLE OF THE INVENTION)

BLOOD SAMPLE OF
SUBJECT TO BE EVALUATED

S-11
MEASUREMENT OF AMINO ACID CONCENTRATION DATA

S-12
EVALUATION OF IGT STATE

AMINO ACID CONCENTRATION DATA OF SUBJECT TO BE EVALUATED

EVALUATION RESULTS

# FIG.2

START

MEASURE AMINO ACID CONCENTRATION DATA — SA-11

REMOVE DATA — SA-12

DISCRIMINATE BETWEEN IGT AND NGT — SA-13

END

# FIG.3

(BASIC PRINCIPLE OF THE INVENTION)

IGT-EVALUATING
APPARATUS

| MEMORY DEVICE | CONTROL DEVICE |
| --- | --- |
| MULTIVARIATE DISCRIMINANT | S-21 CALCULATION OF DISCRIMINANT VALUE |
| | S-22 EVALUATION OF IGT STATE |

AMINO ACID CONCENTRATION DATA OF SUBJECT TO BE EVALUATED

EVALUATION RESULTS

# FIG.4

200

CLIENT APPARATUS
(INFORMATION
COMMUNICATION
TERMINAL APPARATUS)

200

CLIENT APPARATUS
(INFORMATION
COMMUNICATION
TERMINAL APPARATUS)

200

CLIENT APPARATUS
(INFORMATION
COMMUNICATION
TERMINAL APPARATUS)

200

CLIENT APPARATUS
(INFORMATION
COMMUNICATION
TERMINAL APPARATUS)

100

IGT-
EVALUATING
APPARATUS

300

NETWORK

# FIG.5

# FIG.6

IGT-EVALUATING APPARATUS (100)

INPUT DEVICE (112)  OUTPUT DEVICE (114)

INPUT/OUTPUT INTERFACE (108)

**MEMORY DEVICE (106)**
- USER INFORMATION FILE (106a)
- AMINO ACID CONCENTRATION DATA FILE (106b)
- IGT STATE INFORMATION FILE (106c)
- DESIGNATED IGT STATE INFORMATION FILE (106d)
- MULTIVARIATE DISCRIMINATE-RELATED INFORMATION DATABASE (106e)
  - CANDIDATE MULTIVARIATE DISCRIMINANT FILE 106e1
  - VERIFICATION RESULT FILE 106e2
  - SELECTED IGT STATE INFORMATION FILE 106e3
  - MULTIVARIATE DISCRIMINANT FILE 106e4
- DISCRIMINANT VALUE FILE (106f)
- EVALUATION RESULT FILE (106g)

**CONTROL DEVICE (102)**
- REQUEST-INTERPRETING PART (102a)
- BROWSING PROCESSING PART (102b)
- AUTHENTICATION-PROCESSING PART (102c)
- ELECTRONIC MAIL-GENERATING PART (102d)
- WEB PAGE-GENERATING PART (102e)
- RECEIVING PART (102f)
- IGT STATE INFORMATION-DESIGNATING PART (102g)
- MULTIVARIATE DISCRIMINANT-PREPARING PART (102h)
- DISCRIMINANT VALUE-CALCULATING PART (102i)
- DISCRIMINANT VALUE CRITERION-EVALUATING PART (102j)
- RESULT OUTPUTTING PART (102k)
- SENDING PART (102m)

COMMUNICATION INTERFACE (104)

NETWORK (300)

# FIG.7

106a

| USER ID | USER PASSWORD | NAME | ORGANI-ZATION ID | DEPART-MENT ID | DEPART-MENT NAME | E-MAIL ADDRESS | ... |
|---|---|---|---|---|---|---|---|
| : | : | : | : | : | : | : | : |

# FIG.8

106b

| INDIVIDUAL (SAMPLE) NO. | AMINO ACID CONCENTRATION DATA | | | | | |
|---|---|---|---|---|---|---|
| | Gly | Leu | Val | Ile | Phe | ... |
| U-1 | 9.5 | 11.2 | 2.7 | 8.5 | 4.9 | ... |
| U-2 | 8.5 | 10.5 | 3.9 | 9.8 | 6.1 | ... |
| : | : | : | : | : | : | : |

# FIG.9

EP 2 209 001 A1

| INDIVIDUAL (SAMPLE) NO. | IGT STATE INDEX DATA (T) | | | | AMINO ACID CONCENTRATION DATA | | | | | |
|---|---|---|---|---|---|---|---|---|---|---|
| | $T_1$ | $T_2$ | $T_3$ | ... | Gly | Leu | Val | Ile | Phe | ... |
| A-1 | 23.4 | 62.5 | 37.1 | ... | 9.5 | 11.2 | 2.7 | 8.5 | 4.9 | ... |
| A-2 | 27.5 | 66.1 | 39.5 | ... | 8.5 | 10.5 | 3.9 | 9.8 | 6.1 | ... |
| ⋮ | ⋮ | ⋮ | ⋮ | ⋮ | ⋮ | | | | | |

106c

# FIG.10

106d

| INDIVIDUAL (SAMPLE) NO. | IGT STATE INDEX DATA (T) | | AMINO ACID CONCENTRATION DATA | | | |
|---|---|---|---|---|---|---|
| | $T_2$ | | Gly | Leu | Phe | ⋯ |
| A-1 | 62.5 | | 9.5 | 11.2 | 4.9 | ⋯ |
| A-2 | 66.1 | | 8.5 | 10.5 | 6.1 | ⋯ |
| ⋮ | ⋮ | | ⋮ | | ⋮ | ⋮ |

# FIG.11

106e1

| RANK | CANDIDATE MULTIVARIATE DISCRIMINANT |
|---|---|
| 1 | $F_1(Gly, Leu, Phe, \cdots)$ |
| 2 | $F_2(Gly, Leu, Phe, \cdots)$ |
| 3 | $F_3(Gly, Leu, Phe, \cdots)$ |
| ⋮ | ⋮ |

# FIG.12

106e2

| RANK | CANDIDATE MULTIVARIATE DISCRIMINANT | VERIFICATION RESULT |
|---|---|---|
| 1 | $F_k$(Gly,Leu,Phe,⋯) | 1.22 |
| 2 | $F_m$(Gly,Leu,Phe,⋯) | 2.28 |
| 3 | $F_l$(Gly,Leu,Phe,⋯) | 2.95 |
| ⋮ | ⋮ | ⋮ |

# FIG.13

106e3

| INDIVIDUAL (SAMPLE) NO. | IGT STATE INDEX DATA (T) | AMINO ACID CONCENTRATION DATA | | |
|---|---|---|---|---|
| | $T_2$ | Leu | Phe | ⋯ |
| A-1 | 62.5 | 11.2 | 4.9 | ⋯ |
| A-2 | 66.1 | 10.5 | 6.1 | ⋯ |
| ⋮ | ⋮ | ⋮ | ⋮ | ⋮ |

# FIG.14

106e4

| RANK | MULTIVARIATE DISCRIMINANT | THRESHOLD VALUE | VERIFICATION RESULT |
|------|--------------------------|-----------------|---------------------|
| 1 | $F_p(\text{Phe},\cdots)$ | 0.23 | 0.62 |
| 2 | $F_p(\text{Gly},\text{Leu},\text{Phe})$ | -2.12 | 1.02 |
| 3 | $F_k(\text{Gly},\text{Leu},\text{Phe},\cdots)$ | 1.23 | 1.22 |
| ⋮ | ⋮ | ⋮ | ⋮ |

# FIG.15

106f

| INDIVIDUAL (SAMPLE) NO. | RANK | DISCRIMINANT VALUE |
|-------------------------|------|--------------------|
| U-1 | 1 | 1.13 |
| ⋮ | ⋮ | |

# FIG.16

106g

| INDIVIDUAL (SAMPLE) NO. | AMINO ACID CONCENTRATION DATA | | | | DISCRIMINANT VALUE | EVALUATION RESULT |
|---|---|---|---|---|---|---|
| | Gly | Leu | Phe | ... | | |
| U-1 | 9.5 | 11.2 | 4.9 | ... | | |
| U-2 | 8.5 | 10.5 | 6.1 | ... | | |
| ⋮ | ⋮ | ⋮ | ⋮ | ⋮ | | . |

# FIG.17

102h

MULTIVARIATE DISCRIMINANT-PREPARING PART

CANDIDATE MULTIVARIATE DISCRIMINANT-PREPARING PART   ~102h1

CANDIDATE MULTIVARIATE DISCRIMINANT-VERIFYING PART   ~102h2

EXPLANATORY VARIABLE-SELECTING PART   ~102h3

# FIG.18

~102j

DISCRIMINANT VALUE CRITERION-EVALUATING PART

DISCRIMINANT VALUE CRITERION-DISCRIMINATING PART ~102j1

# FIG.19

~200

CLIENT APPARATUS

~250
INPUT DEVICE

~270
INPUT/OUTPUT IF

~240
RAM

~220
ROM

~230
HD

~280
COMMUNICATION IF

~300
NETWORK

~260
OUTPUT DEVICE

261 — MONITOR

262 — PRINTER

~210
CONTROL DEVICE

WEB BROWSER ~211

ELECTRONIC MAILER ~212

RECEIVING PART ~213

SENDING PART ~214

# FIG.20

412
INPUT
DEVICE

414
OUTPUT
DEVICE

400
DATABASE APPARATUS

INPUT/OUTPUT INTERFACE ~ 408

406
MEMORY DEVICE

402
CONTROL DEVICE

REQUEST-
INTERPRETING PART ~ 402a

BROWSING
PROCESSING PART ~ 402b

AUTHENTICATION-
PROCESSING PART ~ 402c

ELECTRONIC MAIL-
GENERATING PART ~ 402d

WEB PAGE-
GENERATING PART ~ 402e

SENDING PART ~ 402f

404
COMMUNICATION INTERFACE

300
NETWORK

# FIG.21

(DATABASE APPARATUS 400)  (IGT-EVALUATING APPARATUS 100)  (CLIENT APPARATUS 200)

START                          START                          START

SA-22                          SA-23                          SA-21

TRANSMIT               RECEIVE MULTIVARIATE DISCRIMINANT      TRANSMIT AMINO
MULTIVARIATE      →    AND AMINO ACID CONCENTRATION DATA  ←       ACID
DISCRIMINANT                                                  CONCENTRATION
                                                                 DATA

                               SA-24

                          REMOVE DATA

                               SA-25

                       CALCULATE DISCRIMINANT VALUE

                               SA-26

                       DISCRIMINATE BETWEEN IGT AND NGT

                               SA-27

              ←       TRANSMIT EVALUATION RESULTS      →

SA-28                                                          SA-29

STORE EVALUATION                                              OUTPUT
    RESULTS                                                  EVALUATION
                                                              RESULTS

END                            END                            END

EP 2 209 001 A1

# FIG.22

START

PREPARE CANDIDATE MULTIVARIATE DISCRIMINANT — SB-21

VERIFY CANDIDATE MULTIVARIATE DISCRIMINANT — SB-22

SELECT EXPLANATORY VARIABLE OF CANDIDATE MULTIVARIATE DISCRIMINANT — SB-23

SB-24

NO — ALL COMBINATIONS WERE ENDED?

YES

SELECT CANDIDATE MULTIVARIATE DISCRIMINANT (DECIDE MULTIVARIATE DISCRIMINANT) — SB-25

END

# FIG.23

(BASIC PRINCIPLE OF THE INVENTION)

SUBSTANCE GROUP

INDIVIDUAL

INDIVIDUAL AFTER ADMINISTRATION

BLOOD SAMPLE OF INDIVIDUAL

**S-31** ADMINISTRATION OF SUBSTANCE GROUP

**S-32** COLLECTION OF BLOOD

**S-33** MEASUREMENT OF AMINO ACID CONCENTRATION DATA → AMINO ACID CONCENTRATION DATA OF INDIVIDUAL

**S-34** EVALUATION OF IGT STATE → EVALUATION RESULTS

**S-35** JUDGMENT OF SBSTANCE GROUP → JUDGMENT RESULTS

# FIG.24

```
        ┌─────────────┐
        │    START    │
        └──────┬──────┘
               ↓
   ┌───────────────────────┐
   │ ADMINISTER SUBSTANCE  │ ～SA-31
   │        GROUP          │
   └───────────┬───────────┘
               ↓
   ┌───────────────────────┐
   │     COLLECT BLOOD     │ ～SA-32
   └───────────┬───────────┘
               ↓
   ┌───────────────────────┐
   │  MEASURE AMINO ACID   │ ～SA-33
   │  CONCENTRATION DATA   │
   └───────────┬───────────┘
               ↓
   ┌───────────────────────┐
   │     REMOVE DATA       │ ～SA-34
   └───────────┬───────────┘
               ↓
   ┌───────────────────────┐
   │ DISCRIMINATE BETWEEN  │ ～SA-35
   │     IGT AND NGT       │
   └───────────┬───────────┘
               ↓
   ┌───────────────────────┐
   │ JUDGE SBSTANCE GROUP  │ ～SA-36
   └───────────┬───────────┘
               ↓
        ┌─────────────┐
        │     END     │
        └─────────────┘
```

# FIG.25

# FIG.26

# FIG.27

# FIG.28

| RANK | VALUE | FORMULA |
|------|-------|---------|
| 1 | 0.781 | (Glu)/(His+Cit)+(Phe+Tyr)/(Gly) |
| 2 | 0.779 | (Glu)/(His+Asn)+(Phe+Tyr)/(Gly) |
| 3 | 0.778 | (Glu)/(His+Tau)+(Phe+Tyr)/(Gly) |
| 4 | 0.777 | (Glu)/(His+Cit)+(Ile+Phe)/(Gly) |
| 5 | 0.777 | (Glu+Phe)/(His+Asn+Ser+Gly) |
| 6 | 0.776 | (Glu+Tyr)/(His+Cit+Asn+Gly) |
| 7 | 0.775 | (Tyr)/(Gly)+(Glu+ABA)/(His+Ser) |
| 8 | 0.775 | (Glu)/(His+Ser)+(Asp+Tyr)/(Gly) |
| 9 | 0.775 | (Glu+Phe+Met)/(His+Cit+Gly) |
| 10 | 0.775 | (Glu+Phe)/(His+Cit+Gly) |
| 11 | 0.774 | (Glu+Phe+Met)/(His+Asn+Gly) |
| 12 | 0.774 | (Glu+Asp+Phe)/(His+Cit+Gly) |
| 13 | 0.774 | (Glu+Asp+Phe)/(His+Ser+Gly) |
| 14 | 0.774 | (Tyr)/(Gly)+(Glu+Phe)/(His+Cit) |
| 15 | 0.774 | (Glu+Phe)/(His+Cit+Asn+Gly) |
| 16 | 0.774 | (Tyr)/(Gly)+(Glu+Asp)/(His+Ser) |
| 17 | 0.774 | (Glu)/(His+Ser)+(Tyr)/(Gly) |
| 18 | 0.773 | (Glu+Asp+Tyr)/(His+Tau+Gly) |
| 19 | 0.773 | (Glu)/(Cit+Ser)+(Tyr+Orn)/(Gly) |
| 20 | 0.773 | (Glu)/(His)+(Ile+Phe+Tyr)/(Gly) |
| 21 | 0.773 | (Glu)/(His)+(Phe+Tyr+ABA)/(Gly) |
| 22 | 0.773 | (Phe)/(His+Cit)+(Glu+Met)/(Gly) |
| 23 | 0.773 | (Glu)/(Tau+Ser)+(Tyr+ABA)/(Gly) |
| 24 | 0.772 | (Phe)/(His+Asn)+(Glu+Met)/(Gly) |
| 25 | 0.772 | (Glu+Tyr)/(His+Asn+Gly) |
| 26 | 0.772 | (Glu)/(His+Cit)+(Tyr+Met)/(Gly) |
| 27 | 0.772 | (Glu)/(His+Cit+Asn)+(Tyr)/(Gly) |
| 28 | 0.772 | (Glu)/(Cit+Ser)+(Phe+Orn)/(Gly) |
| 29 | 0.772 | (Glu+Asp+Tyr)/(Tau+Asn+Gly) |
| 30 | 0.772 | (Glu)/(His+Ser)+(Tyr)/(Cit+Gly) |
| 31 | 0.772 | (Glu)/(Gly)+(Phe)/(His+Cit+Ser) |
| 32 | 0.772 | (Phe)/(His+Ser)+(Glu+Met)/(Gly) |
| 33 | 0.772 | (Glu)/(Ser)+(Tyr+ABA+Orn)/(Gly) |
| 34 | 0.772 | (Glu+Asp+Phe)/(His+Asn+Gly) |
| 35 | 0.772 | (Phe)/(Gly)+(Glu+Met)/(His+Ser) |
| 36 | 0.772 | (Glu)/(Tau+Asn+Ser)+(Tyr)/(Gly) |
| 37 | 0.772 | (Glu)/(Gly)+(Phe)/(His+Cit+Asn) |
| 38 | 0.772 | (Glu)/(His+Cit)+(Tyr+Orn)/(Gly) |
| 39 | 0.772 | (Glu)/(His+Tau+Cit)+(Tyr)/(Gly) |
| 40 | 0.772 | (Glu+Asp+Phe)/(His+Gly) |
| 41 | 0.772 | (Glu+Phe)/(His+Gly) |
| 42 | 0.771 | (Glu)/(His+Ser)+(Tyr)/(Tau+Gly) |
| 43 | 0.771 | (Glu+Asp+Tyr)/(His+Asn+Gly) |
| 44 | 0.771 | (Glu)/(His+Tau)+(Tyr+ABA)/(Gly) |
| 45 | 0.771 | (Glu+Tyr)/(His+Tau+Asn+Gly) |
| 46 | 0.771 | (Glu+Phe)/(His+Asn+Gly) |
| 47 | 0.771 | (Glu)/(Gly)+(Phe)/(His+Tau+Cit) |
| 48 | 0.771 | (Glu)/(His+Ser)+(Phe)/(Gly) |
| 49 | 0.771 | (Glu)/(His)+(Phe+Tyr+Met)/(Gly) |
| 50 | 0.771 | (Glu)/(His+Asn)+(Ile+Tyr)/(Gly) |

# FIG.29

| 51 | 0.771 | (Asp)/(Gln)+(Glu+Phe)/(His+Gly) |
|---|---|---|
| 52 | 0.771 | (Glu)/(Cit+Asn+Ser)+(Tyr)/(Gly) |
| 53 | 0.771 | (Glu+Phe)/(His+Cit+Ser+Gly) |
| 54 | 0.771 | (Glu)/(Asn+Ser)+(Tyr+Met)/(Gly) |
| 55 | 0.771 | (Glu+Phe)/(His+Tau+Cit+Gly) |
| 56 | 0.770 | (Glu)/(His+Tau+Asn)+(Tyr)/(Gly) |
| 57 | 0.770 | (Glu)/(Gly)+(Phe)/(His+Cit) |
| 58 | 0.770 | (Glu)/(His+Ser)+(Asp+Phe)/(Gly) |
| 59 | 0.770 | (Glu)/(Asn+Ser)+(Tyr+ABA)/(Gly) |
| 60 | 0.770 | (Asp)/(Ser)+(Glu+Phe)/(His+Gly) |
| 61 | 0.770 | (Phe)/(Gly)+(Glu+Asp)/(His+Ser) |
| 62 | 0.770 | (Glu)/(His)+(Phe+Tyr+Orn)/(Gly) |
| 63 | 0.770 | (Glu)/(His+Cit)+(Phe+Orn)/(Gly) |
| 64 | 0.770 | (Met)/(Gln)+(Glu+Phe)/(His+Gly) |
| 65 | 0.770 | (Glu)/(His)+(Phe+Tyr)/(Gly) |
| 66 | 0.770 | (Glu+Phe)/(His+Ser+Gly) |
| 67 | 0.770 | (Glu)/(Ser)+(Phe+Tyr)/(Asn+Gly) |
| 68 | 0.770 | (Glu+Phe)/(His+Tau+Asn+Gly) |
| 69 | 0.770 | (Glu)/(Ser)+(Phe+Tyr)/(Cit+Gly) |
| 70 | 0.770 | (Glu)/(His)+(Asp+Phe+Tyr)/(Gly) |
| 71 | 0.769 | (Tyr)/(Gly)+(Glu+Phe)/(His+Asn) |
| 72 | 0.769 | (Phe)/(His+Ser)+(Glu+Asp)/(Gly) |
| 73 | 0.769 | (Glu)/(Ser)+(Asp+Tyr+Orn)/(Gly) |
| 74 | 0.769 | (Glu+Phe+Met)/(His+Tau+Gly) |
| 75 | 0.769 | (Glu)/(His+Cit)+(Phe+Met)/(Gly) |
| 76 | 0.769 | (Glu)/(Gly)+(Phe)/(His+Ser) |
| 77 | 0.769 | (Ile)/(Gly)+(Glu+Phe)/(His+Cit) |
| 78 | 0.769 | (Glu)/(His)+(Ile+Tyr+Met)/(Gly) |
| 79 | 0.769 | (Phe)/(His+Cit)+(Glu+Orn)/(Gly) |
| 80 | 0.769 | (Glu)/(Tau+Ser)+(Tyr+Orn)/(Gly) |
| 81 | 0.769 | (Glu)/(His+Asn)+(Ile+Phe)/(Gly) |
| 82 | 0.769 | (Glu)/(His+Tau)+(Tyr+Met)/(Gly) |
| 83 | 0.769 | (Glu)/(Ser)+(Ile+Tyr)/(Cit+Gly) |
| 84 | 0.769 | (Glu+Asp)/(His)+(Phe+Tyr)/(Gly) |
| 85 | 0.769 | (Glu+Phe)/(His+Tau+Ser+Gly) |
| 86 | 0.768 | (Glu)/(His+Ser)+(Tyr)/(Asn+Gly) |
| 87 | 0.768 | (Glu)/(Cit+Gly)+(Phe)/(His+Ser) |
| 88 | 0.768 | (Glu)/(Gly)+(Phe)/(His+Asn+Ser) |
| 89 | 0.768 | (Glu)/(Ser)+(Tyr+Orn+Met)/(Gly) |
| 90 | 0.768 | (Glu)/(His+Asn)+(Tyr+Met)/(Gly) |
| 91 | 0.768 | (Glu)/(Asn+Ser)+(Tyr+Orn)/(Gly) |
| 92 | 0.768 | (Tyr)/(Gly)+(Glu+Asp)/(Tau+Ser) |
| 93 | 0.768 | (Glu)/(Ser)+(Tyr+Orn)/(Gly) |
| 94 | 0.768 | (Phe)/(His+Cit)+(Glu+Asp)/(Gly) |
| 95 | 0.768 | (Glu+Asp+Phe)/(His+Tau+Gly) |
| 96 | 0.768 | (Glu)/(Ser)+(Phe+ABA+Orn)/(Gly) |
| 97 | 0.768 | (Glu)/(Gly)+(Asp+Phe)/(His+Cit) |
| 98 | 0.768 | (Glu)/(Asn+Ser)+(Tyr)/(Gly) |
| 99 | 0.768 | (Glu)/(Ser)+(Phe+Tyr)/(Tau+Gly) |
| 100 | 0.768 | (Glu)/(Asn+Ser)+(Phe+Met)/(Gly) |

# FIG.30

| RANK | VALUE | FORMULA |
|---|---|---|
| 1 | 0.754 | Glu,Gly,Phe |
| 2 | 0.753 | Glu,Gly,Cit,Phe |
| 3 | 0.753 | Glu,Gly,Tyr |
| 4 | 0.753 | Glu,Gly,Arg |
| 5 | 0.752 | Glu,Gly,Cit,Phe,Arg |
| 6 | 0.751 | Glu,Gly,Phe,Arg |
| 7 | 0.751 | Glu,Gly,Cit,Tyr |
| 8 | 0.751 | Glu,Gly,Cit,Orn,Arg |
| 9 | 0.751 | Glu,Gly,Met |
| 10 | 0.751 | Glu,Gly,Cit,Phe,Orn,Arg |
| 11 | 0.751 | Glu,Gly,Cit,Phe,Orn |
| 12 | 0.751 | Glu,Gly,Phe,Trp |
| 13 | 0.750 | Glu,Gly,Tyr,Trp |
| 14 | 0.750 | Glu,Gly,Met,Phe |
| 15 | 0.750 | Glu,Gly,Ile |
| 16 | 0.749 | Glu,Gly,Cit,Phe,His,Arg |
| 17 | 0.749 | Glu,Gly,Ile,Arg |
| 18 | 0.749 | Glu,Gly,Met,Arg |
| 19 | 0.749 | Glu,Gly,Phe,Trp,Arg |
| 20 | 0.749 | Glu,Gly,Cit,Orn |
| 21 | 0.749 | Glu,Gly,Phe,His |
| 22 | 0.749 | Glu,Gln,Gly,Arg |
| 23 | 0.749 | Asn,Glu,Gly,Tyr |
| 24 | 0.749 | Glu,Gly,Phe,His,Arg |
| 25 | 0.749 | Glu,Gly,Trp,Arg |
| 26 | 0.749 | Glu,Gly,Cit,Met,Phe |
| 27 | 0.749 | Glu,Gly,Tyr,Arg |
| 28 | 0.749 | Glu,Gly,Orn |
| 29 | 0.749 | Glu,Gly,Cit,Met,Phe,Arg |
| 30 | 0.749 | Glu,Gly,Cit,Arg |
| 31 | 0.749 | Glu,Gly,Met,Phe,Arg |
| 32 | 0.749 | Glu,Gly,Cit |
| 33 | 0.748 | Glu,Gln,Gly,Cit,Orn,Arg |
| 34 | 0.748 | Thr,Glu,Gly,Cit,Phe |
| 35 | 0.748 | Glu,Gly,Cit,Phe,Trp |
| 36 | 0.748 | Glu,Gly,Phe,Orn |
| 37 | 0.748 | Glu,Gln,Gly,Cit,Phe |
| 38 | 0.748 | Glu,Gln,Gly,Tyr |
| 39 | 0.748 | Glu,Gly,Cit,Tyr,Orn |
| 40 | 0.748 | Ser,Glu,Gly,Phe |
| 41 | 0.748 | Glu,Gly,Tyr,His |
| 42 | 0.748 | Glu,Gln,Gly,Phe |
| 43 | 0.748 | Glu,Gly,Cit,Phe,Trp,Orn |
| 44 | 0.748 | Glu,Gly,Cit,Phe,His,Orn |
| 45 | 0.748 | Glu,Gly,Cit,Phe,Trp,Arg |
| 46 | 0.748 | Glu,Gly,Cit,Tyr,Phe |
| 47 | 0.748 | Glu,Gly,Tyr,Trp,Arg |
| 48 | 0.748 | Glu,Gly,Cit,Tyr,Trp |
| 49 | 0.748 | Glu,Gly,Cit,Met |
| 50 | 0.748 | Glu,Gln,Gly,Cit,Phe,Arg |

# FIG.31

| 51 | 0.748 | Glu,Gly,Val,Tyr |
|---|---|---|
| 52 | 0.748 | Glu,Gly,Ile,Tyr |
| 53 | 0.747 | Glu,Gly,Trp |
| 54 | 0.747 | Glu,Gly,Tyr,Phe |
| 55 | 0.747 | Glu,Gly,Ile,Trp |
| 56 | 0.747 | Glu,Gly,Cit,Met,Arg |
| 57 | 0.747 | Ser,Glu,Gly,Cit,Phe,Arg |
| 58 | 0.747 | Glu,Gln,Gly |
| 59 | 0.747 | Glu,Gly,Val,Arg |
| 60 | 0.747 | Glu,Gly,Cit,Tyr,Arg |
| 61 | 0.747 | Glu,Gly,Ile,Leu,Phe |
| 62 | 0.747 | Glu,Gly,Cit,Ile,Arg |
| 63 | 0.747 | Thr,Glu,Gly,Phe |
| 64 | 0.747 | Glu,Gly,Ile,Phe |
| 65 | 0.747 | Glu,Gly,Cit,Ile,Phe |
| 66 | 0.747 | Glu,Gly,Cit,Phe,His |
| 67 | 0.747 | Glu,Gly,Ile,Tyr,Trp |
| 68 | 0.747 | Glu,Gly,Cit,Ile,Leu,Phe |
| 69 | 0.747 | Glu,Gly,Met,Phe,His |
| 70 | 0.747 | Ser,Glu,Gly,Cit,Phe |
| 71 | 0.747 | Glu,Gly,Lys,Arg |
| 72 | 0.747 | Glu,Gln,Gly,Phe,Arg |
| 73 | 0.746 | Glu,Gly,Leu,Tyr |
| 74 | 0.746 | Glu,Gly,Trp,Orn |
| 75 | 0.746 | Glu,Gln,Gly,Phe,His |
| 76 | 0.746 | Glu,Gln,Gly,Cit,Met,Phe |
| 77 | 0.746 | Glu,Gly,Cit,Ile |
| 78 | 0.746 | Ser,Glu,Gly,Arg |
| 79 | 0.746 | Glu,Gly,Met,Phe,Trp |
| 80 | 0.746 | Glu,Gly,Cit,Trp,Orn,Arg |
| 81 | 0.746 | Glu,Gly,Tyr,Phe,His |
| 82 | 0.746 | Glu,Gly,Cit,Tyr,His |
| 83 | 0.746 | Glu,Gly,Phe,His,Orn |
| 84 | 0.746 | Ser,Glu,Gly,Phe,Arg |
| 85 | 0.746 | Glu,Gly,Ile,Trp,Arg |
| 86 | 0.746 | Glu,Gly,Met,Orn |
| 87 | 0.746 | Glu,Gly,Cit,Met,Orn |
| 88 | 0.746 | Glu,Gly,Orn,Arg |
| 89 | 0.746 | Asn,Glu,Gly,Cit,Phe,Orn |
| 90 | 0.746 | Glu,Gly,Cit,Tyr,Trp,Orn |
| 91 | 0.746 | Glu,Gly,Ala,Phe |
| 92 | 0.746 | Thr,Glu,Gly,Tyr |
| 93 | 0.746 | Glu,Gly,Cit,Met,Orn,Arg |
| 94 | 0.746 | Glu,Gly,Lys |
| 95 | 0.746 | Glu,Gly,Cit,Tyr,Orn,Arg |
| 96 | 0.746 | Glu,Gln,Gly,Ile |
| 97 | 0.746 | Glu,Gly,Tyr,Lys |
| 98 | 0.746 | Glu,Gly,Ile,Phe,Trp |
| 99 | 0.746 | Glu,Gly,Cit,Ile,Phe,Arg |
| 100 | 0.746 | Glu,Gln,Gly,Met,Phe |

# FIG.32

| RANK | VALUE | FORMULA |
|------|-------|---------|
| 1 | 0.754 | [-2.0752]+[0.0384]Glu+[-0.0126]Gly+[0.02]Phe |
| 2 | 0.753 | [-2.0534]+[0.0385]Glu+[-0.0119]Gly+[-0.0112]Cit+[0.0232]Phe |
| 3 | 0.753 | [-1.4057]+[0.0375]Glu+[-0.0133]Gly+[0.0112]Tyr |
| 4 | 0.753 | [-1.1681]+[0.0389]Glu+[-0.0139]Gly+[0.0065]Arg |
| 5 | 0.752 | [-2.1534]+[0.0384]Glu+[-0.0125]Gly+[-0.0143]Cit+[0.0212]Phe+[0.005]Arg |
| 6 | 0.751 | [-2.1491]+[0.0384]Glu+[-0.0131]Gly+[0.018]Phe+[0.0034]Arg |
| 7 | 0.751 | [-1.3461]+[0.0374]Glu+[-0.0129]Gly+[-0.0072]Cit+[0.0128]Tyr |
| 8 | 0.751 | [-1.3979]+[0.0368]Glu+[-0.0159]Gly+[-0.0255]Cit+[0.0244]Orn+[0.0071]Arg |
| 9 | 0.751 | [-1.3903]+[0.0386]Glu+[-0.0137]Gly+[0.0288]Met |
| 10 | 0.751 | [-2.3146]+[0.0366]Glu+[-0.0146]Gly+[-0.0311]Cit+[0.0186]Phe+[0.0229]Orn+[0.0049]Arg |
| 11 | 0.751 | [-2.2164]+[0.0366]Glu+[-0.014]Gly+[-0.028]Cit+[0.0206]Phe+[0.0228]Orn |
| 12 | 0.751 | [-1.8458]+[0.0388]Glu+[-0.0125]Gly+[0.0238]Phe+[-0.0097]Trp |
| 13 | 0.750 | [-1.1875]+[0.0376]Glu+[-0.0134]Gly+[0.0137]Tyr+[-0.0073]Trp |
| 14 | 0.750 | [-2.1102]+[0.0384]Glu+[-0.0127]Gly+[0.0058]Met+[0.0187]Phe |
| 15 | 0.750 | [-1.23]+[0.0367]Glu+[-0.0131]Gly+[0.0092]Ile |
| 16 | 0.749 | [-0.8922]+[0.0386]Glu+[-0.0123]Gly+[-0.0159]Cit+[0.0293]Phe+[-0.0236]His+[0.0074]Arg |
| 17 | 0.749 | [-1.4138]+[0.0371]Glu+[-0.0138]Gly+[0.0071]Ile+[0.0049]Arg |
| 18 | 0.749 | [-1.4776]+[0.0386]Glu+[-0.0141]Gly+[0.0209]Met+[0.0043]Arg |
| 19 | 0.749 | [-1.905]+[0.0389]Glu+[-0.0132]Gly+[0.0218]Phe+[-0.0114]Trp+[0.0046]Arg |
| 20 | 0.749 | [-1.1021]+[0.0369]Glu+[-0.0151]Gly+[-0.0201]Cit+[0.0245]Orn |
| 21 | 0.749 | [-0.9036]+[0.0385]Glu+[-0.0122]Gly+[0.0281]Phe+[-0.021]His |
| 22 | 0.749 | [-1.0288]+[0.0389]Glu+[-5e-04]Gln+[-0.0136]Gly+[0.0073]Arg |
| 23 | 0.749 | [-0.6282]+[-0.0361]Asn+[0.038]Glu+[-0.0121]Gly+[0.019]Tyr |
| 24 | 0.749 | [-0.9132]+[0.0385]Glu+[-0.013]Gly+[0.0257]Phe+[-0.023]His+[0.0055]Arg |
| 25 | 0.749 | [-1.0025]+[0.0391]Glu+[-0.014]Gly+[-0.0043]Trp+[0.0072]Arg |
| 26 | 0.749 | [-2.1245]+[0.0384]Glu+[-0.0121]Gly+[-0.0125]Cit+[0.0117]Met+[0.0209]Phe |
| 27 | 0.749 | [-1.5032]+[0.0378]Glu+[-0.0138]Gly+[0.0087]Tyr+[0.004]Arg |
| 28 | 0.749 | [-1.3283]+[0.0372]Glu+[-0.0155]Gly+[0.0185]Orn |
| 29 | 0.749 | [-2.1783]+[0.0384]Glu+[-0.0125]Gly+[-0.0147]Cit+[0.0051]Met+[0.0203]Phe+[0.0046]Arg |
| 30 | 0.749 | [-1.0918]+[0.0389]Glu+[-0.0137]Gly+[-0.0065]Cit+[0.0074]Arg |
| 31 | 0.749 | [-2.1505]+[0.0384]Glu+[-0.0131]Gly+[3e-04]Met+[0.018]Phe+[0.0034]Arg |
| 32 | 0.749 | [-0.7748]+[0.039]Glu+[-0.013]Gly+[-6e-04]Cit |
| 33 | 0.748 | [-1.0051]+[0.0367]Glu+[-0.0015]Gln+[-0.0152]Gly+[-0.0273]Cit+[0.0267]Orn+[0.0094]Arg |
| 34 | 0.748 | [-2.2341]+[0.0039]Thr+[0.0377]Glu+[-0.0126]Gly+[-0.0132]Cit+[0.023]Phe |
| 35 | 0.748 | [-1.8461]+[0.0388]Glu+[-0.0119]Gly+[-0.0099]Cit+[0.0262]Phe+[-0.0088]Trp |
| 36 | 0.748 | [-2.2167]+[0.0371]Glu+[-0.0147]Gly+[0.0151]Phe+[0.0155]Orn |
| 37 | 0.748 | [-2.0357]+[0.0385]Glu+[-1e-04]Gln+[-0.0118]Gly+[-0.0111]Cit+[0.0232]Phe |
| 38 | 0.748 | [-1.3168]+[0.0375]Glu+[-3e-04]Gln+[-0.0131]Gly+[0.0118]Tyr |
| 39 | 0.748 | [-1.4133]+[0.0361]Glu+[-0.015]Gly+[-0.0222]Cit+[0.0075]Tyr+[0.0225]Orn |
| 40 | 0.748 | [-1.5742]+[-0.0087]Ser+[0.0387]Glu+[-0.0108]Gly+[0.0202]Phe |
| 41 | 0.748 | [-0.28]+[0.0371]Glu+[-0.0133]Gly+[0.0167]Tyr+[-0.0178]His |
| 42 | 0.748 | [-2.0268]+[0.0384]Glu+[-2e-04]Gln+[-0.0124]Gly+[0.0202]Phe |
| 43 | 0.748 | [-1.928]+[0.0369]Glu+[-0.0142]Gly+[-0.0273]Cit+[0.0247]Phe+[-0.0123]Trp+[0.0241]Orn |
| 44 | 0.748 | [-0.851]+[0.0366]Glu+[-0.0138]Gly+[-0.0301]Cit+[0.0301]Phe+[-0.0254]His+[0.0256]Orn |
| 45 | 0.748 | [-1.9214]+[0.0388]Glu+[-0.0126]Gly+[-0.0135]Cit+[0.0245]Phe+[-0.0107]Trp+[0.006]Arg |
| 46 | 0.748 | [-2.112]+[0.0379]Glu+[-0.012]Gly+[-0.0124]Cit+[0.0049]Tyr+[0.0203]Phe |
| 47 | 0.748 | [-1.2664]+[0.0379]Glu+[-0.014]Gly+[0.0111]Tyr+[-0.0085]Trp+[0.0047]Arg |
| 48 | 0.748 | [-1.1517]+[0.0375]Glu+[-0.013]Gly+[-0.0063]Cit+[0.0149]Tyr+[-0.0068]Trp |
| 49 | 0.748 | [-1.3474]+[0.0386]Glu+[-0.0133]Gly+[-0.0075]Cit+[0.0339]Met |
| 50 | 0.748 | [-1.9955]+[0.0385]Glu+[-6e-04]Gln+[-0.0121]Gly+[-0.0144]Cit+[0.0213]Phe+[0.0059]Arg |

# FIG.33

| 51 | 0.748 | [-1.3728]+[0.0377]Glu+[-0.0133]Gly+[-3e-04]Val+[0.0116]Tyr |
|---|---|---|
| 52 | 0.748 | [-1.5009]+[0.0366]Glu+[-0.0133]Gly+[0.0055]Ile+[0.0082]Tyr |
| 53 | 0.747 | [-0.7636]+[0.0391]Glu+[-0.013]Gly+[-5e-04]Trp |
| 54 | 0.747 | [-2.1139]+[0.0381]Glu+[-0.0127]Gly+[0.0032]Tyr+[0.0179]Phe |
| 55 | 0.747 | [-0.9887]+[0.0365]Glu+[-0.0131]Gly+[0.0117]Ile+[-0.0075]Trp |
| 56 | 0.747 | [-1.4369]+[0.0386]Glu+[-0.0138]Gly+[-0.01]Cit+[0.026]Met+[0.0053]Arg |
| 57 | 0.747 | [-1.6525]+[-0.0091]Ser+[0.0387]Glu+[-0.0109]Gly+[-0.0132]Cit+[0.0204]Phe+[0.0061]Arg |
| 58 | 0.747 | [-0.8847]+[0.039]Glu+[3e-04]Gln+[-0.0132]Gly |
| 59 | 0.747 | [-1.3234]+[0.0382]Glu+[-0.0139]Gly+[9e-04]Val+[0.0061]Arg |
| 60 | 0.747 | [-1.4457]+[0.0377]Glu+[-0.0134]Gly+[-0.0099]Cit+[0.0102]Tyr+[0.0051]Arg |
| 61 | 0.747 | [-1.5504]+[0.0402]Glu+[-0.0134]Gly+[0.034]Ile+[-0.0248]Leu+[0.0283]Phe |
| 62 | 0.747 | [-1.3603]+[0.0366]Glu+[-0.0133]Gly+[-0.0124]Cit+[0.0097]Ile+[0.0061]Arg |
| 63 | 0.747 | [-2.2199]+[0.0031]Thr+[0.0378]Glu+[-0.0132]Gly+[0.0194]Phe |
| 64 | 0.747 | [-2.1173]+[0.0376]Glu+[-0.0127]Gly+[0.0036]Ile+[0.018]Phe |
| 65 | 0.747 | [-2.1297]+[0.037]Glu+[-0.0118]Gly+[-0.0148]Cit+[0.0066]Ile+[0.0206]Phe |
| 66 | 0.747 | [-0.8887]+[0.0386]Glu+[-0.0115]Gly+[-0.0112]Cit+[0.0313]Phe+[-0.021]His |
| 67 | 0.747 | [-1.2326]+[0.0362]Glu+[-0.0134]Gly+[0.008]Ile+[0.0103]Tyr+[-0.0105]Trp |
| 68 | 0.747 | [-1.534]+[0.0397]Glu+[-0.0122]Gly+[-0.0213]Cit+[0.0411]Ile+[-0.0271]Leu+[0.0331]Phe |
| 69 | 0.747 | [-0.8348]+[0.0383]Glu+[-0.0129]Gly+[0.0268]Met+[0.0232]Phe+[-0.0251]His |
| 70 | 0.747 | [-1.577]+[-0.0082]Ser+[0.0387]Glu+[-0.0103]Gly+[-0.0095]Cit+[0.0229]Phe |
| 71 | 0.747 | [-1.04]+[0.0391]Glu+[-0.0139]Gly+[-0.0011]Lys+[0.0074]Arg |
| 72 | 0.747 | [-1.9971]+[0.0384]Glu+[-6e-04]Gln+[-0.0128]Gly+[0.0181]Phe+[0.0043]Arg |
| 73 | 0.746 | [-1.1913]+[0.0386]Glu+[-0.0135]Gly+[-0.0035]Leu+[0.0143]Tyr |
| 74 | 0.746 | [-1.0629]+[0.0374]Glu+[-0.0156]Gly+[-0.0061]Trp+[0.0196]Orn |
| 75 | 0.746 | [-1.0197]+[0.0385]Glu+[7e-04]Gln+[-0.0127]Gly+[0.028]Phe+[-0.0227]His |
| 76 | 0.746 | [-2.0666]+[0.0384]Glu+[-2e-04]Gln+[-0.012]Gly+[-0.0125]Cit+[0.0128]Met+[0.0208]Phe |
| 77 | 0.746 | [-1.1625]+[0.0363]Glu+[-0.0126]Gly+[-0.0088]Cit+[0.0114]Ile |
| 78 | 0.746 | [-0.6594]+[-0.0099]Ser+[0.0391]Glu+[-0.0121]Gly+[0.0077]Arg |
| 79 | 0.746 | [-1.891]+[0.0387]Glu+[-0.0129]Gly+[0.0135]Met+[0.0212]Phe+[-0.0111]Trp |
| 80 | 0.746 | [-1.1131]+[0.037]Glu+[-0.0162]Gly+[-0.025]Cit+[-0.0079]Trp+[0.0255]Orn+[0.0082]Arg |
| 81 | 0.746 | [-0.8872]+[0.0377]Glu+[-0.0125]Gly+[0.0076]Tyr+[0.0237]Phe+[-0.023]His |
| 82 | 0.746 | [-0.2232]+[0.037]Glu+[-0.0129]Gly+[-0.0074]Cit+[0.0183]Tyr+[-0.0178]His |
| 83 | 0.746 | [-0.9035]+[0.037]Glu+[-0.0146]Gly+[0.0238]Phe+[-0.0241]His+[0.0175]Orn |
| 84 | 0.746 | [-1.6339]+[-0.0095]Ser+[0.0387]Glu+[-0.0114]Gly+[0.0175]Phe+[0.0047]Arg |
| 85 | 0.746 | [-1.1401]+[0.0369]Glu+[-0.0139]Gly+[0.01]Ile+[-0.0096]Trp+[0.0058]Arg |
| 86 | 0.746 | [-1.5328]+[0.0372]Glu+[-0.0156]Gly+[0.0118]Met+[0.0171]Orn |
| 87 | 0.746 | [-1.4281]+[0.0368]Glu+[-0.0153]Gly+[-0.0226]Cit+[0.0206]Met+[0.0228]Orn |
| 88 | 0.746 | [-1.5495]+[0.0372]Glu+[-0.0159]Gly+[0.0174]Orn+[0.0043]Arg |
| 89 | 0.746 | [-1.3835]+[-0.0357]Asn+[0.0377]Glu+[-0.0131]Gly+[-0.0265]Cit+[0.0238]Phe+[0.0263]Orn |
| 90 | 0.746 | [-1.1674]+[0.0361]Glu+[-0.0151]Gly+[-0.0214]Cit+[0.0101]Tyr+[-0.0086]Trp+[0.023]Orn |
| 91 | 0.746 | [-2.2169]+[0.0378]Glu+[-0.0129]Gly+[8e-04]Ala+[0.0195]Phe |
| 92 | 0.746 | [-1.4353]+[0.0016]Thr+[0.0373]Glu+[-0.0136]Gly+[0.01]Tyr |
| 93 | 0.746 | [-1.537]+[0.0367]Glu+[-0.0158]Gly+[-0.0262]Cit+[0.0111]Met+[0.0235]Orn+[0.0062]Arg |
| 94 | 0.746 | [-0.9936]+[0.0388]Glu+[-0.0131]Gly+[0.0012]Lys |
| 95 | 0.746 | [-1.531]+[0.0364]Glu+[-0.0157]Gly+[-0.0259]Cit+[0.0042]Tyr+[0.0233]Orn+[0.0061]Arg |
| 96 | 0.746 | [-1.2109]+[0.0367]Glu+[-1e-04]Gln+[-0.013]Gly+[0.0093]Ile |
| 97 | 0.746 | [-1.2624]+[0.0376]Glu+[-0.0132]Gly+[0.0129]Tyr+[-0.0014]Lys |
| 98 | 0.746 | [-1.8523]+[0.0373]Glu+[-0.0126]Gly+[0.0069]Ile+[0.0211]Phe+[-0.0128]Trp |
| 99 | 0.746 | [-2.2067]+[0.0372]Glu+[-0.0123]Gly+[-0.017]Cit+[0.0056]Ile+[0.0192]Phe+[0.0044]Arg |
| 100 | 0.746 | [-2.0418]+[0.0384]Glu+[-2e-04]Gln+[-0.0126]Gly+[0.007]Met+[0.0187]Phe |

# FIG.34

```
┌─────────────────────────────────────────┐
│  FPG ≧ 100 mg/dL  OR   HbA1c ≧ 5.2 %     │
└─────────────────────────────────────────┘
```

FPG ≧ 100 mg/dL  OR   HbA1c ≧ 5.2 %

FPGHb=0
NGT

FPGHb=1

AMINO INDEX

AI=0
NGT

AI=1
IGT/DM

# FIG.35

| RANK | VALUE | FORMULA |
|------|-------|---------|
| 1 | 0.767 | (Thr)/(Gly)+(Phe+Orn)/(Met+Asn) |
| 2 | 0.757 | (Phe)/(Asn)+(Thr+Leu)/(Trp+Tyr) |
| 3 | 0.754 | (Phe)/(Asn)+(Thr+Ile)/(Trp+Tyr) |
| 4 | 0.750 | (Thr)/(Trp)+(Phe+Orn+Leu)/(Asn) |
| 5 | 0.748 | (Phe+Leu)/(Asn)+(Orn+Thr)/(Trp) |
| 6 | 0.748 | (Phe)/(Met+Asn)+(Orn+Thr)/(Lys) |
| 7 | 0.748 | (Phe+Orn)/(Asn)+(Thr+Arg)/(Gly) |
| 8 | 0.747 | (Phe+Orn)/(Asn)+(Thr+Glu)/(Gly) |
| 9 | 0.747 | (Phe+Orn)/(Asn)+(Thr+His)/(Gly) |
| 10 | 0.746 | (Phe+Thr)/(Trp)+(Orn+Leu)/(Asn) |
| 11 | 0.746 | (Thr)/(Lys)+(Phe+Orn)/(Met+Asn) |
| 12 | 0.746 | (Phe)/(Trp)+(Thr+Leu)/(Met+Asn) |
| 13 | 0.745 | (Phe)/(Met+Asn)+(Thr+His)/(Gly) |
| 14 | 0.745 | (Phe)/(Met+Asn)+(Orn+Thr)/(Gly) |
| 15 | 0.745 | (Thr)/(Gly)+(Phe+Cit)/(Met+Asn) |
| 16 | 0.745 | (Orn)/(Met)+(Phe+Thr+Leu)/(Asn) |
| 17 | 0.744 | (Phe+Orn)/(Asn)+(Thr+Glu)/(Lys) |
| 18 | 0.744 | (Phe)/(Asn)+(Orn+Thr+Glu)/(Gly) |
| 19 | 0.744 | (Phe+Orn)/(Asn)+(Thr+Ile)/(Gly) |
| 20 | 0.744 | (Phe+Cit)/(Asn)+(Thr+Glu)/(Gly) |
| 21 | 0.744 | (Phe)/(Asn)+(Orn+Thr)/(Trp+Tyr) |
| 22 | 0.744 | (Phe+Leu)/(Asn)+(Thr+Glu)/(Trp) |
| 23 | 0.744 | (Orn)/(Trp)+(Thr+Leu)/(Met+Asn) |
| 24 | 0.743 | (Phe)/(Asn)+(Thr+Glu)/(Trp+Tyr) |
| 25 | 0.743 | (Phe)/(Asn)+(Orn+Thr)/(Pro+Trp) |
| 26 | 0.743 | (Val)/(Gly)+(Phe+Orn+Thr)/(Asn) |
| 27 | 0.743 | (Phe)/(Gly)+(Orn+Arg)/(Lys+Asn) |
| 28 | 0.742 | (Phe)/(Asn)+(Thr+Tau)/(Trp+Tyr) |
| 29 | 0.742 | (Thr)/(Trp)+(Phe+Leu+Cit)/(Asn) |
| 30 | 0.742 | (Phe+Cit)/(Asn)+(Thr+His)/(Gly) |
| 31 | 0.741 | (Phe)/(Asn)+(Orn+Thr+Tau)/(Gly) |
| 32 | 0.741 | (Thr)/(Gly)+(Orn+Ile)/(Met+Asn) |
| 33 | 0.741 | (Val)/(Lys)+(Phe+Orn+Thr)/(Asn) |
| 34 | 0.741 | (Phe)/(Asn)+(Orn+Thr+His)/(Gly) |
| 35 | 0.741 | (Leu)/(Gly)+(Phe+Thr)/(Met+Asn) |
| 36 | 0.741 | (Phe+Thr)/(Asn)+(Orn+Val)/(Lys) |
| 37 | 0.741 | (Phe)/(Tyr+Asn)+(Thr)/(Lys+Gly) |
| 38 | 0.741 | (Phe+Orn)/(Trp)+(Thr+Leu)/(Asn) |
| 39 | 0.741 | (Phe)/(Asn)+(Orn+Thr)/(Met+Lys) |
| 40 | 0.740 | (Phe+Orn)/(Asn)+(Thr+His)/(Lys) |
| 41 | 0.740 | (Phe)/(Asn)+(Orn+Thr+Cit)/(Gly) |
| 42 | 0.740 | (Phe)/(Asn)+(Thr+Leu)/(Met+Trp) |
| 43 | 0.740 | (Phe)/(Asn)+(Thr+Leu)/(Pro+Trp) |
| 44 | 0.740 | (Phe+Orn)/(Asn)+(Thr+Cit)/(Gly) |
| 45 | 0.740 | (Phe+Thr)/(Asn)+(Val+Leu)/(Lys) |
| 46 | 0.740 | (Phe+Orn+Thr+Leu)/(Met+Asn) |
| 47 | 0.740 | (Phe)/(Met+Asn)+(Thr+Tau)/(Gly) |
| 48 | 0.740 | (Phe)/(Asn)+(Orn+Thr)/(Trp+Gly) |
| 49 | 0.740 | (Phe+Orn)/(Asn)+(Thr+Leu)/(Lys) |
| 50 | 0.740 | (Thr)/(Trp)+(Phe+Ile+Leu)/(Asn) |

# FIG.36

| 51 | 0.740 | (Phe)/(Met+Asn)+(Thr)/(Trp+Gly) |
|---|---|---|
| 52 | 0.740 | (Phe)/(Tyr+Asn)+(Thr)/(Trp+Lys) |
| 53 | 0.740 | (Phe)/(Gly)+(Thr+Arg)/(Lys+Asn) |
| 54 | 0.739 | (Phe)/(Met+Asn)+(Thr+Cit)/(Gly) |
| 55 | 0.739 | (Phe+Orn)/(Asn)+(Thr+Tau)/(Gly) |
| 56 | 0.739 | (Orn+Leu)/(Asn)+(Thr+Glu)/(Trp) |
| 57 | 0.739 | (Phe)/(Asn)+(Thr+Tau)/(Gly) |
| 58 | 0.739 | (Phe)/(Asn)+(Thr+Glu+Tau)/(Gly) |
| 59 | 0.739 | (Phe)/(Trp)+(Thr+Leu)/(Tyr+Asn) |
| 60 | 0.739 | (Phe+Orn)/(Asn)+(Thr+Leu)/(Gly) |
| 61 | 0.739 | (Phe+Thr)/(Asn)+(Leu+His)/(Lys) |
| 62 | 0.739 | (Phe+Leu)/(Asn)+(Thr+Cit)/(Trp) |
| 63 | 0.739 | (Phe+Thr)/(Asn)+(Orn+Leu)/(Lys) |
| 64 | 0.739 | (Ile)/(Gly)+(Phe+Orn)/(Trp+Asn) |
| 65 | 0.739 | (Phe+Thr)/(Asn)+(Val+His)/(Gly) |
| 66 | 0.739 | (Orn)/(Met)+(Phe+Thr+Ile)/(Asn) |
| 67 | 0.739 | (Thr)/(Trp)+(Phe+Leu)/(Asn) |
| 68 | 0.739 | (Phe+Orn)/(Lys)+(Thr+Glu)/(Gly) |
| 69 | 0.739 | (Phe)/(Asn)+(Thr+Tau+Cit)/(Gly) |
| 70 | 0.739 | (Thr)/(Lys)+(Orn+Ile)/(Met+Asn) |
| 71 | 0.739 | (Thr)/(Gly)+(Phe+Orn)/(Asn) |
| 72 | 0.738 | (Phe)/(Asn)+(Orn+Thr)/(Trp+Lys) |
| 73 | 0.738 | (Phe+Orn)/(Asn)+(Thr+Ile)/(Lys) |
| 74 | 0.738 | (Phe)/(Met)+(Orn+Thr+Leu)/(Asn) |
| 75 | 0.738 | (Phe)/(Gly)+(Orn+Arg)/(Tyr+Lys) |
| 76 | 0.738 | (Phe)/(Asn)+(Thr+His)/(Trp+Tyr) |
| 77 | 0.738 | (Leu)/(Lys)+(Phe+Thr)/(Met+Asn) |
| 78 | 0.738 | (Phe)/(Asn)+(Orn+Thr+Tau)/(Lys) |
| 79 | 0.738 | (Phe)/(Met+Asn)+(Thr)/(Pro+Tyr) |
| 80 | 0.738 | (Orn)/(Met)+(Thr+Ile+Leu)/(Asn) |
| 81 | 0.738 | (Orn)/(Asn)+(Phe+Thr+Glu)/(Gly) |
| 82 | 0.738 | (Phe)/(Met+Asn)+(Thr)/(Tyr+Gly) |
| 83 | 0.738 | (Thr)/(Trp)+(Orn+Leu+Cit)/(Asn) |
| 84 | 0.738 | (Phe)/(Asn)+(Orn+Thr)/(Gly) |
| 85 | 0.738 | (Phe)/(Asn)+(Thr+His+Tau)/(Gly) |
| 86 | 0.738 | (Phe+Cit)/(Asn)+(Thr+Leu)/(Gly) |
| 87 | 0.737 | (Phe)/(Asn)+(Thr+His)/(Met+Gly) |
| 88 | 0.737 | (Phe)/(Asn)+(Thr+His)/(Gly) |
| 89 | 0.737 | (Phe)/(Asn)+(Orn+Thr+Glu)/(Lys) |
| 90 | 0.737 | (Thr)/(Trp)+(Orn+Ile+Leu)/(Asn) |
| 91 | 0.737 | (Phe+Orn)/(Met)+(Thr+Leu)/(Asn) |
| 92 | 0.737 | (Phe)/(Asn)+(Orn+Thr)/(Met+Gly) |
| 93 | 0.737 | (Phe)/(Asn)+(Thr+Arg+Tau)/(Gly) |
| 94 | 0.737 | (Orn)/(Asn)+(Phe+Thr+Glu)/(Lys) |
| 95 | 0.737 | (Phe)/(Met+Asn)+(Thr)/(Trp+Lys) |
| 96 | 0.737 | (Phe)/(Gly)+(Thr+His)/(Lys+Asn) |
| 97 | 0.737 | (Phe)/(Asn)+(Thr+Glu+Arg)/(Gly) |
| 98 | 0.737 | (Phe)/(Asn)+(Thr+Ile)/(Pro+Trp) |
| 99 | 0.737 | (Thr)/(Trp)+(Phe+Leu+Tau)/(Asn) |
| 100 | 0.737 | (Phe+Cit)/(Asn)+(Orn+Thr)/(Gly) |

# FIG.37

| RANK | VALUE | FORMULA |
|---|---|---|
| 1 | 0.798 | (-0.869539*Tyr)/(Thr)+(0.0883756*Phe-0.0338308*Gly)/(Cit-0.123027*Pro)+1.04769 |
| 2 | 0.794 | (-1.65604*Asn)/(Thr)+(0.0872225*Phe-0.0323972*Gly)/(Cit-0.12598*Pro)+1.12203 |
| 3 | 0.792 | (-0.511402*Asn)/(Orn)+(0.0706713*Thr-0.0687869*Lys)/(Phe-0.493461*Tyr)+1.01902 |
| 4 | 0.792 | (2.23744*Phe)/(Gly)+(-0.0813712*Met+0.0278369*Thr)/(ABA-0.116015*Arg)-0.471522 |
| 5 | 0.789 | (-1.6376*Asn)/(Thr)+(-0.285714*Tyr+0.132292*Orn)/(Phe-0.139255*Lys)+1.41413 |
| 6 | 0.788 | (-1.68187*Asn)/(Thr)+(0.988016*Phe-0.639718*Tyr)/(Gly-0.209336*Gln)+0.803209 |
| 7 | 0.788 | (-0.0725615*Lys)/(Phe-0.375936*Tyr)+(-2.12324*Asn+0.546472*Orn)/(Thr)+1.35497 |
| 8 | 0.786 | (-1.6663*Asn)/(Thr)+(0.115518*Orn-0.0836806*Lys)/(Phe-0.42453*Tyr)+1.36723 |
| 9 | 0.783 | (-0.376603*Asn+0.22695*Phe-0.291022*ABA+0.0986588*Thr)/(Tyr-0.597321*His)+0.176319 |
| 10 | 0.783 | (-0.0743465*Lys)/(Phe-0.371289*Tyr)+(-0.970699*Asn+0.233063*Thr)/(Orn)+1.09197 |
| 11 | 0.782 | (0.582316*Orn)/(Trp-0.462068*Thr+0.273201*Lys)+(0.270255*Leu)/(Asn)-0.935042 |
| 12 | 0.782 | (-1.79035*Asn)/(Thr)+(-0.445272*ABA-0.0418849*Gly)/(Phe-0.376311*Tyr)+1.60341 |
| 13 | 0.782 | (-2.73157*Asn)/(Thr+0.0557917*Gln)(-0.281598*Tyr)/(Phe-0.116324*Lys)+1.71897 |
| 14 | 0.782 | (-0.245409*Tyr)/(Phe-0.119437*Lys)+(-1.98099*Asn+0.0914907*Val)/(Thr)+1.40626 |
| 15 | 0.782 | (-0.49795*Asn+0.359927*Phe-0.192384*Tyr+0.130952*Thr)/(Trp-0.393893*His)+0.263123 |
| 16 | 0.781 | (-0.187883*Lys)/(Phe)+(-0.866697*Asn+0.312834*Arg)/(Thr-0.242053*Gly)+1.09302 |
| 17 | 0.781 | (-0.256901*Asn+0.174329*Phe+0.0849601*Thr-0.0344367*Lys)/(Tyr-0.615535*His)+0.187236 |
| 18 | 0.781 | (-0.0869448*Gly)/(Orn)+(-2.16367*Asn-1.43014*ABA+1.08686*Phe)/(Thr)+1.20987 |
| 19 | 0.781 | (-0.224791*Tyr)/(Phe-0.12723*Lys)+(-2.069*Asn+0.544503*Orn)/(Thr)+1.36328 |
| 20 | 0.779 | (-0.276278*Tyr)/(Phe-0.11705*Lys)+(-2.13396*Asn+0.0380323*Gln)/(Thr)+1.52462 |
| 21 | 0.779 | (-1.65449*Asn)/(Thr)+(-0.266056*Tyr+0.113512*Cit)/(Phe-0.129831*Lys)+1.45529 |
| 22 | 0.779 | (-0.343609*Asn+0.160054*Phe+0.0667897*Thr)/(Tyr-0.577557*His-0.0539005*Ile)+0.169516 |
| 23 | 0.779 | (-1.74137*Asn)/(Thr)+(-0.802639*ABA-0.419053*Tyr-0.078118*Gly)/(Phe)+2.0671 |
| 24 | 0.779 | (-1.58515*Asn)/(Thr)+(-1.26401*Met+1.0611*Phe)/(Gly-0.173695*Gln)+0.665896 |
| 25 | 0.779 | (-0.246026*Tyr)/(Phe-0.124248*Lys)+(-2.06079*Asn+0.302094*Arg)/(Thr)+1.39092 |
| 26 | 0.778 | (-0.200444*Lys)/(Phe)+(-1.04364*Asn+0.374327*Thr+0.592921*Orn)/(Tyr)+0.587074 |
| 27 | 0.778 | (-0.394554*Asn+0.127085*Phe+0.0736949*Thr+0.0657293*Ile)/(Tyr-0.608342*His)+0.126207 |
| 28 | 0.778 | (-2.05264*ABA)/(Thr)+(-0.752908*Asn+0.655522*Phe-0.10871*Gly)/(Orn)+1.02815 |
| 29 | 0.777 | (-1.62969*Asn)/(Thr)+(-0.466394*Tyr+0.343162*Orn-0.168941*Lys)/(Phe)+1.78582 |
| 30 | 0.777 | (-0.898453*Asn+0.31934*Phe)/(Orn)+(0.398543*Thr-0.391999*Lys)/(Leu)+0.98919 |
| 31 | 0.777 | (-0.170887*Lys)/(Phe)+(-0.533236*Asn+0.115889*Thr)/(Orn-0.0764518*Gly)+1.20027 |
| 32 | 0.776 | (-2.66185*Asn+3.29992*Phe)/(Gly)+(-1.15531*ABA+0.431998*Thr)/(Tyr)-0.456752 |
| 33 | 0.776 | (-2.04746*Asn+0.502068*Orn)/(Thr)+(-0.373926*Tyr-0.125412*Lys)/(Phe)+1.7521 |
| 34 | 0.776 | (-1.74941*Asn)/(Thr)+(-0.315719*Tyr-0.0595397*Gly)/(Phe-0.701333*ABA)+1.801 |
| 35 | 0.776 | (-1.01246*Asn)/(Phe)+(0.190413*Thr-0.0672862*Lys)/(Tyr-0.640639*Orn)+0.818399 |
| 36 | 0.776 | (-0.278961*Lys)/(Thr)+(-0.496422*Asn+0.298988*Phe+0.227183*Orn)/(Cit)+0.548606 |
| 37 | 0.776 | (-1.64985*Asn)/(Thr)+(-0.26903*Tyr-0.111571*Trp)/(Phe-0.0995073*Lys)+1.64031 |
| 38 | 0.776 | (-1.67759*Asn)/(Thr)(-0.0870392*Lys)/(Phe-0.437398*Tyr+0.132551*Orn)+1.49311 |
| 39 | 0.776 | (0.222129*Thr)/(Asn)+(0.111829*Orn-0.0826218*Lys)/(Phe-0.427644*Tyr)+0.122183 |
| 40 | 0.776 | (-0.187598*Lys)/(Phe)+(-0.916971*Asn+0.537604*Orn+0.303457*Thr)/(Trp)+0.570317 |
| 41 | 0.776 | (-0.0690709*Lys)/(Phe-0.388621*Tyr)+(0.20555*Thr+0.211613*Orn)/(Asn)-0.0345207 |
| 42 | 0.776 | (-1.19532*Asn)/(Phe)+(-0.643718*ABA+0.158104*Thr)/(Tyr-0.720385*Orn)+1.01887 |
| 43 | 0.775 | (-0.217379*Tyr)/(Phe-0.129624*Lys)+(-0.958566*Asn+0.227629*Thr)/(Orn)+1.10899 |
| 44 | 0.775 | (-0.700274*Asn)/(Orn)+(-1.21544*Cit+0.848963*Phe-0.319267*Lys)/(Thr)+1.37266 |
| 45 | 0.775 | (-0.930066*Asn+0.392204*Phe)/(Orn)+(0.902117*Thr-0.731866*Lys)/(Val)+0.832832 |
| 46 | 0.775 | (-1.58335*Asn)/(Thr)+(0.680062*Phe-0.165226*Lys)/(Gly-0.218682*Gln)+0.81381 |
| 47 | 0.774 | (1.77115*Phe)/(Gly)+(-0.893743*Asn+0.26936*Arg)/(Thr-0.256443*Lys)+0.0409654 |
| 48 | 0.774 | (-0.26637*Tyr)/(Phe-0.116617*Lys)+(-2.2452*Asn+0.367367*His)/(Thr)+1.47361 |
| 49 | 0.774 | (-1.73888*Asn)/(Thr)+(0.0825765*His-0.0912335*Lys)/(Phe-0.402151*Tyr)+1.40253 |
| 50 | 0.774 | (-0.076009*Lys)/(Phe-0.374436*Tyr)+(-2.0992*Asn+0.295373*Arg)/(Thr)+1.36802 |

# FIG.38

| 51 | 0.773 | (-1.60199*Asn)/(Thr)+(-0.436893*ABA+0.363931*Phe-0.0751772*Gly)/(Cit)+1.05947 |
|---|---|---|
| 52 | 0.773 | (-0.314555*Lys)/(Thr)+(0.187217*Phe-0.135138*Tyr)/(Asn-0.344638*Ile)+0.781088 |
| 53 | 0.773 | (-0.0875393*Gly)/(Orn)+(-1.08499*Asn-0.771497*ABA+0.211627*Thr)/(Phe)+1.34601 |
| 54 | 0.773 | (-0.504672*Asn+0.378613*Phe)/(Cit)+(-1.34794*ABA-0.159926*Gly)/(Thr)+0.858514 |
| 55 | 0.773 | (-1.68043*Asn)/(Thr)+(-0.0826648*Trp-0.0764662*Lys)/(Phe-0.327455*Tyr)+1.55979 |
| 56 | 0.773 | (-0.198669*Lys)/(His)+(-1.16452*Asn+0.196497*Ile+0.168394*Thr)/(Orn)+1.18356 |
| 57 | 0.772 | (-0.0779764*Lys)/(Phe-0.382316*Trp)+(-1.02587*Asn+0.209604*Thr)/(Orn)+1.1754 |
| 58 | 0.772 | (-0.520195*Asn)/(Orn)+(1.64702*Phe+0.770962*Thr-0.435842*Lys)/(Gly)+0.282905 |
| 59 | 0.772 | (-0.24987*Lys)/(Leu)+(-1.12316*Asn+0.617905*Phe+0.312594*Thr)/(Trp)+0.346935 |
| 60 | 0.772 | (-1.19457*Asn+1.27248*Phe-0.747556*Tyr+0.464446*Thr)/(Gly-0.187233*Gln)+0.0595845 |
| 61 | 0.772 | (-0.211904*Lys)/(Phe+0.291457*Glu)+(-0.975449*Asn+0.195906*Thr)/(Orn)+1.3002 |
| 62 | 0.772 | (-1.26305*Asn+0.729735*Phe+0.388403*Thr)/(Tyr-0.971837*His+0.410265*Gly)-0.116236 |
| 63 | 0.772 | (-0.849889*Asn+0.189913*Thr)/(Orn)+(2.14964*Phe-0.277681*Lys)/(Gly)+0.256637 |
| 64 | 0.772 | (-0.97715*Asn)/(Phe)+(0.463896*Orn+0.285405*Thr-0.160773*Lys)/(Trp)+0.622881 |
| 65 | 0.772 | (-1.87266*Asn+0.257623*Arg)/(Thr)+(2.72631*Phe-1.14587*Tyr)/(Gly)+0.431533 |
| 66 | 0.771 | (-1.10663*Asn+0.652154*Phe+0.3107*Thr+0.211888*Leu-0.141422*Lys)/(Trp)-0.0751372 |
| 67 | 0.771 | (-0.331511*Asn+0.199218*Phe+0.0866695*Thr-0.0202779*Gly)/(Tyr-0.595663*His)+0.154542 |
| 68 | 0.771 | (-0.48678*Asn+0.294962*Phe-0.0627609*Tyr+0.0934901*Thr)/(Trp-0.361519*Arg)+0.190306 |
| 69 | 0.771 | (-0.525998*Asn)/(Orn)+(-0.459613*Tyr+0.256088*Thr-0.193895*Lys)/(Phe)+1.48033 |
| 70 | 0.771 | (-0.562862*Asn)/(Phe-0.302421*Tyr)+(0.333945*Thr-0.108288*Lys)/(Trp)+0.702125 |
| 71 | 0.771 | (0.967739*Thr)/(Gly)+(-1.37516*Asn-0.900996*ABA)/(Phe+0.430431*Orn)+0.74424 |
| 72 | 0.771 | (-1.61749*Asn)/(Thr)+(3.31224*Phe-2.03511*ABA-1.19501*Tyr)/(Gly)+0.599999 |
| 73 | 0.771 | (-1.71076*Asn)/(Thr)(-0.0764284*Lys)/(Phe-0.59134*Tyr+0.274188*Ile)+1.44985 |
| 74 | 0.771 | (-0.98159*Asn+0.19335*Thr)/(Orn)+(0.353676*Phe-0.079626*Lys)/(Cit)+0.53043 |
| 75 | 0.771 | (-0.504052*Asn+0.167174*Phe+0.0583171*Thr+0.0499894*Leu)/(Trp-0.111525*Ala)+0.268563 |
| 76 | 0.771 | (-0.0385693*Lys)/(Phe-0.0563942*Gln)+(-0.944282*Asn+0.17388*Thr)/(Orn)+1.04537 |
| 77 | 0.771 | (0.567763*Phe)/(Gly-0.181194*Gln)+(-1.84333*Asn+0.579697*Cit)/(Thr)+0.545747 |
| 78 | 0.770 | (-1.65278*Asn)/(Thr)+(-0.382841*Tyr-0.193398*Trp-0.106082*Lys)/(Phe)+1.98438 |
| 79 | 0.770 | (-1.82343*Asn+0.304404*Arg)/(Thr)+(2.23444*Phe-0.263514*Lys)/(Gly)+0.369729 |
| 80 | 0.770 | (-1.41903*Asn+1.00943*Phe-0.445449*Tyr+0.354479*Thr)/(Gly-0.323723*Ala)+0.198899 |
| 81 | 0.770 | (0.895505*Thr)/(Gly)+(-0.788461*Asn+0.477722*Phe-0.0682303*Lys)/(Orn)+0.206769 |
| 82 | 0.769 | (-1.47718*Asn)/(Thr)+(0.286645*Phe+0.202411*Orn-0.10293*Lys)/(Cit)+0.65575 |
| 83 | 0.769 | (-1.76711*Asn)/(Thr)+(-0.508674*Tyr+0.274049*His-0.168479*Lys)/(Phe)+1.8212 |
| 84 | 0.769 | (-0.970327*Asn+0.67648*Phe+0.412853*Orn+0.29627*Thr-0.155628*Lys)/(Trp)-0.0637976 |
| 85 | 0.769 | (-2.12941*Asn+0.270761*Leu)/(Thr)+(0.273718*Phe-0.0804584*Lys)/(Cit)+0.843963 |
| 86 | 0.769 | (-2.29752*Asn+0.391159*His)/(Thr)+(-0.415933*Tyr-0.118377*Lys)/(Phe)+1.82934 |
| 87 | 0.769 | (-0.0757821*Lys)/(Phe-0.375337*Tyr)+(-2.11205*Asn+0.0333618*Gln)/(Thr)+1.45904 |
| 88 | 0.769 | (-2.13117*Asn+0.0365802*Gln)/(Thr)+(-0.443069*Tyr-0.119164*Lys)/(Phe)+1.89755 |
| 89 | 0.769 | (0.288267*Thr)/(Trp)+(-1.34225*Asn+0.324573*Phe+0.17919*Leu)/(Orn)+0.123659 |
| 90 | 0.769 | (-0.0841718*Lys)/(Orn)+(-2.78637*Asn+1.86579*Phe+0.862863*Thr)/(Gly)+0.21465 |
| 91 | 0.769 | (-2.09223*Asn+0.399239*Ile)/(Thr)+(-0.429246*Tyr-0.309318*Trp)/(Phe)+1.73565 |
| 92 | 0.769 | (-0.94855*Asn+0.164719*Leu)/(Orn)+(0.217326*Thr-0.203552*Lys)/(Phe)+1.01297 |
| 93 | 0.768 | (-0.320048*Lys)/(Thr)+(-1.15858*Asn-0.571294*Tyr)/(Phe+0.42948*Orn)+2.00989 |
| 94 | 0.768 | (-1.49813*Asn)/(Thr)+(0.341927*Phe-0.0587333*Lys-0.0534383*Gly)/(Cit)+0.996279 |
| 95 | 0.768 | (-1.31461*Asn+0.833902*Phe+0.304582*Thr-0.127291*Lys)/(Gly-0.336033*Ala)+0.254265 |
| 96 | 0.768 | (-0.265857*Lys)/(Thr)+(-0.768235*Asn+0.487796*Phe-0.0755521*Gly)/(Orn)+1.20509 |
| 97 | 0.768 | (-0.639285*Lys)/(Thr+0.582213*Leu)+(-0.913715*Asn+0.321741*Phe)/(Orn)+1.42643 |
| 98 | 0.768 | (1.06563*Asn+1.06563*Phe+1.06563*Tyr+1.06563*Thr)/(Gly+1.06563*Ser)+0 |
| 99 | 0.768 | (-0.073804*Lys)/(Phe-0.37409*Tyr)+(-2.28503*Asn+0.369142*His)/(Thr)+1.41403 |
| 100 | 0.767 | (-0.927042*Asn+0.192321*Thr)/(Orn)+(-0.114595*Lys-0.0737284*Gly)/(Phe)+1.33614 |

# FIG.39

| RANK | VALUE | FORMULA |
|------|-------|---------|
| 1 | 0.766 | [1.3714]+[0.0236]Thr+[-0.1103]Asn+[0.0221]Leu+[0.0415]Phe+[-0.0371]Trp+[-0.0153]Lys |
| 2 | 0.761 | [0.9467]+[0.0164]Thr+[-0.0876]Asn+[-0.0039]Pro+[0.0219]Ile+[0.0359]Orn+[-0.0132]Lys |
| 3 | 0.761 | [1.7611]+[0.0269]Thr+[-0.0884]Asn+[-0.0101]Gly+[-0.0604]ABA+[-0.0396]Tyr+[0.0719]Phe |
| 4 | 0.759 | [-0.8868]+[0.0190]Thr+[-0.0949]Asn+[0.0106]Glu+[0.0402]Phe+[0.0266]Orn+[-0.0119]Lys |
| 5 | 0.759 | [-0.1198]+[0.0207]Thr+[-0.1150]Asn+[0.0156]Leu+[0.0251]Phe+[0.0265]Orn+[-0.0147]Lys |
| 6 | 0.759 | [1.1272]+[0.0184]Thr+[-0.0911]Asn+[-0.0077]Gly+[0.0382]Phe+[0.0255]Orn+[-0.0106]Lys |
| 7 | 0.758 | [-0.5900]+[0.0060]Tau+[0.0190]Thr+[-0.0974]Asn+[0.0415]Phe+[0.0239]Orn+[-0.0115]Lys |
| 8 | 0.758 | [0.4652]+[0.0204]Thr+[-0.1137]Asn+[0.0030]Ile+[0.0200]Leu+[0.0326]Orn+[-0.0152]Lys |
| 9 | 0.757 | [1.5111]+[0.0198]Thr+[-0.1046]Asn+[-0.0059]Gly+[0.0179]Leu+[0.0334]Orn+[-0.0134]Lys |
| 10 | 0.756 | [-0.0009]+[0.0199]Thr+[-0.0982]Asn+[-0.0452]Cit+[0.0452]Phe+[0.0399]Orn+[-0.0118]Lys |
| 11 | 0.756 | [1.7677]+[0.0234]Thr+[-0.1122]Asn+[0.0315]Leu+[-0.0410]Trp+[0.0415]Orn+[-0.0191]Lys |
| 12 | 0.756 | [0.6066]+[0.0209]Thr+[-0.1115]Asn+[-0.0007]Ala+[0.0220]Leu+[0.0317]Orn+[-0.0152]Lys |
| 13 | 0.756 | [-0.2855]+[0.0264]Thr+[-0.1050]Asn+[-0.0570]ABA+[0.0147]Ile+[-0.0401]Tyr+[0.0650]Phe |
| 14 | 0.756 | [-0.2847]+[0.0210]Thr+[-0.1099]Asn+[-0.0090]Gly+[-0.0523]ABA+[0.0495]Phe+[0.0220]His |
| 15 | 0.755 | [-0.8952]+[0.0180]Thr+[-0.0987]Asn+[0.0052]Val+[0.0374]Phe+[0.0236]Orn+[-0.0128]Lys |
| 16 | 0.755 | [0.4608]+[0.0208]Thr+[-0.1136]Asn+[0.0214]Leu+[0.0328]Orn+[-0.0152]Lys |
| 17 | 0.754 | [-0.2092]+[0.0182]Thr+[-0.1014]Asn+[0.0114]Ile+[0.0342]Phe+[0.0248]Orn+[-0.0126]Lys |
| 18 | 0.754 | [0.6238]+[0.0196]Thr+[0.0152]Ser+[-0.1078]Asn+[-0.0112]Gly+[-0.0600]ABA+[0.0502]Phe |
| 19 | 0.754 | [0.5099]+[0.0195]Thr+[-0.0987]Asn+[-0.0091]Gly+[-0.0472]ABA+[0.0450]Phe+[0.0171]Orn |
| 20 | 0.754 | [-0.8220]+[0.0213]Thr+[-0.1258]Asn+[0.0180]Leu+[0.0276]His+[0.0369]Orn+[-0.0168]Lys |
| 21 | 0.754 | [0.4917]+[0.0209]Thr+[-0.1138]Asn+[0.0214]Leu+[0.0330]Orn+[-0.0151]Lys+[-0.0007]Arg |
| 22 | 0.754 | [0.7695]+[0.0196]Thr+[-0.0969]Asn+[-0.0092]Gly+[-0.0493]ABA+[0.0502]Phe+[0.0030]Arg |
| 23 | 0.754 | [0.6475]+[0.0209]Thr+[-0.1073]Asn+[-0.0081]Gly+[-0.0514]ABA+[0.0071]Leu+[0.0447]Phe |
| 24 | 0.754 | [0.7870]+[0.0166]Thr+[-0.0923]Asn+[-0.0003]Gln+[0.0204]Ile+[0.0339]Orn+[-0.0125]Lys |
| 25 | 0.754 | [-0.4841]+[0.0277]Thr+[-0.1108]Asn+[-0.0542]ABA+[0.0094]Leu+[-0.0350]Tyr+[0.0612]Phe |
| 26 | 0.753 | [0.6648]+[0.0168]Thr+[-0.0933]Asn+[0.0207]Ile+[0.0335]Orn+[-0.0125]Lys |
| 27 | 0.753 | [0.6405]+[0.0168]Thr+[-0.0937]Asn+[0.0001]Ala+[0.0206]Ile+[0.0336]Orn+[-0.0125]Lys |
| 28 | 0.753 | [-0.0084]+[0.0189]Thr+[-0.0856]Asn+[-0.0089]Pro+[0.0513]Phe+[0.0271]Orn+[-0.0130]Lys |
| 29 | 0.753 | [1.2686]+[0.0204]Thr+[-0.0901]Asn+[-0.0083]Gly+[-0.0377]ABA+[0.0521]Phe+[-0.0058]Lys |
| 30 | 0.753 | [0.6198]+[0.0200]Thr+[-0.0965]Asn+[0.0040]Glu+[-0.0087]Gly+[-0.0488]ABA+[0.0512]Phe |
| 31 | 0.753 | [0.6729]+[0.0168]Thr+[-0.0933]Asn+[0.0207]Ile+[0.0336]Orn+[-0.0125]Lys+[-0.0002]Arg |
| 32 | 0.753 | [0.6702]+[0.0166]Thr+[-0.0934]Asn+[0.0041]ABA+[0.0204]Ile+[0.0335]Orn+[-0.0128]Lys |
| 33 | 0.753 | [0.4628]+[0.0207]Thr+[-0.1137]Asn+[0.0011]ABA+[0.0213]Leu+[0.0328]Orn+[-0.0152]Lys |
| 34 | 0.753 | [0.3837]+[0.0205]Thr+[-0.1130]Asn+[0.0007]Val+[0.0206]Leu+[0.0326]Orn+[-0.0151]Lys |
| 35 | 0.753 | [0.3014]+[0.0211]Thr+[-0.1154]Asn+[0.0004]Gln+[0.0216]Leu+[0.0322]Orn+[-0.0152]Lys |
| 36 | 0.753 | [0.8951]+[0.0265]Thr+[-0.0905]Asn+[-0.0453]Tyr+[0.0633]Phe+[0.0320]Orn+[-0.0161]Lys |
| 37 | 0.752 | [0.6103]+[0.0074]Tau+[0.0195]Thr+[-0.0981]Asn+[-0.0090]Gly+[-0.0469]ABA+[0.0510]Phe |
| 38 | 0.752 | [-0.8242]+[0.0223]Thr+[-0.1152]Asn+[-0.0018]Ala+[-0.0477]ABA+[0.0128]Leu+[0.0415]Phe |
| 39 | 0.752 | [0.2082]+[0.0205]Thr+[-0.1117]Asn+[0.0050]Glu+[0.0202]Leu+[0.0337]Orn+[-0.0149]Lys |
| 40 | 0.752 | [-1.8212]+[0.0206]Thr+[-0.1150]Asn+[0.0369]Phe+[0.0329]His+[0.0293]Orn+[-0.0145]Lys |
| 41 | 0.752 | [-0.0755]+[0.0224]Thr+[-0.1121]Asn+[-0.0260]ABA+[0.0145]Leu+[0.0390]Phe+[-0.0105]Lys |
| 42 | 0.752 | [-1.1757]+[0.0182]Thr+[-0.1184]Asn+[0.0207]Ile+[0.0382]His+[0.0389]Orn+[-0.0159]Lys |
| 43 | 0.752 | [-0.4740]+[0.0201]Thr+[-0.0960]Asn+[-0.0166]ABA+[0.0448]Phe+[0.0234]Orn+[-0.0108]Lys |
| 44 | 0.752 | [0.0846]+[0.0253]Thr+[-0.1119]Asn+[0.0128]Leu+[-0.0302]Tyr+[0.0539]Phe+[-0.0264]Trp |
| 45 | 0.752 | [0.6961]+[0.0210]Thr+[-0.1126]Asn+[-0.0241]Cit+[0.0206]Leu+[0.0409]Orn+[-0.0147]Lys |
| 46 | 0.751 | [0.6816]+[0.0204]Thr+[-0.0987]Asn+[0.0005]Gln+[-0.0091]Gly+[-0.0486]ABA+[0.0513]Phe |
| 47 | 0.751 | [-1.2087]+[0.0220]Thr+[-0.1221]Asn+[0.0002]Gln+[-0.0447]ABA+[0.0117]Leu+[0.0410]Phe |
| 48 | 0.751 | [1.3732]+[0.0256]Thr+[-0.0939]Asn+[0.0165]Ile+[-0.0439]Tyr+[0.0604]Phe+[-0.0141]Lys |
| 49 | 0.751 | [-0.8677]+[0.0219]Thr+[-0.1134]Asn+[-0.0075]Pro+[-0.0496]ABA+[0.0113]Leu+[0.0511]Phe |
| 50 | 0.751 | [0.0373]+[0.0165]Thr+[-0.0930]Asn+[0.0116]Glu+[0.0200]Ile+[0.0355]Orn+[-0.0123]Lys |

# FIG.40

| | | |
|---|---|---|
| 51 | 0.751 | [-0.3410]+[0.0195]Thr+[-0.0962]Asn+[-0.0000]Ala+[0.0416]Phe+[0.0244]Orn+[-0.0119]Lys |
| 52 | 0.750 | [-0.0468]+[0.0046]Tau+[0.0209]Thr+[-0.1131]Asn+[0.0140]Leu+[0.0346]Phe+[-0.0117]Lys |
| 53 | 0.750 | [-0.1251]+[0.0191]Thr+[-0.0948]Asn+[-0.0005]Gln+[0.0412]Phe+[0.0254]Orn+[-0.0119]Lys |
| 54 | 0.750 | [0.8421]+[0.0196]Thr+[-0.1011]Asn+[-0.0087]Gly+[-0.0516]ABA+[0.0067]Ile+[0.0480]Phe |
| 55 | 0.750 | [2.0209]+[0.0163]Thr+[-0.0873]Asn+[-0.0075]Gly+[0.0167]Ile+[0.0339]Orn+[-0.0109]Lys |
| 56 | 0.750 | [-0.8606]+[0.0268]Thr+[-0.0950]Asn+[0.0106]Glu+[-0.0490]ABA+[-0.0382]Tyr+[0.0701]Phe |
| 57 | 0.750 | [0.3584]+[0.0026]Tau+[0.0205]Thr+[-0.1140]Asn+[0.0213]Leu+[0.0326]Orn+[-0.0150]Lys |
| 58 | 0.749 | [0.8777]+[0.0202]Thr+[-0.0969]Asn+[-0.0090]Gly+[-0.0496]ABA+[0.0515]Phe |
| 59 | 0.749 | [0.8874]+[0.0202]Thr+[-0.0970]Asn+[-0.0090]Gly+[-0.0011]Cit+[-0.0496]ABA+[0.0517]Phe |
| 60 | 0.749 | [-0.3494]+[0.0195]Thr+[-0.0964]Asn+[0.0415]Phe+[0.0245]Orn+[-0.0119]Lys |
| 61 | 0.749 | [-1.0932]+[0.0219]Thr+[-0.1209]Asn+[-0.0452]ABA+[0.0115]Leu+[0.0412]Phe |
| 62 | 0.749 | [-0.2873]+[0.0280]Thr+[-0.1022]Asn+[-0.0421]Tyr+[0.0663]Phe+[0.0323]His+[-0.0154]Lys |
| 63 | 0.749 | [1.5749]+[0.0227]Thr+[-0.0980]Asn+[-0.0088]Gly+[0.0085]Ile+[-0.0375]Tyr+[0.0533]Phe |
| 64 | 0.749 | [1.7493]+[0.0169]Thr+[-0.0877]Asn+[0.0322]Ile+[-0.0335]Trp+[0.0408]Orn+[-0.0147]Lys |
| 65 | 0.749 | [1.0453]+[0.0204]Thr+[-0.0929]Asn+[-0.0089]Gly+[-0.0011]Ala+[-0.0507]ABA+[0.0524]Phe |
| 66 | 0.749 | [0.3913]+[0.0234]Thr+[-0.1035]Asn+[0.0194]Ile+[-0.0367]Tyr+[0.0585]Phe+[-0.0273]Trp |
| 67 | 0.749 | [0.4088]+[0.0064]Tau+[0.0162]Thr+[-0.0945]Asn+[0.0209]Ile+[0.0331]Orn+[-0.0122]Lys |
| 68 | 0.749 | [-1.0870]+[0.0217]Thr+[-0.1209]Asn+[-0.0454]ABA+[0.0014]Ile+[0.0110]Leu+[0.0411]Phe |
| 69 | 0.749 | [1.5585]+[0.0274]Thr+[-0.0800]Asn+[-0.0382]Tyr+[0.0749]Phe+[-0.0213]Trp+[-0.0132]Lys |
| 70 | 0.748 | [0.0311]+[0.0210]Thr+[-0.1120]Asn+[0.0144]Leu+[0.0335]Phe+[-0.0124]Lys+[0.0026]Arg |
| 71 | 0.748 | [0.2229]+[0.0201]Thr+[-0.1039]Asn+[-0.0073]Gly+[0.0434]Phe+[0.0258]His+[-0.0102]Lys |
| 72 | 0.748 | [1.2780]+[0.0202]Thr+[-0.1037]Asn+[-0.0062]Gly+[0.0104]Leu+[0.0359]Phe+[-0.0103]Lys |
| 73 | 0.748 | [-0.2195]+[0.0200]Thr+[-0.0971]Asn+[0.0422]Phe+[0.0255]Orn+[-0.0116]Lys+[-0.0033]Arg |
| 74 | 0.747 | [-0.4245]+[0.0243]Thr+[-0.0921]Asn+[0.0138]Glu+[-0.0344]Tyr+[0.0659]Phe+[-0.0225]Trp |
| 75 | 0.747 | [0.4606]+[0.0217]Thr+[-0.1068]Asn+[-0.0018]Ala+[0.0158]Leu+[0.0340]Phe+[-0.0124]Lys |
| 76 | 0.747 | [2.3828]+[0.0240]Thr+[-0.0857]Asn+[-0.0103]Gly+[-0.0346]Tyr+[0.0658]Phe+[-0.0248]Trp |
| 77 | 0.747 | [-1.2792]+[0.0218]Thr+[0.0035]Ser+[-0.1241]Asn+[-0.0471]ABA+[0.0119]Leu+[0.0405]Phe |
| 78 | 0.747 | [-0.8569]+[0.0219]Thr+[-0.1207]Asn+[0.0113]Leu+[0.0357]Phe+[0.0220]His+[-0.0132]Lys |
| 79 | 0.747 | [0.1444]+[0.0214]Thr+[-0.1125]Asn+[0.0144]Leu+[0.0343]Phe+[-0.0120]Lys |
| 80 | 0.747 | [0.1435]+[0.0214]Thr+[-0.1125]Asn+[-0.0002]Ile+[0.0144]Leu+[0.0344]Phe+[-0.0120]Lys |
| 81 | 0.747 | [-0.0933]+[0.0213]Thr+[0.0053]Ser+[-0.1167]Asn+[0.0151]Leu+[0.0329]Phe+[-0.0126]Lys |
| 82 | 0.747 | [-0.0675]+[0.0211]Thr+[-0.1106]Asn+[0.0039]Glu+[0.0133]Leu+[0.0351]Phe+[-0.0117]Lys |
| 83 | 0.747 | [0.4130]+[0.0193]Thr+[-0.0993]Asn+[-0.0086]Gly+[-0.0474]ABA+[0.0030]Val+[0.0484]Phe |
| 84 | 0.747 | [-0.7897]+[0.0228]Thr+[-0.1211]Asn+[-0.0464]ABA+[0.0117]Leu+[0.0434]Phe+[-0.0056]Arg |
| 85 | 0.747 | [-1.3009]+[0.0201]Thr+[-0.0938]Asn+[0.0134]Glu+[-0.0103]Pro+[-0.0439]ABA+[0.0645]Phe |
| 86 | 0.746 | [0.8441]+[0.0180]Thr+[-0.0934]Asn+[-0.0070]Gly+[0.0043]Val+[0.0420]Phe+[-0.0091]Lys |
| 87 | 0.746 | [-1.3269]+[0.0214]Thr+[-0.1180]Asn+[0.0053]Glu+[-0.0437]ABA+[0.0102]Leu+[0.0419]Phe |
| 88 | 0.746 | [-1.8153]+[0.0186]Thr+[-0.1207]Asn+[0.0081]Glu+[0.0089]Leu+[0.0272]Phe+[0.0185]Orn |
| 89 | 0.746 | [-0.5832]+[0.0193]Thr+[0.0044]Ser+[-0.0989]Asn+[0.0407]Phe+[0.0252]Orn+[-0.0124]Lys |
| 90 | 0.746 | [0.5357]+[0.0263]Thr+[-0.0850]Asn+[0.0102]Glu+[-0.0407]Tyr+[0.0670]Phe+[-0.0120]Lys |
| 91 | 0.746 | [-0.0424]+[0.0250]Thr+[-0.1032]Asn+[-0.0555]Met+[0.0149]Leu+[0.0426]Phe+[-0.0104]Lys |
| 92 | 0.746 | [0.1781]+[0.0214]Thr+[-0.1124]Asn+[-0.0028]Cit+[0.0142]Leu+[0.0350]Phe+[-0.0119]Lys |
| 93 | 0.746 | [-0.1467]+[0.0205]Thr+[-0.1103]Asn+[0.0023]Val+[0.0118]Leu+[0.0351]Phe+[-0.0120]Lys |
| 94 | 0.744 | [0.0222]+[0.0197]Thr+[-0.0907]Asn+[0.0060]Val+[0.0528]Phe+[-0.0253]Trp+[-0.0117]Lys |
| 95 | 0.744 | [-1.8776]+[0.0193]Thr+[-0.1056]Asn+[0.0077]Glu+[-0.0372]ABA+[0.0037]Val+[0.0475]Phe |
| 96 | 0.743 | [-1.6509]+[0.0208]Thr+[-0.1078]Asn+[0.0081]Glu+[0.0457]Phe+[0.0269]His+[-0.0115]Lys |
| 97 | 0.743 | [2.3598]+[0.0223]Thr+[-0.1086]Asn+[0.0109]Ile+[0.0258]Leu+[-0.0322]Trp+[-0.0142]Lys |
| 98 | 0.743 | [-0.3168]+[0.0222]Thr+[-0.1176]Asn+[0.0010]Gln+[0.0151]Leu+[0.0336]Phe+[-0.0123]Lys |
| 99 | 0.743 | [-1.6754]+[0.0198]Thr+[-0.1104]Asn+[0.0045]Val+[0.0429]Phe+[0.0254]His+[-0.0124]Lys |
| 100 | 0.743 | [1.9547]+[0.0233]Thr+[0.0049]Ser+[-0.1128]Asn+[0.0299]Leu+[-0.0280]Trp+[-0.0144]Lys |

## INTERNATIONAL SEARCH REPORT

| | International application No. |
|---|---|
| | PCT/JP2008/068981 |

A.  CLASSIFICATION OF SUBJECT MATTER
*G01N33/68*(2006.01)i, *A61B5/145*(2006.01)i, *G01N33/15*(2006.01)i, *G01N33/50* (2006.01)i

According to International Patent Classification (IPC) or to both national classification and IPC

B.  FIELDS SEARCHED

Minimum documentation searched (classification system followed by classification symbols)
G01N33/68, A61B5/145, G01N33/15, G01N33/50

Documentation searched other than minimum documentation to the extent that such documents are included in the fields searched
Jitsuyo Shinan Koho          1922-1996   Jitsuyo Shinan Toroku Koho   1996-2008
Kokai Jitsuyo Shinan Koho     1971-2008   Toroku Jitsuyo Shinan Koho   1994-2008

Electronic data base consulted during the international search (name of data base and, where practicable, search terms used)
JSTPlus(JDreamII), JMEDPlus(JDreamII), JST7580(JDreamII),
TAITONO, AMINOSAN (in Japanese)

C.  DOCUMENTS CONSIDERED TO BE RELEVANT

| Category* | Citation of document, with indication, where appropriate, of the relevant passages | Relevant to claim No. |
|---|---|---|
| X | WO 2004/052191 A1  (Ajinomoto Co., Inc.), 24 June, 2004 (24.06.04), & US 2005/283347 A1      & EP 1570779 A1 & CA 2508136 A            & KR 10-2005-84227 A & CN 1744850 A | 1-34 |
| X | WO 2006/98192 A1  (Ajinomoto Co., Inc.), 21 September, 2006 (21.09.06), & EP 1862797 A1 | 1-34 |
| A | WO 2003/083133 A1  (Asahi Kasei Pharma Corp.), 09 October, 2003 (09.10.03), & US 2005/0214885 A1     & EP 1491636 A1 & CN 1650025 A | 1-34 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search 07 November, 2008 (07.11.08) | Date of mailing of the international search report 18 November, 2008 (18.11.08) |
|---|---|
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2008/068981

C (Continuation). DOCUMENTS CONSIDERED TO BE RELEVANT

| Category* | Citation of document, with indication, where appropriate, of the relevant passages | Relevant to claim No. |
|---|---|---|
| A | JP 2001-190299 A  (Asahi Kasei Corp.),<br>17 July, 2001 (17.07.01),<br>(Family: none) | 1-34 |
| A | JP 2000-298131 A  (Ryuzo KAWAMORI),<br>24 October, 2000 (24.10.00),<br>(Family: none) | 1-34 |

Form PCT/ISA/210 (continuation of second sheet) (April 2007)

## REFERENCES CITED IN THE DESCRIPTION

**Patent documents cited in the description**

- WO 2004052191 A
- WO 2006098192 A
- WO 2003069328 A
- WO 2005116629 A

**Non-patent literature cited in the description**

- The Expert Committee on the Diagnosis and Classification of Diabates Mellitus. *Diabates Care,* 1997, vol. 20, 1183
- **KUZUYA, Takeshi et al.** Commission report on classification and diagnostic criteria for diabetes mellitus. *Journal of the Japan Diabetes Society,* 1999, vol. 42, 3835
- **Tominaga, M. et al.** The Funagata Diabates Study. *Diabetes Care,* 1999, vol. 22, 920
- The Expert Committee on the Diagnosis and Classification of Diabates Mellitus. *Diabetes Care,* 2003, vol. 26, 3160
- **Ko, G. et al.** *Diabetes Care,* 1998, vol. 21, 1221
- **Wasim, H. et al.** *Cardiovasc Diabetol.,* 2006, vol. 25, 10
- **Felig, P. ; Marliss, E. et al.** *New Engl. J. Med.,* 1969, vol. 281, 811
- **Felig, P. ; Marliss, E. et al.** *Diabates,* 1970, vol. 19, 727